# FASCICULE DE BREVET EUROPEEN

(11) **EP 2 830 591 B1**
(45) Date de publication et mention de la délivrance du brevet: **22.03.2023**
(21) Numéro de dépôt: 13717207.8
(22) Date de dépôt: 02.04.2013
(51) Int. Cl.: A61K 9/107, A61K 47/24, A61K 47/30, A61K 47/34, A61K 47/18, A61K 47/59, A61K 47/69

(54) **MATERIAU, SON PROCEDE DE PREPARATION ET SES UTILISATIONS**
MATERIAL, VERFAHREN ZUR HERSTELLUNG DAVON UND VERWENDUNGEN DAVON
MATERIAL, ITS PROCESS OF PREPARATION AND UTILISATIONS THEREOF

(30) Priorité: 30.03.2012 FR 1252941
(43) Date de publication de la demande: 04.02.2015
(73) Titulaire: Commissariat à l'Énergie Atomique et aux Énergies Alternatives, 75015 Paris (FR)
(72) Inventeur: COUFFIN, Anne-Claude, F-38640 CLAIX (FR); DELMAS, Thomas, 06410 BIOT (FR); HEINRICH, Emilie, F-38360 Sassenage (FR); TEXIER-NOGUES, Isabelle, F-38000 Grenoble (FR); THOMANN, Jean-Sébastien, F-54400 Longwy (FR); MOURIER, Véronique, F-38430 Saint Jean de Moirans (FR)
(74) Mandataire: Lavoix
(86) Numéro de dépôt international: PCT/EP2013/056923
(87) Numéro de publication internationale: WO 2013/144369

(56) Documents cités:
- WO-A1-2010/018223
- WO-A1-2011/101602
- MENG F ET AL: "Reduction-sensitive polymers and bioconjugates for biomedical applications", BIOMATERIALS, ELSEVIER SCIENCE PUBLISHERS BV., BARKING, GB, vol. 30, no. 12, 1 avril 2009 (2009-04-01) , pages 2180-2198, XP025990543, ISSN: 0142-9612, DOI: 10.1016/J.BIOMATERIALS.2009.01.026 [extrait le 2009-02-05]
- SHEN J ET AL: "Mucoadhesive effect of thiolated PEG stearate and its modified NLC for ocular drug delivery", JOURNAL OF CONTROLLED RELEASE, ELSEVIER, AMSTERDAM, NL, vol. 137, no. 3, 4 août 2009 (2009-08-04), pages 217-223, XP026223534, ISSN: 0168-3659, DOI: 10.1016/J.JCONREL.2009.04.021 [extrait le 2009-04-21]
- KOVACEVIC A ET AL: "Polyhydroxy surfactants for the formulation of lipid nanoparticles (SLN and NLC): Effects on size, physical stability and particle matrix structure", INTERNATIONAL JOURNAL OF PHARMACEUTICS, ELSEVIER BV, NL, vol. 406, no. 1, 27 décembre 2010 (2010-12-27), pages 163-172, XP028363295, ISSN: 0378-5173, DOI: 10.1016/J.IJPHARM.2010.12.036 [extrait le 2011-01-08]
- DELMAS T ET AL: "Preparation and characterization of highly stable lipid nanoparticles with amorphous core of tuneable viscosity", JOURNAL OF COLLOID AND INTERFACE SCIENCE 2011 ACADEMIC PRESS INC. USA LNKD- DOI:10.1016/J.JCIS.2011.04.080, vol. 360, no. 2, 15 août 2011 (2011-08-15) , pages 471-481, XP002686819, ISSN: 0021-9797

## Description

La présente invention concerne un matériau comprenant une phase aqueuse continue et une phase dispersée sous forme de gouttelettes liées entre-elles de façon covalente.

La littérature décrit des matériaux à base d'eau et dont la forme physique et les propriétés sont modulables, permettant des applications dans des domaines très variés. Par exemple, on peut citer les hydrogels, qui sont des réseaux tridimensionnels à base de polymères ayant de nombreuses applications dans les domaines alimentaire, agricole et médical (lentilles de contact, délivrance d'agent thérapeutique, implants mammaires, pansement, revêtement de prothèses) (Hamidi et al. Advanced drug delivery reviews, 60, 2008, 1638).

La présente invention fournit un matériau à base d'eau alternatif aux hydrogels et présentant les mêmes propriétés avantageuses, car il est possible de moduler et maintenir une forme physique et d'adapter les propriétés physiques et chimiques du matériau en fonction de l'application souhaitée.

Par ailleurs, la demande WO 2011/101602 décrit une nanoémulsion huile dans l'eau sous forme de gel pour la délivrance d'agents intérêts hydrophile et lipophile. Le réseau tridimensionnel de ce gel est formé par les gouttelettes liées entre-elles par des interactions non covalentes. Toutefois, ce gel est relativement fluide, et après mise en forme, il ne se maintient pas suffisamment en forme. De plus, ce gel mis en présence d'une phase aqueuse (et notamment de liquide physiologique lors de son administration *in vivo*) se désagrège rapidement et le réseau tridimensionnel du gel est détruit. Par ailleurs, ces nanoémulsions nécessitent des proportions en phase dispersée élevée. Ces trois inconvénients constituent un frein pour de nombreuses applications d'un gel.

La présente invention fournit un matériau sous forme d'une émulsion huile dans l'eau qui ne présente pas ces inconvénients : Le matériau peut être mis en forme, y rester et résiste à la dilution dans l'eau. Il peut ainsi être utilisé dans des applications très diverses, notamment des applications nécessitant la présence d'une phase aqueuse.

L'invention concerne un procédé de préparation d'un matériau comprenant une phase aqueuse continue et une phase dispersée sous forme de gouttelettes, ce procédé étant tel que défini aux revendications 1 à 5.

L'invention concerne également le matériau tel que défini aux revendications 6 à 12.

### [Matériau]

Le matériau comprend une phase aqueuse continue et une phase dispersée sous forme de gouttelettes comprenant :
- un phospholipide,
- un lipide solubilisant comprenant au moins un glycéride d'acides gras,
- un co-tensioactif comprenant une chaîne composée de motifs d'oxyde d'éthylène ou d'oxyde d'éthylène et d'oxyde de propylène,
- une huile, et
- un tensioactif de formule (I) suivante :

   (L₁-X₁-H₁-Y₁)ᵥ-G-(Y₂-H₂-X₂-L₂)_{w} (I),

   dans laquelle :
   - les radicaux L₁-X₁-H₁- et L₂-X₂-H₂- consistent indépendamment en un groupe de formule suivante : 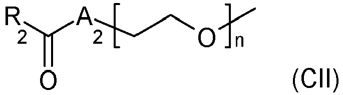 dans laquelle :
      - R₂ représente une chaîne hydrocarbonée linéaire comprenant de 11 à 23 atomes de carbone,
      - A₂ représente O ou NH,
      - n représente un nombre entier de 3 à 500,
   - Y₁ et Y₂ sont indépendamment choisis parmi :
      - une liaison simple,
      - un groupe Z choisi parmi -O-, -NH-, -O(OC)-, -(CO)O-, -(CO)NH-, -NH(CO)-, - O-(CO)-O-, -NH-(CO)-O-, -O-(CO)-NH- et -NH-(CO)-NH,
      - un groupe Alk étant un alkylène comprenant de 1 à 6 atomes de carbone, et
      - un groupe Z-Alk, Alk-Z, Alk-Z-Alk ou Z-Alk-Z où Alk et Z sont tels que définis ci-dessus et où les deux groupes Z du groupe Z-Alk-Z sont identiques ou différents,
   - G représente un groupe de liaison et comprend au moins un groupe G' ayant l'une des formules suivantes : 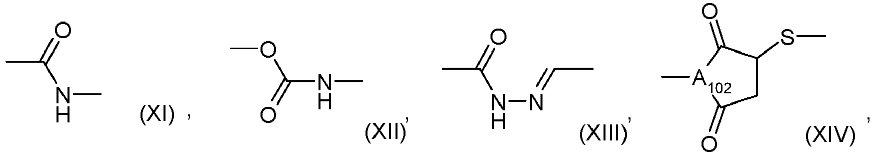 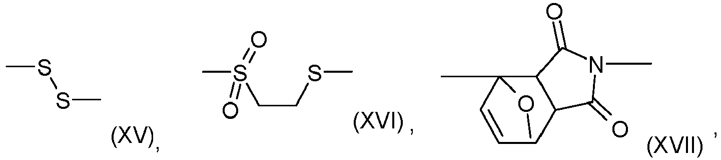 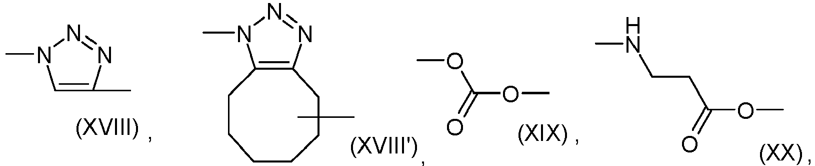 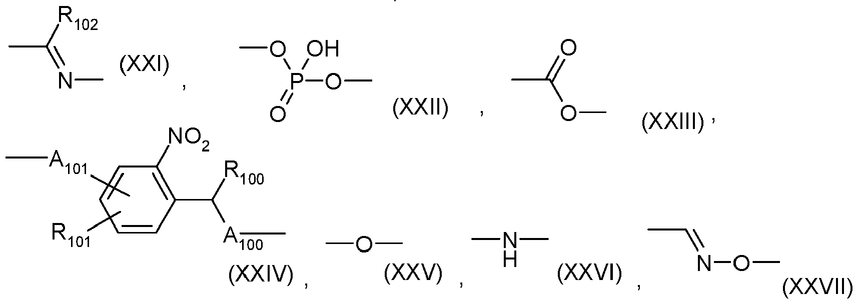
où A₁₀₂ représente CH ou N, R₁₀₂ représente H ou une chaîne hydrocarbonée linéaire comprenant de 1 à 6 atomes de carbone, A₁₀₁ représente -O-, -NH-(CO)- ou -O-(CO)-, R₁₀₀ représente H ou un méthyle, A₁₀₀ représente -O- ou -NH- et R₁₀₁ représente H, Me ou -OMe,
v et w représentent 1,
les gouttelettes de la phase dispersée étant liées entre-elles de façon covalente par le tensioactif de formule (I).

Le matériau se présente sous forme d'une émulsion huile dans l'eau dans laquelle les gouttelettes de phase dispersée sont liées entre-elles par liaison covalente. L'émulsion peut être simple ou multiple, notamment en comportant dans la phase dispersée une seconde phase aqueuse.

### • Définitions

Au sens de la présente demande, l'expression « phase dispersée » signifie les goutelettes comprenant l'huile/le lipide solubilisant/ le lipide amphiphile/ le co-tensioactif/ l'éventuel agent d'intérêt lipophile/le tensioactif de formule (I). La phase dispersée est généralement exempte de phase aqueuse. Le matériau est typiquement exempt de liposomes.

Le terme « gouttelette » englobe à la fois les gouttelettes d'huile liquide proprement dites ainsi que les particules solides issues d'émulsions de type huile-dans-eau dans lesquelles la phase huileuse est solide.

Les gouttelettes du matériau sont avantageusement monodisperses. L'écart type entre les diamètres minimum et maximum des gouttelettes par rapport au diamètre moyen est généralement inférieur ou égal à 30%, de préférence 20%. Le matériau se présente généralement sous forme d'une nanoémulsion huile dans l'eau : le diamètre moyen des gouttelettes de la phase dispersée est de préférence de 20 à 200 nm, notamment de 40 à 150 nm et en particulier de 50 à 120 nm. Ces diamètres sont mesurés par diffusion de la lumière. On peut également obtenir la taille de gouttelettes par microscopie électronique en transmission (TEM), par cryomicroscopie électronique en transmission (cryoTEM) ou encore par microscopie à force atomique (AFM). Des diamètres inférieurs à 20 nm et supérieurs à 200 nm sont difficiles à atteindre en pratique.

Le terme « lipide » désigne dans le cadre de cet exposé l'ensemble des corps gras ou des substances contenant des acides gras présents dans les graisses d'origine animales et dans les huiles végétales. Ce sont de molécules hydrophobes ou amphiphiles principalement constituées de carbone, d'hydrogène et d'oxygène et ayant une densité inférieure à celle de l'eau. Les lipides peuvent être à l'état solide à température ambiante (25°C), comme dans les cires, ou liquide, comme dans les huiles.

Le terme «amphiphile» désigne une molécule possédant une partie hydrophobe et une partie hydrophile, par exemple une partie apolaire hydrophobe et une partie polaire hydrophile.

Le terme « phospholipide » vise des lipides possédant un groupe phosphate, notamment les phosphoglycérides. Le plus souvent, les phospholipides comportent une extrémité hydrophile formée par le groupe phosphate éventuellement substitué et deux extrémités hydrophobes formées par des chaînes d'acides gras. Parmi les phospholipides, on citera en particulier la phosphatidylcholine, la phosphatidyl éthanolamine, la phosphatidyl inositol, la phosphatidyl sérine et la sphingomyéline.

Le terme « lécithine » désigne la phosphatidylcholine, c'est-à-dire un lipide formé à partir d'une choline, d'un phosphate, d'un glycérol et de deux acides gras. Il couvre de manière plus large les phospholipides extraits du vivant, d'origine végétale ou animale, dans la mesure où ils sont majoritairement constitués de phosphatidylcholine. Ces lécithines constituent généralement des mélanges de lécithines portant différents acides gras.

On entend par le terme « tensioactif » des composés à structure amphiphile qui leur confère une affinité particulière pour les interfaces de type huile/eau et eau/huile ce qui leur donne la capacité d'abaisser l'énergie libre de ces interfaces et de stabiliser des systèmes dispersés.

On entend par le terme « co-tensioactif » un tensioactif agissant en plus d'un tensioactif pour abaisser davantage l'énergie de l'interface.

On entend par agent d'intérêt « lipophile », un agent d'intérêt qui se situe majoritairement, de préférence totalement, dans la phase dispersée, à l'intérieur ou en surface des gouttelettes. Un agent d'intérêt lipophile a des affinités pour des composés huileux (graisses, huiles, cires...) et solvants apolaires (toluène, hexane...). Les forces permettant la solubilisation de l'agent d'intérêt lipophile sont majoritairement des forces de London (interactions de Van der Waals). Un agent d'intérêt lipophile présente un coefficient de partage huile/eau élevé.

On entend par agent d'intérêt « hydrophile », un agent d'intérêt qui se situe majoritairement, de préférence totalement, dans la phase aqueuse continue. Sa solubilité dans l'eau est généralement supérieure à 1% en poids. La solubilisation dans l'eau des agents d'intérêt hydrophile provient généralement de liaisons hydrogène et/ou ioniques entre les agents d'intérêt hydrophile et l'eau.

On entend par le terme « acide gras » désigner des acides carboxyliques aliphatiques présentant une chaîne carbonée d'au moins 4 atomes de carbone[]. Les acides gras naturels possèdent une chaîne carbonée de 4 à 28 atomes de carbone (généralement un nombre pair). On parle d'acide gras à longue chaîne pour une longueur de 14 à 22 carbones et à très longue chaîne s'il y a plus de 22 carbones.

On entend par le terme « chaîne hydrocarbonée » désigner une chaîne composée d'atomes de carbone et d'hydrogène, saturée ou insaturée (double ou triple liaison). Les chaînes hydrocarbonées préférées sont les alkyle ou alcényle.

On entend par le terme « alkylène » désigner un groupe divalent aliphatique hydrocarboné, saturé, linéaire ou ramifié, de préférence linéaire.

Par « ester activé », on entend un groupe de formule -CO-LG, et par « carbonate activé », on entend un groupe de formule -O-CO-LG où LG est un bon groupe partant notamment choisi parmi un brome, un chlore, un imidazolyle, un pentafluorophénolate, un pentachlorophénolate, un 2,4,5-trichlorophénolate, 2,4,6-trichlorophénolate, un groupe -O-succinimidyle, -O-benzotriazolyle, -O-(7-aza-benzotriazolyle) et -O-(4-nitrophényle).

### • Tensioactif de formule (I)

Le matériau selon l'invention comprend un tensioactif de formule (I) qui se situe partiellement dans la phase aqueuse continue et partiellement dans la phase dispersée. En effet, le tensioactif de formule (I) du matériau selon l'invention comprend deux groupes lipophiles (L₁ et L₂) et deux groupes hydrophiles (H₁ et H₂). Les groupes hydrophiles sont situés majoritairement à la surface des gouttelettes, dans la phase aqueuse continue alors que les groupes lipophiles se situent dans les gouttelettes du matériau.

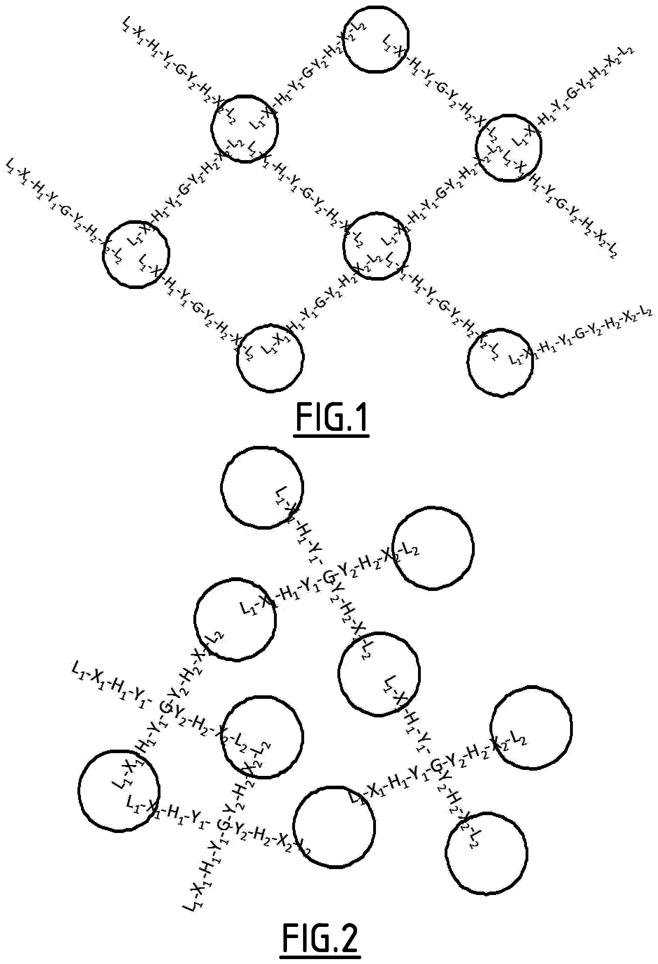

Plus précisément, le groupe lipophile L₁ se situe dans certaines gouttelettes, et le groupe L₂ dans des gouttelettes adjacentes. Les gouttelettes du matériau selon l'invention sont donc reliées de façon covalente entre-elles par le groupe -(X₁-H₁-Y₁)ᵥ-G-(Y₂-H₂₋X₂)_{w}-du tensioactif de formule (I). Les figures 1 et 2 illustrent le positionnement du tensioactif de formule (I) respectivement lorsque v et w représentent 1 (selon l'invention) et lorsque v et w représentent 2 (illustratif).

Les groupes X₁ et X₂ sont des groupes de liaison liant les groupes lipophile et hydrophobe. Le groupe G est un groupe de liaison entre au moins deux parties [lipophile-hydrophile] du tensioactif de formule (I). Les groupes Y₁ et Y₂ sont des groupes de liaison liant le groupe G aux parties [lipophile-hydrophile].

Le matériau selon l'invention peut avantageusement être mis en forme (par exemple en le plaçant dans un moule ou un récipient ayant une forme donnée), et rester dans la forme désirée selon l'application souhaitée.

D'autre part le matériau résiste à la dilution à une phase aqueuse. Plus précisément, lorsqu'une phase aqueuse est ajoutée au matériau selon l'invention, celui-ci reste en forme et ne se dilue pas. Dans le milieu, on observe d'une part le matériau comprenant les gouttelettes, et d'autre part une phase aqueuse essentiellement exempte de gouttelettes.

Sans vouloir être liés à une théorie particulière, il semble que ces propriétés du matériau selon l'invention puissent être expliquées par la présence des liaisons covalentes entre les gouttelettes, qui confèrent une cohésion très forte entre elles.

Dans la formule (I) susmentionnée, G comprend au moins un groupe G' ayant l'une des formules suivantes (les groupes Y₁ et Y₂ étant reliés à gauche et à droite des formules décrites ci-dessous): 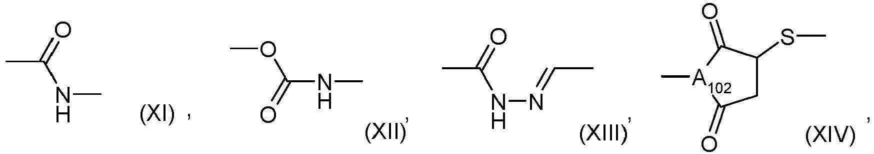 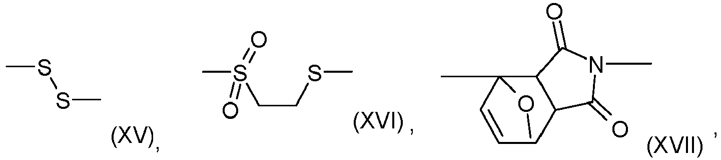 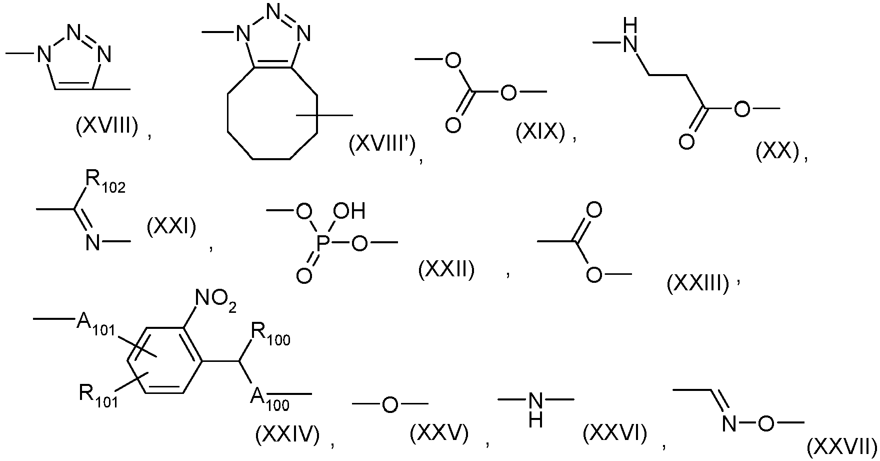 où A₁₀₂ représente CH ou N, R₁₀₂ représente H ou une chaîne hydrocarbonée linéaire comprenant de 1 à 6 atomes de carbone, A₁₀₁ représente -O-, -NH-(CO)- ou -O-(CO)-, R₁₀₀ représente H ou un méthyle, A₁₀₀ représente -O- ou -NH- et R₁₀₁ représente H, Me ou -OMe.

Par la formule 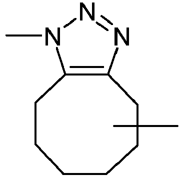 , on entend que le groupe Y₂ peut être relié à n'importe lequel des six atomes du cyclooctyle et par la formule 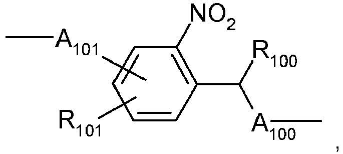 on entend que les groupe A₁₀₁ et R₁₀₁ peuvent être relié à n'importe lequel des quatre atomes du phényle.
v et w représentent 1.

Le groupe G peut comprendre un ou plusieurs des groupes G' définis ci-dessus.

Ainsi, dans un premier mode de réalisation, le groupe G est constitué d'un groupe G'. Dans un second mode de réalisation, le groupe G répond à la formule -G'-Y₃-G'- dans laquelle :
- Y₃ représente un groupe de liaison, notamment choisi parmi :
   - une liaison simple,
   - un groupe Z choisi parmi -O-, -NH-, -O(OC)-, -(CO)O-, -(CO)NH-, -NH(CO)-, -O-(CO)-O-, -NH-(CO)-O-, -O-(CO)-NH- et -NH-(CO)-NH,
   - un groupe Alk étant un alkylène comprenant de 1 à 6 atomes de carbone, et
   - un groupe Z-Alk, Alk-Z, Alk-Z-Alk ou Z-Alk-Z où Alk et Z sont tels que définis ci-dessus et où les deux groupes Z du groupe Z-Alk-Z sont identiques ou différents
- chacun des G' représente indépendamment un groupe de formule (XI) à (XXVI) susmentionnés, et de préférence, les deux groupes G' de la formule -G'-Y₃-G'- sont identiques.

Ce mode de réalisation est particulièrement intéressant lorsque les deux groupes G' sont identiques et comprennent une fonction clivable. En effet, il suffit alors de cliver une seule des deux fonctions pour rompre les liaisons covalentes entre les gouttelettes du matériau.

De préférence, L₁ et L₂ sont identiques et/ou X₁ et X₂ sont identiques et./ou H₁ et H₂ sont identiques. Des tensioactifs de formule (I) particulièrement préférés sont ceux dans lesquels L₁ et L₂ sont identiques, X₁ et X₂ sont identiques et.H₁ et H₂ sont identiques. Ces tensioactifs sont en effet des composés symétriques et sont donc généralement plus faciles à synthétiser, et donc moins coûteux.

Dans la formule (I) susmentionnée, les radicaux L₁-X₁-H₁- et L₂-X₂-H₂- consistent en un groupe de formule suivante (le groupe Y₁ ou Y₂ étant relié à droite de la formule décrite ci-dessous): 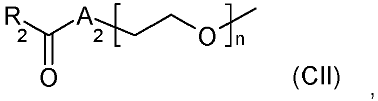 dans laquelle :
- R₂ représente une chaîne hydrocarbonée linéaire comprenant de 11 à 23 atomes de carbone,
- A₂ représente O ou NH,
- n représente un nombre entier de 3 à 500, de préférence 20 à 200.

Les radicaux L₁-X₁-H₁-et/ou L₂-X₂-H₂- de formule (CII) sont faciles à préparer (notamment par formation d'un ester ou d'une amide entre un acide gras et un dérivé de poly(éthylène glycol). De plus, un matériau comprenant un tensioactif comprenant un radical L₁-X₁-H₁-et/ou L₂-X₂-H₂- de formule (CII) peut généralement être préparé avec une quantité plus importante de ce tensioactif qu'un matériau comprenant un tensioactif comprenant un radical L₁-X₁-H₁-et/ou L₂-X₂-H₂- de formule (CIII) suivante : 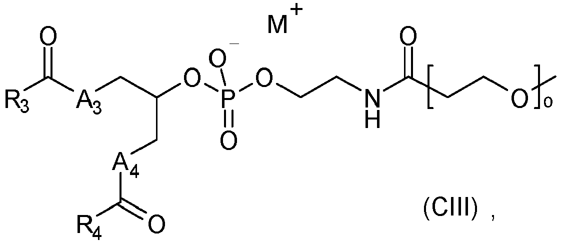 dans laquelle:
- R₃ et R₄ représentent indépendamment une chaîne hydrocarbonée linéaire comprenant de 11 à 23 atomes de carbone,
- A₃ et A₄ représentent O ou NH,
- o représente un nombre entier de 3 à 500, de préférence 20 à 200, et
- M représente H ou un cation.

Or, plus la proportion de tensioactif de formule (I) dans le matériau est élevée, plus la cohésion entre les gouttelettes est grande, et plus le matériau reste en forme et résiste à la dilution. Ainsi, ces deux propriétés peuvent être encore exacerbées pour un matériau comprenant un tensioactif comprenant un radical L₁-X₁-H₁-et/ou L₂-X₂-H₂- de formule (CII).

Les radicaux L₁-X₁-H₁-et/ou L₂-X₂-H₂- de formule (CII) avec A₂ représente NH sont particulièrement préférés, car les tensioactifs comprenant de tels radicaux permettent d'éviter la fuite des agents d'intérêts lipophiles éventuellement présents hors des gouttelettes du matériau plus efficacement que des tensioactifs comprenant des radicaux L₁-X₁-H₁-et/ou L₂-X₂-H₂- de formule (CII) avec A₂ représente O.

Dans un mode de réalisation, dans la formule (I), G est constitué d'un groupe G' représentant -S-S- (groupe de formule (XV) ci-dessus) et Y₁ et Y₂ représentent -CH₂-CH₂-NH-CO-CH₂-CH₂- (groupe Alk-Z-Alk ci-dessus avec Alk représente -CH₂-CH₂- et Z représente -NH-(CO)-) et le tensioactif du matériau a alors la formule (I') suivante : 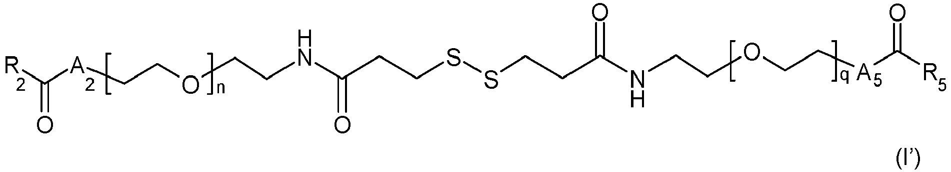 dans laquelle :
- R₂ et R₅ représentent indépendamment une chaîne hydrocarbonée linéaire comprenant de 11 à 23 atomes de carbone, de préférence 17,
- A₂ et A₅ représentent O ou NH, de préférence NH, et
- n et q représentent indépendamment des nombres entiers de 3 à 500, de préférence de 20 à 200.

Dans un mode de réalisation, les groupes H₁ et H₂ sont indépendamment choisis parmi un poly(oxyde d'éthylène) comprenant plus de 3 unités de poly(oxyde d'éthylène), voire plus de 20 unités, notamment plus de 50 (dans les formules susmentionnées, n et/ou q sont de préférence supérieurs à 3, voire 20, notamment plus de 50).

Dans un mode de réalisation, le groupe G du tensioactif de formule (I) du matériau comprend une fonction clivable, notamment chimiquement (lorsque le tensioactif de formule (I) est mis en contact avec un composé chimique capable de cliver la fonction du groupe G), électrochimiquement, à certains pH (pH basique ou acide), par des enzymes, par la lumière (lumière visible, ultraviolets ou infrarouge) et/ou au-delà de certaines températures. Généralement, le groupe G comprend alors un groupe G' comprenant une fonction clivable.

Par exemple :
- la fonction β-cétoaminoester du groupe G' de formule (XX) est clivable à pH acide (typiquement autour de 5),
- la fonction disulfure du groupe G' de formule (XV) est clivable aux ultraviolets, électrochimiquement, chimiquement (par exemple par mise en contact avec un agent réducteur, tel que la tris(2-carboxyéthyl)phosphine (TCEP) ou le dithiothréitol (DTT)), ou par des enzymes telles que des thioréductases,
- la fonction amide du groupe G' de formule (XI) est clivable par des enzymes telles que des protéases,
- la fonction phosphate du groupe G' de formule (XXII) est clivable par des enzymes telles que des phosphatases,
- la fonction imine des groupes G' de formules (XXI) et (XIII) sont clivables à pH acide ou au-delà de certaines températures,
- le cycle cyclohexène du groupe G' de formule (XVII) est clivable au-delà de certaines températures (par rétro Diels-Alder),
- la fonction carbonate du groupe G' de formule (XIX) et la fonction carbamate du groupe G de formule (XII) sont clivables à pH acide ou chimiquement (par exemple par réaction avec un agent nucléophile),
- la fonction orthonitrobenzyle du groupe G' de formule (XXIV) est clivable sous l'action de la lumière à 365 nm.

L'homme du métier, au vu de ses connaissances générales, sait quelles fonctions sont clivables et dans quelles conditions. Il est notamment à même de choisir la fonction du groupe G' du tensioactif de formule (I) pour qu'elle soit clivable dans les conditions rencontrées dans l'application souhaitée du matériau selon l'invention.

Dans un mode de réalisation, le clivage de la fonction clivable du groupe G du tensioactif de formule (I) du matériau est réversible, c'est-à-dire que la fonction est susceptible de se reformer pour recréer des liaisons covalentes entre les gouttelettes. L'homme du métier connait les réactions chimiques réversibles et les conditions de clivage et de reformation des liaisons. A titre purement illustratif, par exemple, la fonction disulfure du groupe G' de formule (XV) est clivable par mise en contact avec un agent réducteur et peut être reformée par mise en contact avec un agent oxydant.

### • Lipide amphiphile

Le matériau comprend un lipide amphiphile à titre de tensioactif qui permet la formation des gouttelettes de la phase dispersée. La nature amphiphile du tensioactif assure la stabilisation des gouttelettes d'huile au sein de la phase continue aqueuse.

Les lipides amphiphiles comportent une partie hydrophile et une partie lipophile. Ils sont généralement choisis parmi les composés dont la partie lipophile comprend une chaîne saturée ou insaturée, linéaire ou ramifiée, ayant de 8 à 30 atomes de carbone. Il s'agit de phospholipide.

Les phospholipides sont notamment choisis parmi la phosphatidylcholine, la phosphatidyléthanolamine, la phosphatidylsérine, le phosphatidylglycérol, le phosphatidylinositol, le phosphatidyl-acide phosphatidique non-hydrogéné ou hydrogéné, notamment vendu par la société Lipoid.

La lécithine est le lipide amphiphile préféré.

Généralement, la phase huileuse comportera de 0,01 à 99% en poids, de préférence de 5 à 75% en poids, en particulier de 10 à 60% et tout particulièrement de 15 à 45% en poids de lipide amphiphile.

La quantité de lipide amphiphile contribue avantageusement à contrôler la taille des gouttelettes de la phase dispersée du matériau.

### • Lipide solubilisant

Le matériau selon l'invention comprend un lipide solubilisant, qui permet notamment :
- d'augmenter la stabilité physicochimique du matériau, et
- lorsque le matériau comprend un agent d'intérêt lipophile encapsulé dans les gouttelettes:
   - de solubiliser l'agent d'intérêt lipophile, et
   - d'améliorer le contrôle du relargage de l'agent d'intérêt lipophile.

De préférence, le lipide solubilisant est solide à température ambiante (20°C).

Les lipides solubilisants sont les glycérides d'acides gras, notamment d'acides gras saturés, et en particulier d'acides gras saturés comportant 8 à 18 atomes de carbone, encore préféré 12 à 18 atomes de carbone. Avantageusement, le lipide solubilisant est constitué d'un mélange complexe de différents glycérides. Par « mélange complexe », on entend un mélange de mono, di et triglycérides, comprenant des chaînes grasses de différentes longueurs, les dites longueurs s'étendant préférentiellement de C8 à C18, par exemple, en association, des chaînes en C8, C10, C12, C14, C16 et C18, ou de C10 à C18, comprenant par exemple en association, chaînes en C10, C12, C14, C16 et C18.

Selon un mode de réalisation, lesdites chaînes grasses peuvent contenir une ou plusieurs insaturations.

De préférence, le lipide solubilisant est constitué d'un mélange de glycérides d'acides gras saturés comportant au moins 10% en poids d'acides gras en C12, au moins 5% en poids d'acides gras en C14, au moins 5% en poids d'acides gras en C16 et au moins 5% en poids d'acides gras en C18.

De préférence, le lipide solubilisant est constitué d'un mélange de glycérides d'acides gras saturés comportant 0% à 20% en poids d'acides gras en C8, 0% à 20% en poids d'acides gras en C10, 10% à 70% en poids d'acides gras en C12, 5% à 30% en poids d'acides gras en C14, 5% à 30% en poids d'acides gras en C16 et 5% à 30% en poids d'acides gras en C18.

Les mélanges des glycérides semi-synthétiques solides à température ambiante vendus sous la dénomination commerciale Suppocire^{®}NB par la société Gattefossé et approuvé pour un usage chez l'homme sont des lipides solubilisants particulièrement préférés. Les Suppocire^{®} de type N sont obtenues par estérification directe d'acides gras et de glycérol. Il s'agit de glycérides hémi-synthétiques d'acides gras saturés de C8 à C18, dont la composition quali-quantitative est indiquée dans le tableau ci-dessous.

**Tableau : Composition en acides gras du Suppocire^{®} NB de Gattefossé**

| Longueur de chaînes | [% en poids] |
|---|---|
| C8 | 0,1 à 0,9 |
| C10 | 0,1 à 0,9 |
| C12 | 25 à 50 |
| C14 | 10 à 24,9 |
| C16 | 10 à 24,9 |
| C18 | 10 à 24,9 |

Les lipides solubilisants précités permettent d'obtenir un matériau avantageusement stable. Sans vouloir être lié à une théorie particulière, il est supposé que les lipides solubilisants précités permettent d'obtenir dans le matériau des gouttelettes présentant un coeur amorphe. Le coeur ainsi obtenu présente une viscosité interne élevée sans pour autant présenter de cristallinité. Or, la cristallisation est néfaste pour la stabilité du matériau car elle conduit généralement à une agrégation des gouttelettes et/ou à une expulsion de l'agent d'intérêt lipophile, si présent, à l'extérieur des gouttelettes. Ces propriétés physiques favorisent la stabilité physique du matériau.

La quantité de lipide solubilisant peut varier largement en fonction de la nature et de la quantité de lipide amphiphile présent dans la phase dispersée. Généralement, la phase dispersée comportera de 1 à 99% en poids, de préférence de 5 à 80% en poids et tout particulièrement de 30 à 75% en poids de lipide solubilisant.

### • Huile

La phase dispersée du matériau selon l'invention comporte également une ou plusieurs huiles.

Les huiles utilisées présentent de préférence une balance hydrophile-lipophile (HLB) inférieure à 10 et encore plus préférentiellement comprise entre 3 et 9. Avantageusement, les huiles sont utilisées sans modification chimique ou physique préalablement à la formation du matériau.

Selon les applications envisagées, les huiles peuvent être choisies parmi les huiles biocompatibles, et en particulier parmi les huiles d'origine naturelle (végétale ou animale) ou synthétique. Parmi de telles huiles, on peut notamment citer les huiles d'origine naturelle végétale parmi lesquelles figurent notamment les huiles de soja, de lin, de palme, d'arachide, d'olives, de sésame, de pépin de raisins et de tournesol ; les huiles synthétiques parmi lesquelles figurent notamment les triglycérides, di glycérides et les mono glycérides. Ces huiles peuvent être de premières expressions, raffinées ou interestérifiées.

L'huile préférée est l'huile de soja.

Généralement, l'huile sera contenue dans la phase dispersée dans une proportion allant de 1 à 80% en poids, de préférence entre 5 et 50 % en poids et tout particulièrement 10 à 30% en poids par rapport au poids total de la phase huileuse.

### • Phase aqueuse

La phase aqueuse continue du matériau selon l'invention est de préférence constituée d'eau et/ou d'un tampon tel qu'un tampon phosphate comme par exemple du PBS ("Phosphate Buffer Saline") ou d'une solution saline, notamment de chlorure de sodium. Généralement, le pH de la phase aqueuse est de l'ordre du pH physiologique.

Selon un mode de réalisation, la phase continue comporte également un agent épaississant tel que du glycérol, un saccharide, oligosaccharide ou polysaccharide, une gomme ou encore une protéine, de préférence du glycérol. En effet, l'utilisation d'une phase continue de viscosité plus élevée facilite l'émulsification et permet de ce fait de réduire le temps de sonication.

La phase aqueuse comporte avantageusement de 0 à 50% en poids, de préférence de 1 à 30% en poids et tout particulièrement de 5 à 20% en poids d'agent épaississant.

Bien entendu, la phase aqueuse peut contenir en outre d'autres additifs tels que des colorants, stabilisants et conservateurs en quantité appropriée.

### • Co-tensioactif

Le matériau selon l'invention comprend également un co-tensioactif. Ce co-tensioactif se situe partiellement dans la phase aqueuse continue et partiellement dans les gouttelettes de la phase dispersée.

Le co-tensioactif comporte au moins une chaîne composée de motifs d'oxyde d'éthylène ou d'oxyde d'éthylène et d'oxyde de propylène. Il peut être choisi en particulier parmi les composés conjugués polyéthylèneglycol/phosphatidyl-éthanolamine (PEG-PE), les éthers d'acide gras et de polyéthylèneglycol, les esters d'acide gras et de polyéthylèneglycol et les copolymères blocs d'oxyde d'éthylène et d'oxyde de propylène.

La chaîne polyalcoxylée du co-tensioactif comprend généralement de 10 à 200, typiquement de 10 à 150, notamment de 20 à 100, de préférence de 30 à 80, motifs oxyde d'éthylène/oxyde de propylène. En dessous de 10 motifs, le matériau est inhomogène car la phase dispersée comprend des gouttelettes polydisperses, et ne permettant pas le contrôle du temps de libération de l'agent d'intérêt lipophile s'il est présent. Au-delà de 200 motifs, d'une part l'émulsion est inhomogène car la phase dispersée comprend des gouttelettes polydisperses, ne permettant pas le contrôle du temps de libération de l'agent d'intérêt lipophile.

A titre d'exemple de co-tensioactifs, on peut en particulier citer les composés conjugués à base de polyéthylèneglycol/phosphatidyl-éthanolamine (PEG-PE), les éthers d'acide gras et de polyéthylèneglycol tels que les produits vendus sous les dénominations commerciales Brij^{®} (par exemple Brij^{®} 35, 58, 78 ou 98) par la société ICI Americas Inc., les esters d'acide gras et de polyéthylèneglycol tels que les produits vendus sous les dénominations commerciales Myrj^{®} par la société ICI Americas Inc. (par exemple Myrj^{®} s20, s40 ou s100, anciennemnent nommés 49, 52 ou 59) et les copolymères blocs d'oxyde d'éthylène et d'oxyde de propylène tels que les produits vendus sous les dénominations commerciales Pluronic^{®} par la société BASF AG (par exemple Pluronic^{®} F68, F127, L64, L61, 10R4, 17R2, 17R4, 25R2 ou 25R4) ou les produits vendus sous la dénomination commerciale Synperonic^{®} par la société Unichema Chemie BV (par exemple Synperonic^{®} PE/F68, PE/L61 ou PE/L64).

De préférence, le rapport de la masse de tensioactif de formule (I) sur la masse de l'ensemble (tensioactif de formule (I) / co-tensioactif) est supérieur ou égal à 15%. Il a en effet été observé que de tels matériaux sont plus faciles à préparer.

La phase aqueuse comporte généralement de 0,01 à 50% en poids, de préférence de 1 à 30% en poids et tout particulièrement de 5 à 20% en poids de co-tensioactif.

Généralement, la fraction massique de l'ensemble [co-tensioactif/lipide amphiphile/tensioactif de formule (I)] par rapport au poids total du coeur des gouttelettes [huile / lipide solubilisant/ co-tensioactif/lipide amphiphile/ éventuel(s) agent(s) d'intérêt lipophile(s)] est inférieur ou égal 2, de préférence inférieure ou égale à 1. Ceci permet d'obtenir un système physiquement stable, ne subissant pas les effets de la déstabilisation due au mûrissement d'Ostwald ou à la coalescence (séparation des phases aqueuse et huileuse).

Généralement, la fraction massique de lipide amphiphile par rapport au poids de co-tensioactif est de 0,005 % à 100 %, notamment de 0,01 % à 50 %, de préférence de 0,1 % à 30 %. En effet, en dessous de 0,005 % et au delà de 100 %, les gouttelettes de la phase dispersée ne sont souvent pas suffisamment stables et coalescent en quelques heures et il est souvent difficile d'obtenir des gouttelettes de diamètre inférieur à 200 nm.

Généralement, le matériau ne comporte pas de tensioactifs supplémentaires : les seuls tensioactifs du matériau sont le lipide amphiphile, le co-tensioactif et le tensioactif de formule (I).

Dans un mode de réalisation, le co-tensioactif polyalcoxylé comporte un composé d'intérêt greffé. Typiquement, le composé d'intérêt a été greffé par liaison chimique, généralement covalente, au co-tensioactif tel que défini ci-dessus. Le greffage peut être réalisé avant ou après la formation des émulsions utilisées pour préparer le matériau (émulsions 1 et 2 ci-après). Le dernier cas peut être préconisé lorsque les réactions chimiques employées sont compatibles avec la stabilité des émulsions, notamment en termes de pH. De préférence, le pH lors de la réaction de greffage est compris entre 5 et 11.

Généralement, ce greffage a été effectué à une extrémité de la ou des chaînes polyalcoxylée(s) du co-tensioactif, et le composé d'intérêt est ainsi situé à la surface des gouttelettes de la phase dispersée du matériau.

Les composés d'intérêt peuvent être par exemple :
- des ligands biologiques de ciblage tels que des anticorps, peptides, saccharides, aptamères, oligonucléotides ou des composés comme l'acide folique ; lors de la libération des gouttelettes du matériau, ce ligand biologique sera reconnu de manière spécifique par certaines cellules (par exemple de cellules tumorales comme décrit par exemple dans l'article de S. Achilefu, Technology in Cancer Research & Treatment, 2004, 3, 393-408) ou certains organes que l'on souhaite cibler, ce qui permet de contrôler la localisation de la libération de l'éventuel agent d'intérêt lipophile ;
- un agent de furtivité : une entité ajoutée afin de conférer au matériau une furtivité visà-vis du système immunitaire, d'augmenter son temps de circulation dans l'organisme, et de ralentir son élimination.

### • Agent d'intérêt

Dans un mode de réalisation, le matériau peut comprendre un ou plusieurs agent(s) d'intérêt.

L'agent d'intérêt peut être de nature très variée selon l'application souhaitée du matériau. Ainsi, l'agent d'intérêt peut être :
- un agent capteur chimique (« scavenger » en anglais), c'est à dire un composé susceptible de réagir avec un composé chimique que l'on souhaite éliminer d'un milieu. Le capteur chimique incorporé dans le matériau selon l'invention est choisi selon le composé que l'on souhaite éliminer. Par exemple, un capteur chimique peut réagir avec des impuretés et peut être ainsi être utilisé pour purifier ou dépolluer un milieu. Dans un autre exemple, lors d'une réaction chimique, un capteur chimique peut être utilisé pour éliminer des produits de réaction secondaire ou un excès de réactif.
- un agent détecteur chimique, c'est-à-dire un composé chimique susceptible d'émettre un signal lorsqu'il est mis en contact avec un analyte que l'on souhaite détecter et/ou quantifier. Le détecteur chimique peut être un détecteur fluorogène (la mise en contact du détecteur fluorogène et de l'analyte produit une émission fluorescente détectable) ou un détecteur chromogène (la mise en contact du détecteur chromogène et de l'analyte produit un changement de couleur détectable). L'analyte peut être de toute nature, par exemple une bactérie (comme dans la demande FR 2 954 911), un métal, un polluant... Le détecteur chimique incorporé dans le matériau selon l'invention est choisi selon l'analyte que l'on souhaite détecter. A titre purement illustratif, pour détecter des métaux lourds, un dérivé de quinoléine peut être incorporé dans les gouttelettes du matériau selon l'invention à titre de détecteur fluorogène (Da-Yu Wu et al., Dalton Trans., 2006, 3528-3533).

- un catalyseur, tel qu'un catalyseur métallique ou organométallique.
- un agent optique tel que un colorant, un chromophore, un fluorophore, par exemple le perchlorate 1,1'-dioctadecyl 3,3,3',3'-tetramethylindodicarbocyanine (DiD), le iodure de 1,1'-dioctadecyl 3,3,3',3'-tetramethylindotricarbocyanine (DiR), vert d'indocyanine (ICG), ou encore des composants ayant des propriétés optoélectronique, tels que les saturants ou les absorbants optiques.
- un agent phytosanitaire, tel qu'une substance minérale (ex : sulfate de cuivre) ou organique (ex : carbamate de type carbofuran, furadan...), naturelle( ex : Bacillus thuringiènsis Bt) ou issue de la chimie de synthèse (ex : glyphosate).
- un agent de masquage de goût/odeur, tel qu'une substance gustative et/ou odorante, comme le menthol ou la cinnamaldéhyde, pour un usage pharmaceutique (galénique) ou agroalimentaire.
- un agent cosmétique,
- un agent thérapeutique, tel qu'un principe actif pharmaceutique ou un agent physique, tel qu'un isotope radioactif ou un photo-sensibilisateur.
   - Les agents thérapeutiques susceptibles d'être compris dans le matériau selon l'invention comprennent en particulier les principes actifs agissant par voie chimique, biologique ou physique. Ainsi, il peut s'agir de principes actifs pharmaceutiques ou d'agents biologiques tels que de l'ADN, des protéines, peptides ou anticorps encore des agents utiles pour des thérapies physiques tels que des composés utiles pour la thermothérapie, les composés relarguant de l'oxygène singulet lorsqu'ils sont excités par une lumière utiles pour la photothérapie et des agents radioactifs.

Parmi les principes actifs pharmaceutiques intéressants comme agents thérapeutiques, on peut citer en particulier les agents utilisés dans le traitement du SIDA, les agents utilisés dans le traitement des maladies cardiaques, les analgésiques, les anesthésiques, les anorexigènes, les anthelmintiques, les antiallergiques, les antiangineux, les antiarythmisants, les anticholinergiques, les anticoagulants, les antidépresseurs, les antidiabétiques, les antidiurétiques, les antiémétiques, les anticonvulsivants, les antifongiques, les antihistaminiques, les antihypertenseurs, les anti-inflammatoires, les anti-migraineux, les antimuscariniques, les antimycobactériens, les anticancéreux y compris les antiparkinsoniens, les antithyroïdiens, les antiviraux, les astringents, les agents bloquants, les produits sanguins, les substituts sanguins, les agents inotropes cardiaques, les agents cardiovasculaires, les agents du système nerveux central, les chélateurs, les agents de chimiothérapie, les facteurs de croissance hématopoïétiques, les corticostéroïdes, les antitussifs, les agents dermatologiques, les diurétiques, les dopaminergiques, les inhibiteurs de l'élastase, les agents endocrines, les alkaloïdes de l'ergot, les expectorants, les agents gastro-intestinaux, les agents génito-urinaires, le facteur de déclenchement de l'hormone de croissance, les hormones de croissance, les agents hématologiques, les agents hématopoïétiques, les hemostatiques, les hormones, les agents immunologiques, les immunosuppresseurs, les interleukines, les analogues d'interleukines, les agents de régulation des lipides, la gonadolibérine, les myorelaxants, les antagonistes narcotiques, les nutriments, les agents nutritifs, les thérapies oncologiques, les nitrates organiques, les vagomimétiques, les prostaglandines, les antibiotiques, les agents rénaux, les agents respiratoires, les sédatifs, les hormones sexuelles, les stimulants, les sympathomimétiques, les anti-infectieux systémiques, le tacrolimus, les agents thrombolytiques, les agents thyroïdiens, les traitements pour les troubles de l'attention, les vaccins, les vasodilatateurs, les xanthines, les agents diminuant le cholestérol, les cicatrisants. Parmi les principes actifs agissant par voie biologique, on peut mentionner les oligonucléotides, de l'ADN, de l'ARN, les SiRNA, les microRNA, les peptides et les protéines.

Bien entendu, les agents thérapeutiques peuvent être formulés directement sous leur forme active ou sous forme de prodrug.
- Parmi les agents physiques, on peut citer notamment les isotopes radioactifs et les photo-sensibilisateurs.

Parmi les photo-sensibilisateurs, on peut citer notamment ceux appartenant à la classe des tétrapyrroles comme les porphyrines, les bactériochlorines, les phtalocyanines, les chlorines, les purpurines, les porphycènes, les phéophorbides, ou encore ceux appartenant à la classe des texaphyrines ou des hypericines. On peut également citer les dérivés de l'acide 5-aminolévulique et ses dérivés d'esters, ces composants étant connus comme précurseurs métabolique de la Protoporphyrine IX. Parmi les photo-sensibilisateurs de première génération, on peut mentionner l'hémato-porphyrine et un mélange de dérivés d'hématoporphyrine (HpD) (vendu sous la marque commerciale Photofrin^{®} par Axcan Pharma). Parmi les photo-sensibilisateurs de seconde génération, on peut mentionner le méta-tetra-hydroxyphenyl chlorine (mTHPC ; nom commercial Foscan^{®}, Biolitec AG) et le dérivé monoacide du cycle A de la benzoporphyrine (BPD-MA vendu sous la marque commerciale Visudyne^{®} par QLT et Novartis Opthalmics). Les formulations des photo-sensibilisateurs de seconde génération qui associent à ces photo-sensibilisateurs une molécule (lipide, peptide, sucre etc..) qualifiée de transporteur qui permet leur acheminement sélectif au niveau du tissu tumoral sont appelées photo-sensibilisateurs de troisième génération.

Par ailleurs, la phase huileuse peut, en plus de l'agent thérapeutique, également comporter un agent d'imagerie, notamment pour l'IRM (imagerie par résonance magnétique), le PET (en anglais Positron Emission Tomography), le SPECT (Single Photon Emission Computed Tomography), l'échographie ultrasonore, la radiographie, la tomographie X et l'imagerie optique (fluorescence, bioluminescence, diffusion...). Ces agents peuvent permettre de suivre la position des gouttelettes (et donc de l'agent thérapeutique) après administration du matériau au patient.

Les quantités d'agent d'intérêt dépendent de l'application visée ainsi que de la nature des agents.

Toutefois, lorsque les agents d'intérêt sont des agents thérapeutiques, on cherchera généralement à formuler le matériau avec une concentration maximale en agent thérapeutique, afin de limiter le volume et/ou la durée d'application, notamment le volume et/ou la durée d'administration au patient.

Or, il a été constaté que la présence du lipide solubilisant dans la phase huileuse permet d'incorporer une quantité importante d'agent d'intérêt lipophile. Le lipide solubilisant facilite en effet l'incorporation dans le coeur des gouttelettes des agents d'intérêt liposphiles. Les agents d'intérêt amphiphiles sont principalement incorporés dans la membrane des gouttelettes.

Le matériau selon l'invention contiendra le plus souvent une quantité de 0,001 à 30% en poids, de préférence 0,01 à 20% en poids, et encore préférée 0,1 à 10% en poids d'agent d'intérêt.

L'agent d'intérêt peut être hydrophile (il sera alors situé dans la phase aqueuse continue du matériau) ou lipophile (il sera alors encapsulé dans les gouttelettes formant la phase dispersée du matériau ou se situer à l'interface des phases aqueuses et huileuses sur la surface des gouttelettes, selon son affinité lipophile ou amphiphile).

Dans un mode de réalisation, le matériau comprend au moins un agent d'intérêt hydrophile et au moins un agent d'intérêt lipophile. Par exemple, le matériau comprend au moins un agent thérapeutique hydrophile et au moins un agent thérapeutique lipophile.

### • Autres propriétés du matériau

Grâce à sa formulation, le matériau selon l'invention est stable et présente une excellente stabilité au stockage (supérieure à 5 mois voire à 8 mois).

De plus, le matériau peut être formulé de manière à ce que la surface de la phase dispersée présente un potentiel zêta faible, idéalement compris entre -25 mV et + 25 mV, voire entre -20 et +20 mV, et en particulier entre -10 et +10 mV, voire nul. En effet, les chaînes polyalcoxylées du co-tensioactif et du tensioactif de formule (I), hydratées et non chargées, couvrant la surface des gouttelettes, écrantent les charges apportées par les lipides amphiphiles à la surface solide des gouttelettes. On se trouve donc dans le cas d'une stabilisation stérique des gouttelettes, et non une stabilisation électrostatique. Le potentiel zêta est un paramètre clé qui influe sur les interactions avec les milieux biologiques Les gouttelettes possédant une charge de surface très positive, c'est-à-dire supérieure à 25 mV, sont généralement plus cytotoxiques que des gouttelettes de potentiel zêta négatif ou neutre.

Par ailleurs, un faible potentiel zêta faible a comme avantage de limiter la reconnaissance non spécifique des gouttelettes par les macrophages lorsque le matériau se disperse, notamment *in vivo.*

Contrairement aux émulsions dont la stabilité repose sur des effets électrostatiques, lesquelles présentent un potentiel zêta élevé et peuvent être spontanément stables, les émulsions décrites dans le cadre de cette invention requièrent un apport d'énergie pour se former, par exemple la sonication. L'émulsion ainsi obtenue est alors métastable et comporte des gouttelettes dont la charge est faible ou nulle.

De préférence, le pH du matériau est compris entre 5,5 et 8,5, et de préférence entre 6 et 7,5. Un pH dans ces plages est compatible avec l'utilisation d'un tampon aqueux adapté au pH d'un milieu physiologique.

### [Procédé de préparation]

L'invention concerne un procédé de préparation du matériau défini ci-dessus, comprenant la mise en contact :
- d'une émulsion 1 comprenant une phase aqueuse continue et une phase dispersée sous forme de gouttelettes comprenant un lipide amphiphile et un tensioactif de formule (LI) suivante :

   L₁-X₁-H₁-Y₁-G₁ (LI),
- avec une émulsion 2 comprenant une phase aqueuse continue et une phase dispersée sous forme de gouttelettes comprenant un lipide amphiphile et un tensioactif de formule (LII) suivante :

   G₂-Y₂-H₂₋X₂-L₂ (LII)

   où L₁, X₁, H₁, Y₁, L₂, X₂, H₂ et Y₂ sont tels que définis ci-dessus, et G₁ et G₂ sont des
   groupes susceptibles de réagir pour former le groupe G tel que défini ci-dessus, dans des conditions permettant la réaction des tensioactifs de formules (LI) et (LII) pour former le tensioactif de formule (I) tel que défini ci-dessus, ce par quoi des liaisons covalentes entre les gouttelettes de la phase dispersée se forment.

Selon un mode de réalisation préféré de l'invention, la réaction de G1 et G2 pour former le groupe G est réalisé par irradiation du mélange formé par l'émulsion 1 et l'émulsion 2 par un rayonnement lumineux.

Lorsque le groupe G comprend un seul groupe G', les groupes G₁ et G₂ sont typiquement des groupes susceptibles de réagir l'un avec l'autre pour former le groupe G.

Lorsque le groupe G comprend plusieurs groupes G', on met généralement les émulsions 1 et 2 en contact avec un composé susceptible de réagir avec les tensioactifs de formules (LI) et (LII) pour former le groupe G. Ce composé comprend typiquement au moins v fonctions G'₁ susceptibles de réagir avec le groupe G₁ et w fonctions G'₂ susceptibles de réagir avec le groupe G₂.

Ainsi, dans le mode de réalisation dans lequel le groupe G répond à la formule -G'-Y₃-G'- définie ci-dessus, le procédé de préparation du matériau comprend typiquement la mise en contact :
- d'une émulsion 1 telle que définie ci-dessus,
- et d'une émulsion 2 telle que définie ci-dessus,
- avec un composé de formule G'₁-Y₃-G'₂ dans laquelle Y₃ est tel que défini ci-dessus, G'₁ est un groupe susceptible de réagir avec G₁ pour former le premier groupe G' tel que défini ci-dessus et G'₂ est un groupe susceptible de réagir avec G₂ pour former le second groupe G' tel que défini ci-dessus (de nature identique ou différente du premier groupe G'),
dans des conditions permettant la réaction des tensioactifs de formules (LI) et (LII) et du composé de formule G'₁-Y₃-G'₂ pour former le tensioactif de formule (I) dans lequel le groupe G répond à la formule -G'-Y₃-G'- définie ci-dessus, ce par quoi des liaisons covalentes entre les gouttelettes de la phase dispersée se forment.

### • Formation du tensioactif de formule (I) par réaction entre les tensioactifs de formules (LI) et (LII)

Au vu de ses connaissances générales en chimie, l'homme du métier est à même de choisir la nature des groupes G'₁, G'₂, Y₃, Y₄, G₁ et G₂ à utiliser pour former le groupe G et les conditions permettant la réaction. Les réactions de chimie organique usuelles peuvent être suivies, notamment celles décrites dans « Comprehensive Organic Transformations: A Guide to Functional Group Préparations » de Richard C. Larock édité par John Wiley & Sons Inc, et les références qui y sont citées. Ainsi, les exemples de groupes G₁ et G₂ ci-dessous sont cités à titre illustratif et non limitatif.

Typiquement, lorsque le groupe G est constitué d'un groupe G', les groupes G₁ et G₂ des tensioactifs de formules (LI) et (LII) peuvent par exemple être choisis comme suit :
- G₁ représente un thiol (-SH) et G₂ représente :
   - soit un maléimide, un tensioactif de formule (I) dans lequel G comprend un groupe G' représentant un groupe de formule (XIV) où A₁₀₂ représente N étant alors formé,
   - soit une vinylsulfone, un tensioactif de formule (I) dans lequel G comprend un groupe G' représentant un groupe de formule (XVI) étant alors formé,
   - soit un groupe -S-S-pyridinyle ou -SH, un tensioactif de formule (I) dans lequel G comprend un groupe G' représentant un groupe de formule (XV) étant alors formé,
- G₁ représente un hydroxyle et G₂ représente -COOH ou un ester activé, un tensioactif de formule (I) dans lequel G comprend un groupe G' représentant un groupe de formule (XXIII) étant alors formé,
- G₁ représente une amine -NH₂ et G₂ représente -COOH ou un ester activé, un tensioactif de formule (I) dans lequel G comprend un groupe G' représentant un groupe de formule (XI) étant alors formé,
- G₁ représente un hydroxyle et G₂ représente un carbonate activé, un tensioactif de formule (I) dans lequel G comprend un groupe G' représentant un groupe de formule (XIX) étant alors formé,
- G₁ représente une amine -NH₂ et G₂ représente un carbonate activé, un tensioactif de formule (I) dans lequel G comprend un groupe G' représentant un groupe de formule (XII) étant alors formé,
- G₁ représente une amine -NH₂ et G₂ représente un aldéhyde -CHO, un tensioactif de formule (I) dans lequel G comprend un groupe G' représentant un groupe de formule (XXI) étant alors formé,
- G₁ représente un hydrazide de formule -(C=O)-NH-NH₂ et G₂ représente un groupe - (C=O)-R10₂, un tensioactif de formule (I) dans lequel G comprend un groupe G' représentant un groupe de formule (XIII) étant alors formé,
- G₁ représente un alcyne et G₂ représente un azide, un tensioactif de formule (I) dans lequel G comprend un groupe G' représentant un groupe de formule (XVIII) étant alors formé,
- G₁ représente un cyclooctyne et G₂ représente un azide, un tensioactif de formule (I) dans lequel G comprend un groupe G' représentant un groupe de formule (XVIII') étant alors formé,
- G₁ représente un furane et G₂ représente un maléimide, un tensioactif de formule (I) dans lequel G comprend un groupe G' représentant un groupe de formule (XVII) étant alors formé,
- G₁ représente un aldéhyde et G₂ représente une amine, un tensioactif de formule (I) dans lequel G comprend un groupe G' représentant un groupe de formule (XXI) étant alors formé,
- G₁ représente un phosphate de formule -O-P(=O)(OH)₂ et G₂ représente un hydroxyle, un tensioactif de formule (I) dans lequel G comprend un groupe G' représentant un groupe de formule (XXII) étant alors formé,
- G₁ représente un bon groupe partant LG et G₂ représente un groupe de formule suivante 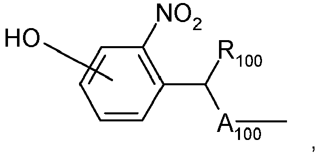 un tensioactif de formule (I) dans lequel G comprend un groupe G' représentant un groupe de formule (XXIV) dans laquelle A₁₀₁ représente O étant alors formé,
- G₁ représente un hydroxyle ou une amine -NH₂ et G₂ représente un groupe de formule suivante 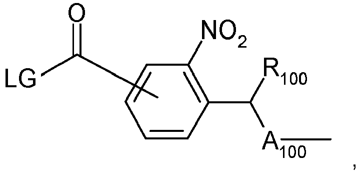 un tensioactif de formule (I) dans lequel G comprend un groupe G' représentant un groupe de formule (XXIV) dans laquelle A₁₀₁ représente respectivement -O-(CO)- ou -NH-(CO) étant alors formé,
- G₁ représente un bon groupe partant LG et G₂ représente un hydroxyle, un tensioactif de formule (I) dans lequel G comprend un groupe G' représentant un groupe de formule (XXV) étant alors formé,
- G₁ représente un bon groupe partant LG et G₂ représente une amine -NH₂, un tensioactif de formule (I) dans lequel G comprend un groupe G' représentant un groupe de formule (XXVI) étant alors formé,
- G₁ représente une oxyamine -O-NH₂ et G₂ représente un aldéhyde, un tensioactif de formule (I) dans lequel G comprend un groupe G' représentant un groupe de formule (XXVII) étant alors formé.

Lorsque le groupe G comprend plusieurs groupes G', le choix des groupes réagissant ensemble : G'₁ et G₁ d'une part et G'₂ et G₂ d'autre part, peut être effectué de la même façon, en remplaçant les groupes G₁ ou G₂ dans les exemples mentionnés ci-dessus par G'₁ ou G'₂.

### • Emulsions 1 et/ou 2 comprenant un tensioactif de formule (LI) ou (LII) où L₁-X₁-H₁- et/ou L₂-X₂-H₂- a(ont) la formule (CII) susmentionnée

La demande décrit un tensioactif de formule (L) suivante : 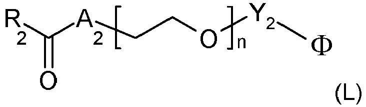 dans laquelle :
- R₂ représente une chaîne hydrocarbonée linéaire comprenant de 11 à 23 atomes de carbone, de préférence 17,
- A₂ représente O ou NH, de préférence NH,
- n représente un nombre entier de 3 à 500, de préférence 20 à 200,
- Y₂ représente un groupe de liaison (de préférence l'un des groupes Y₂ mentionnés ci-dessus), et
- Φ représente un groupe fonctionnel susceptible de se lier à un agent d'intérêt.

La demande décrit également une émulsion (dite émulsion 3, qui ne fait pas partie de l'invention) comprenant une phase aqueuse continue et une phase dispersée sous forme de gouttelettes comprenant un lipide amphiphile, un tensioactif de formule (L), éventuellement un lipide solubilisant et éventuellement un co-tensioactif (notamment les lipide amphiphile/ lipide solubilisant/ co-tensioactif définis ci-dessus).

Les gouttelettes de ces émulsions 3 présentent avantageusement des groupes Φ fonctionnalisables en surface. Il est donc possible de greffer des agents d'intérêt (agent biologique ou thérapeutique, chromophore ou fluorophore par exemple) en surface de ces gouttelettes.

Dans un mode de réalisation, le groupe Φ correspond à un groupe G₁ ou G₂ tel que défini ci-dessus et l'émulsion 3 correspond à l'émulsion 1 ou 2 définies ci-dessus. Lorsque le matériau selon l'invention est formé à partir d'une émulsion 1 et d'une émulsion 2 comprenant des tensioactifs de formules (LI) et (LII) répondant à la formule (L), les radicaux L₁-X₁-H₁- et L₂-X₂-H₂- du tensioactif de formule (I) du matériau répondent à la formule (CII) définie ci-dessus.

Les tensioactifs de formule (L) sont avantageusement faciles à préparer (notamment par formation d'un ester ou d'une amide entre un acide gras et un dérivé de poly(éthylène glycol).)

De plus, une émulsion peut généralement être préparée avec une quantité plus importante de tensioactif de formule (L) que de tensioactif de type ester ou amide d'acides gras comprenant de 12 à 24 atomes de carbone et de phosphatidyléthanolamine (par exemple du DSPE-PEG). Par conséquent, l'émulsion comprenant un tensioactif de formule (L) comprend avantageusement en surface plus de groupes Φ fonctionnalisables, et il est donc possible de greffer plus d'agents d'intérêts en surface des gouttelettes.

Par ailleurs, on a mis en évidence que le tensioactif de formule (L) utilisé dans une émulsion fuit moins hors des gouttelettes qu'un tensioactif de type ester ou amide d'acides gras comprenant de 12 à 24 atomes de carbone et de phosphatidyléthanolamine. Cet effet anti-fuite est plus important pour les tensioactifs de formule (L) dans lesquels A₂ représente NH par rapport à ceux dans lesquels A₂ représente O. De plus, les émulsions comprenant des tensioactifs dans lesquels A₂ représente NH sont généralement plus stables que celles comprenant des tensioactifs dans lesquels A₂ représente O.

### • Formation des émulsions 1 et 2

Les émulsions 1 et 2 telles que décrites peuvent être préparées aisément par dispersion de quantités appropriées de phase huileuse et de phase aqueuse sous l'effet d'un cisaillement, typiquement par un procédé comportant les étapes consistant à :
(i) préparer la phase huileuse comprenant un lipide amphiphile;
(ii) préparer une phase aqueuse comprenant un tensioactif de formule (LI) ou (LII);
(iii) disperser la phase huileuse dans la phase aqueuse sous l'action d'un cisaillement suffisant pour former une émulsion; et
(iv) récupérer l'émulsion ainsi formée.

Dans ce procédé, on mélange d'abord les différents constituants huileux pour préparer un pré-mélange huileux pour la phase dispersée de l'émulsion. Le mélange des différents constituants huileux peut éventuellement être facilité par mise en solution d'un des constituants ou du mélange complet dans un solvant organique approprié et évaporation subséquente du solvant, pour obtenir un pré-mélange huileux homogène pour la phase dispersée. Le choix du solvant organique dépend de la solubilité des constituants. Les solvants employés peuvent être par exemple le méthanol, l'éthanol, le chloroforme, le dichlorométhane, l'hexane, le cyclohexane, le DMSO, le DMF ou encore le toluène. Lorsqu'il s'agit d'un matériau pour l'administration d'agents thérapeutiques, il s'agit de préférence de solvants organiques volatils et/ou non toxiques pour l'homme. Par ailleurs, il est préféré de réaliser le pré-mélange à une température à laquelle l'ensemble des ingrédients est liquide.

Avantageusement, la phase huileuse est dispersée dans la phase aqueuse à l'état liquide. Si l'une des phases se solidifie à température ambiante, il est préférable de réaliser le mélange avec l'une ou de préférence les deux phases chauffées à une température supérieure ou égale à la température de fusion, les deux phases étant chauffées à une température de préférence inférieure à 80°C, et encore préférentiellement inférieure à 70°C, et encore préférentiellement inférieure à 60°C.

L'émulsification sous l'effet de cisaillement est de préférence réalisée à l'aide d'un sonificateur ou d'un microfluidiseur. De préférence, la phase aqueuse puis la phase huileuse sont introduites dans les proportions souhaitées dans un récipient cylindrique approprié puis le sonificateur est plongé dans le milieu et mis en marche pendant une durée suffisante pour obtenir une émulsion, le plus souvent quelques minutes.

Les émulsions 1 et 2 sont généralement des nanoémulsions. Par le procédé susmentionné, on obtient une nanoémulsion homogène dans laquelle le diamètre moyen des gouttelettes est supérieur à 20 nm et inférieur à 200 nm, notamment de 50 à 120 nm.

De préférence, le potentiel zêta de l'émulsion obtenue est inférieur à 25 mV en valeur absolue, c'est-à-dire compris entre -25mV et 25 mV.

Avant préparation du matériau selon l'invention, les émulsions 1 et/ou 2 peuvent être diluées et/ou stérilisées, par exemple par filtration ou dialyse. Cette étape permet d'éliminer les éventuels agrégats qui pourraient s'être formés au cours de la préparation des émulsions.

Les émulsions 1 et 2 ainsi obtenues sont prêtes à l'emploi, le cas échéant après dilution. Bien entendu, il est possible de préparer le matériau selon l'invention en mettant en oeuvre une émulsion 1 et 2 semblable ou identique.

Le matériau selon l'invention peut être préparé quel que soit la proportion en phase aqueuse continue des émulsions 1 et 2, ce qui est avantageux en termes de coût de préparation car il est possible de préparer le matériau à partir d'émulsions 1 et 2 ayant de faibles proportions de phase dispersées. Le ratio entre la phase dispersée et la phase aqueuse peut donc varier dans une large mesure dans les émulsions 1 et 2. La phase dispersée des émulsions 1 et 2 (huile/ lipide solubilisant/lipide amphiphile/ co-tensioactif/éventuel agent d'intérêt lipophile/tensioactif de formule (LI) ou (LII)) représente généralement entre 0,1 et 90% en poids par rapport au poids total de l'émulsion, c'est-à-dire par rapport au poids des phases aqueuse continue et huileuse dispersée.

Selon les réactions mises en jeu dans la formation du tensioactif de formule (I), la formation du matériau selon l'invention peut se produire en présence d'un cross-linker, par changement de pH, l'action de la lumière ou autre.

En pratique, au-delà d'une certaine proportion totale en phase aqueuse lors de la mise en contact des émulsions 1 et 2 (qui varie d'un matériau à l'autre, notamment selon la nature et les proportions des divers composants du matériau), on observe d'une part la formation du matériau comprenant une phase dispersée sous forme de gouttelettes dans une phase aqueuse continue, et d'autre part une phase aqueuse essentiellement, généralement totalement, exempte de gouttelettes.

### [Utilisations]

Le matériau selon l'invention a des applications très variées.

Le matériau tel que défini ci-dessus peut être utilisé comme membrane ou comme revêtement.

Avantageusement, le matériau selon l'invention peut être préparé à partir de constituants biocompatibles et donc être biocompatible. Ainsi, il peut par exemple être utilisé comme revêtement d'un objet ou matériau non biocompatible destiné à être implanté dans le corps humain ou animal, tel qu'une prothèse, un implant ou une électrode implantable.

### • Utilisation du matériau comprenant un tensioactif de formule (I) dont le groupe G comprend une fonction clivable

Le matériau comprenant un tensioactif de formule (I) dont le groupe G comprend une fonction clivable tel que défini ci-dessus peut être utilisé comme vanne.

Ainsi, lorsqu'un matériau tel que défini ci-dessus est introduit dans un milieu fermé (canalisation, tube, capillaire), il peut arrêter ou modifier (généralement ralentir) le débit d'un fluide, de préférence d'un liquide.

Si le matériau est mis dans des conditions permettant le clivage de la fonction clivable du groupe G du tensioactif de formule (I) (par exemple par mise en contact avec un composé chimique ou une enzyme capable de cliver la fonction du groupe G, ou dans des conditions électrochimiques, de pH, de lumière ou de température permettant ce clivage, comme explicité ci-dessus), les liaisons covalentes entre les gouttelettes du matériau selon l'invention sont clivées. Ainsi, les gouttelettes ne sont plus liées les une aux autres et le matériau se désagrège, ce qui a pour conséquence d'augmenter l'écoulement du fluide. De préférence, le fluide dont le débit est modifié ou arrêté par le matériau selon l'invention utilisé comme vanne comprend ou est constitué d'une phase aqueuse.

Lorsque la réaction de clivage de la fonction clivable du groupe G du tensioactif de formule (I) est réversible, après avoir clivé ladite fonction, en mettant le matériau dans des conditions permettant la reformation de la fonction clivable du groupe G du tensioactif de formule (I) (et donc la reformation des liaisons covalentes entre les gouttelettes), il est possible de refermer la vanne. Il est alors possible d'ouvrir et de refermer la vanne.

Le matériau comprenant un tensioactif de formule (I) dont le groupe G comprend une fonction clivable tel que défini ci-dessus peut être utilisé comme masque pour préparer des biopuces. En effet, le matériau selon l'invention est alors un revêtement destructible (par clivage de ladite fonction) et éventuellement reformable (par reformation de ladite fonction) adapté pour une telle utilisation. On peut par exemple utiliser les méthodes décrites dans Nie, Z H, Kumacheva, E, Patterning surfaces with functional polymers, Nature Materials 2008, 7(4), 277-290 ou Hoffmann, J et al, Photopatterning of thermally sensitive hydrogels useful for microactuators. Sensors and actuators A: Physical, 1999, 77(2), 139-144 en utilisant le matériau selon l'invention à la place des matériaux décrits.

### • Utilisation du matériau comprenant un agent d'intérêt

Le matériau comprenant un agent d'intérêt tel que défini ci-dessus peut être utilisé comme détecteur chimique. En effet, lorsque l'agent d'intérêt est un agent détecteur chimique, le matériau selon l'invention peut être utilisé pour détecter et/ou quantifier la présence d'un composé chimique (analyte, polluant, métal...).

Le matériau comprenant un agent d'intérêt tel que défini ci-dessus peut être utilisé comme capteur chimique. Lorsque l'agent d'intérêt est un agent capteur chimique, le matériau selon l'invention peut être utilisé comme capteur chimique, notamment pour purifier un milieu ou comme scavenger (l'agent capteur chimique contenu dans le matériau réagissant avec le composé chimique que l'on souhaite éliminer (par exemple un polluant, ou, lors d'une réaction chimique, un produit de réaction secondaire ou un réactif en excès)).

Le matériau comprenant un agent d'intérêt tel que défini ci-dessus peut être utilisé pour la délivrance d'agent d'intérêt, notamment la délivrance ex *vivo, in vivo* ou *in vitro* d'agents d'intérêts. On peut notamment mentionner la délivrance d'un agent optique, d'un agent phytosanitaire, d'un agent de masquage de goût/odeur ou d'un agent cosmétique. Le cas de la délivrance d'un agent thérapeutique est décrit ci-après.

Lorsque le matériau selon l'invention comprend plusieurs agents d'intérêts, il peut être utilisé pour la délivrance combinée, simultanée ou séparée dans le temps de ces agents d'intérêts.

Dans le mode de réalisation dans lequel le matériau comprend un agent d'intérêt hydrophile et un agent d'intérêt lipophile, il peut être utilisé pour la délivrance combinée, simultanée ou séparée dans le temps de l'agent d'intérêt hydrophile et de l'agent d'intérêt lipophile.

Le matériau comprenant un agent d'intérêt de type catalyseur tel que défini ci-dessus peut être utilisé comme catalyseur.

### • Utilisation du matériau comprenant un agent thérapeutique

Le matériau comprenant un agent thérapeutique peut être utilisé pour traiter ou prévenir une maladie. En effet, le matériau peut être utilisé pour l'administration d'au moins un agent thérapeutique à l'homme ou à l'animal. Le matériau comprenant un agent thérapeutique peut être utilisé comme médicament, ou dans une méthode de traitement thérapeutique comprenant l'administration à un homme ou à un animal qui en a besoin d'une quantité efficace du matériau comprenant un agent thérapeutique pour traiter ou prévenir une maladie.

Comme le matériau selon l'invention peut être préparé exclusivement à partir de constituants approuvés pour l'homme ou l'animal, il est particulièrement intéressant pour une administration par voie parentérale. Cependant, il est également possible d'envisager une administration par d'autres voies, notamment par voie orale ou par voie topique.

Les techniques décrites dans la publication Hoffman, The Origins and évolution of « controlled » drug delivery systems, Journal Controlled release 132 (2008) 153-163 peuvent notamment être utilisées, en remplaçant les matériaux qui y sont décrits par le matériau selon l'invention.

Par exemple, le matériau peut être administré par l'intermédiaire d'une seringue ou d'un timbre transdermique (« patch » en anglais), cette formulation étant particulièrement adaptée car le matériau présente un caractère collant.

### • Utilisation du matériau comprenant un agent thérapeutique hydrophile et d'un agent thérapeutique lipophile

Le matériau comprenant au moins un agent thérapeutique hydrophile et au moins un agent thérapeutique lipophile peut être utilisé pour l'administration d'au moins un agent thérapeutique hydrophile et d'au moins un agent thérapeutique lipophile à l'homme ou à l'animal pour traiter ou prévenir une maladie.

En effet, des gouttelettes, et en particulier des nanogouttelettes, chargées d'agents thérapeutiques constituent une solution idéale pour surmonter la faible sélectivité des médicaments, notamment des médicaments anticancéreux, en permettant grâce à un ciblage passif et/ou actif le ciblage des tissus cancéreux, et ainsi de réduire les effets secondaires sévères.

Certains traitements nécessitent l'administration de plusieurs agents thérapeutiques, parfois de solubilités différentes, ce qui implique alors plusieurs administrations, une gêne et une perte de temps accrue pour les patients. En outre, il est souvent préférable que les différents agents thérapeutiques ne soient pas tous libérés au même moment, voire pas tous au même endroit.

Le développement de formulations permettant la délivrance de plusieurs agents thérapeutiques est donc souhaitable.

Le matériau selon l'invention permet de fournir en une seule administration/application deux agents thérapeutiques ou plus, à des temps de libération différents. Au moins un agent thérapeutique hydrophile est libéré à un temps t_{hydrophile} et au moins un agent thérapeutique lipophile est libéré à un temps tₗᵢₚₒₚₕᵢₗₑ, identique ou différent de t_{hydrophile}.

En effet, l'agent thérapeutique hydrophile est essentiellement situé dans la phase aqueuse continue du matériau. Il est piégé entre les gouttelettes de la phase dispersée. Lorsque le matériau est administré, il entre en contact avec des fluides physiologiques (sang, plasma...) qui s'échangent avec la phase aqueuse continue du matériau, libérant ainsi l'agent thérapeutique hydrophile. Le temps de libération de l'agent thérapeutique hydrophile t_{hydrophile} est lié au temps d'échange entre la phase aqueuse continue du matériau et les fluides physiologiques, au temps de diffusion de l'agent thérapeutique hydrophile à travers le matériau et parfois au temps de libération des gouttelettes t_{gouttelette} lorsque le tensioactif de formule (I) comprend des fonctions susceptibles d'être clivées dans le milieu physiologique (le clivage de ces fonctions conduisant alors au clivage des liens covalents entre les gouttelettes et à la désagrégation du réseau tridimensionnel du matériau).

Par ailleurs, l'agent thérapeutique lipophile est essentiellement situé dans la phase dispersée du matériau, soit à l'intérieur des gouttelettes, soit en surface des gouttelettes. Le temps de libération de l'agent thérapeutique lipophile tₗᵢₚₒₚₕᵢₗₑ est lié au temps de diffusion de l'agent thérapeutique lipophile vers l'extérieur de la gouttelette, au temps de dégradation des gouttelettes et parfois au temps de libération des gouttelettes t_{gouttelette}.

Les localisations de libération des agents thérapeutiques hydrophiles L_{hydrophile} et lipophiles Lₗᵢₚₒₚₕᵢₗₑ peuvent également être différentes. En particulier, si le tensioactif de formule (I) comprend des fonctions susceptibles d'être clivées dans le milieu physiologique lorsque le matériau se désagrège à l'endroit où il a été administré, l'agent thérapeutique hydrophile étant alors libéré à la localisation d'administration et les gouttelettes libérées du matériau sont emportées par le fluide physiologique (sang, plasma), vers un autre endroit du sujet, où sera libéré l'agent thérapeutique lipophile.

Ainsi, en adaptant la composition du matériau selon l'invention (nature des constituants, fraction massique des constituants, taille des gouttelettes...) en fonction des propriétés physicochimiques des agents, comme explicité ci-après, il est avantageusement possible de modifier ces temps de libération t_{hydrophile} et tₗᵢₚₒₚₕᵢₗₑ et localisations L_{hydrophile} et Lₗᵢₚₒₚₕᵢₗₑ.

Bien sûr, si le matériau comporte plus d'un agent thérapeutique hydrophile et/ou plus d'un agent thérapeutique lipophile, il est possible d'adapter la composition du matériau pour ajuster les temps de libération de chaque agent, et que ceux-ci diffèrent les uns des autres. On pourra notamment agir sur les paramètres de la composition du matériau influençant la libération des agents d'intérêt hydrophiles ou des agents d'intérêt lipophiles pour que t_{hydrophile 1} diffère de t_{hydrophile 2} et/ou que t_{lipophile 1} diffère de t_{lipophile 2}, comme explicité ci-dessous. Les différentes localisations des libérations des agents peuvent également être influencées et différer les unes des autres.

Le temps de libération de l'agent thérapeutique hydrophile t_{hydrophile} dépend de la composition du matériau, en particulier:
- de la fraction massique de la phase dispersée par rapport au poids total du matériau,
- du nombre d'unités alcoxylées du co-tensioactif alcoxylé (et donc de la longueur de la chaîne alcoxylée du co-tensioactif alcoxylé),
- du diamètre des gouttelettes,
- de la nature du tensioactif de formule (I).

Le temps de libération de l'agent thérapeutique lipophile tₗᵢₚₒₚₕᵢₗₑ est lié au temps de diffusion de l'agent thérapeutique lipophile vers l'extérieur de la gouttelette et au temps de libération des gouttelettes t_{gouttelette}. Le temps de libération de l'agent thérapeutique lipophile tₗᵢₚₒₚₕᵢₗₑ dépend :
- du diamètre moyen des gouttelettes, comme décrit notamment dans Williams, Y. et al. Small (2009); 5(22):2581-8, Choi, H. S. et al. Nanoletters (2009) 9(6):2354-9 et Massignani, M. et al. Small. (2009) 5(21):2424-32. Les gouttelettes du matériau selon l'invention sont avantageusement monodisperses pour permettre une libération homogène dans le temps de l'agent thérapeutique lipophile.
- de la nature des composants de la phase huileuse, notamment du lipide solubilisant,
- des caractéristiques physicochimiques de l'agent thérapeutique lipophile (Nel, A. E. et al. Nature Materials 8 (2009) pp543-557), notamment de son log P, qui influe sur la localisation de l'agent thérapeutique lipophile à l'intérieur ou en surface de la gouttelette.

Un agent thérapeutique très lipophile reste dans la gouttelette et n'est libéré que lorsque celle-ci est dégradée par dégradation chimique (par hydrolyse des composants des gouttelettes suite à une augmentation ou diminution importante du milieu, par exemple si les gouttelettes sont internalisées à l'intérieur des cellules en passant par les lysosomes) ou par dégradation enzymatique par des lipases (Olbrich, C. et al. International Journal of Pharmaceutics 237 (2002) pp 119-128 et Olbrich, C. International Journal of Pharmaceutics 180 (1999) pp31-39).

Généralement, le temps de l'agent thérapeutique hydrophile t_{hydrophile} est inférieur au temps de libération de l'agent thérapeutique lipophile tₗᵢₚₒₚₕᵢₗₑ.

La localisation de la libération de l'agent thérapeutique hydrophile L_{hydrophile} est généralement la localisation d'administration du matériau.

La localisation de la libération de l'agent thérapeutique lipophile Lₗᵢₚₒₚₕᵢₗₑ est soit la localisation d'administration (dans ce cas, L_{hydrophile} et Lₗᵢₚₒₚₕᵢₗₑ sont généralement identiques), soit un autre endroit du corps de l'homme / de l'animal, notamment lorsque les gouttelettes libérées du matériau sont emportées par le fluide physiologique (liquide interstitiel, liquide lymphatique, sang) vers un autre endroit. Bien sûr, la localisation de la libération de l'agent thérapeutique lipophile dépend également des propriétés physicochimiques :
- de la zone d'administration du matériau, notamment de la densité des tissus et de la présence ou non de barrières physiologiques, et
- de la nature et des propriétés physicochimiques de l'agent thérapeutique lipophile lui même. Ainsi, lorsque plus d'un agent thérapeutique lipophile est utilisé dans le matériau, chaque agent thérapeutique lipophile a une localisation de la libération qui lui est propre.

Il est notamment possible de moduler Lₗᵢₚₒₚₕᵢₗₑ en utilisant dans le matériau un co-tensioactif polyalcoxylé comportant un ligand biologique de ciblage greffé, qui va permettre que les gouttelettes, et donc l'agent thérapeutique lipophile, soient dirigés vers la cible désirée.

De nombreuses utilisations comme médicament du matériau selon l'invention comprenant un agent thérapeutique hydrophile et un agent thérapeutique lipophile sont envisageables.

Par exemple, un des agents thérapeutique peut être un principe actif pharmaceutique pour le traitement de la maladie visée, et l'autre peut être un agent thérapeutique permettant de diminuer les effets secondaires, notamment ceux associés audit principe actif pharmaceutique.

Un matériau selon l'invention dans laquelle l'agent thérapeutique hydrophile est un agent cicatrisant, antibactérien ou anti-inflammatoire et l'agent thérapeutique lipophile est un anticancéreux peut notamment être utilisé pour le traitement post-exérèse d'une tumeur. Ce matériau est appliqué suite à une opération d'exérèse de tumeur sur le site d'excision de la tumeur.

L'agent thérapeutique cicatrisant, antibactérien ou anti-inflammatoire hydrophile est libéré rapidement pour diminuer les effets secondaires de l'exérèse et favoriser la cicatrisation.

L'agent thérapeutique anticancéreux lipophile est libéré plus tardivement, généralement durant les premières heures suivant l'application du matériau, et traite les amas de cellules tumorales restants n'ayant pas été excisés. Il est en effet souvent difficile de curer complètement l'ensemble de la tumeur lors de l'exérèse. Le matériau permet ainsi un traitement complet de la zone tumorale.

Les gouttelettes comprenant l'agent anticancéreux lipophile de la phase dispersée peuvent également rejoindre la circulation lymphatique et sanguine et traiter les éventuelles cellules cancéreuses circulant dans le système circulatoire et étant à l'origine de métastases.

En particulier, le co-tensioactif du matériau peut comporter un ligand biologique de ciblage des cellules cancéreuses pour pouvoir cibler plus efficacement les cellules cancéreuses.

De plus, un matériau selon l'invention dans laquelle l'agent thérapeutique hydrophile est un agent stimulant le système immunitaire et l'agent thérapeutique lipophile est un anticancéreux peut notamment être utilisé pour le traitement post-cryogénie d'une tumeur.

La cryogénie de tumeur consiste en l'injection d'un liquide cryogénique dans un tumeur à l'aide d'une seringue. Les cellules tumorales sont tuées par ce traitement, et restent à l'intérieur du corps du sujet traité.

Le matériau précité peut augmenter l'efficacité du traitement. L'agent hydrophile stimulant le système immunitaire est libéré rapidement pour activer le système immunitaire et l'agent anticancéreux lipophile est libéré plus tardivement, et permet d'éliminer les cellules tumorales encore vivantes. Là encore, les gouttelettes comprenant l'agent anticancéreux lipophile de la phase dispersée peuvent rejoindre la circulation lymphatique et sanguine et traiter les éventuelles cellules cancéreuses circulant dans le système circulatoire et étant à l'origine de métastases. De plus, le co-tensioactif du matériau peut comporter un ligand biologique de ciblage des cellules cancéreuses pour pouvoir cibler plus efficacement les cellules cancéreuses.

L'invention sera décrite plus en détail au moyen des exemples et figures en annexe, lesquelles montrent :

### FIGURES

La figure 1 représente un schéma illustrant le positionnement du tensioactif de formule (I) dans lequel v et w représentent 1 et des gouttelettes du matériau selon l'invention.
La figure 2 représente un schéma illustrant le positionnement du tensioactif de formule (I) dans lequel v et w représentent 2 et des gouttelettes du matériau ( mode de réalisation qui ne relève pas de l'invention).
La figure 3 représente des photographies de matériaux de l'exemple 1 obtenus avec des émulsions 1 et 2 et comprenant des phases huileuses dispersées de 1, 5, 15 ou 23%.
La figure 4 représente des photographies d'un mélange (matériau de l'exemple 1 et solution aqueuse de PBS) juste après l'ajout de PBS, et 5 mois après stockage.
La figure 5 représente les résultats d'analyse en DLS, à savoir l'intensité normalisée en % en fonction de la taille en nm, de l'émulsion 1 (trait plein), du matériau selon l'invention (trait plein/pointillé discontinu) et de l'émulsion obtenue après clivage de la fonction disulfure du matériau (pointillé).
La figure 6 représente le pourcentage de perte de tensioactif C₁₇H₃₅-CO-NH-[(CH₂)₂-O]₁₀₀-(CH₂)₂-NH-FITC hors des gouttelettes de l'émulsion 3 A₂ = NH (losanges, trait plein) ou de tensioactif C₁₇H₃₅-CO-O-[(CH₂)₂-O]₁₀₀-(CH₂)₂-NH-FITC hors des gouttelettes de l'émulsion 3 A₂ = O (carrés, trait en pointillé) en fonction du temps en heures (mode de réalisation qui ne relève pas de l'invention).
La figure 7 représente des photographies de matériaux de l'exemple 7 : A) l'émulsion de gouttelettes LNP-SPDP; B) le gel formé après ajout de PEG dithiol ; C) la suspension de gouttelettes LNP-SH obtenue après ajout de DTT.
La figure 8 représente des photographies de matériaux de l'exemple 9 avant et après irradiation.

### EXEMPLES

### Exemple 1 : Préparation d'un matériau comprenant un tensioactif de formule (I) comprenant un groupe G comprenant une fonction clivable réversible

### ◆ Préparation d'un tensioactif de formule (LII)

Un tensioactif de formule (LII) dans lequel :
- L₂ est acide gras comprenant de 18 atomes de carbone (acide stéarique),
- H₂ est un poly(oxyde d'éthylène) comprenant 100 unités oxyde d'éthylène,
- G₂ représente un groupe -S-S-pyridinyle,
- X₂ représente -NH-,
- Y₂ représente -CH₂-CH₂-NH-CO-CH₂-CH₂-
a été prépare en suivant le schéma réactionnel suivant :

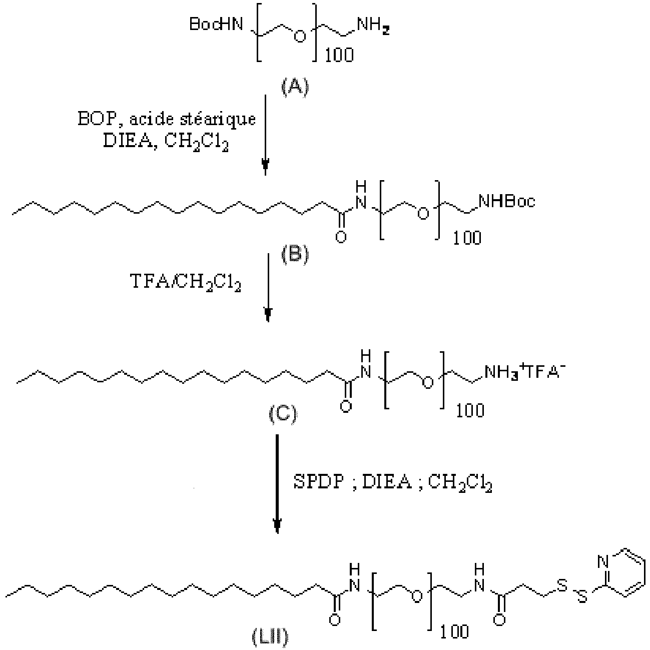

### Synthèse du composé (B)

De l'acide stéarique (2 g ; 0,6 mmol) et du Benzotriazole-1-yl-oxy-tris-(dimethylamino)-phosphonium hexafluorophosphate (BOP) (265,2 mg ; 0,6 mmol) ont été dissous dans du CH₂Cl₂ (15 mL). Après 10 minutes d'agitation, du BocNH-PEG100-NH₂ (PM : 4928 ; 2 g ; 0,4 mmol) (composé (A)) et de la diisopropyléthylamine (DIEA) (104,5 mL ; 0,6 mmol) ont été ajoutés au milieu réactionnel. La disparition de l'amine de départ a été vérifiée par chromatographie sur couche mince (CCM) (CH₂Cl₂/MeOH). Après 2 heures sous agitation, le produit a été précipité dans l'éther à froid, dissous dans un minimum d'eau puis dialysé contre de l'eau milli Q (cut off 1000). La solution a ensuite été récupérée et l'eau a été enlevée soit par évaporation (éthanol comme azéotrope) soit par lyophilisation, pour donner 1,5 g de composé (B) (poudre blanche), soit un rendement de 70 %.

CCM (CH₂Cl₂/ MeOH 9/1): Rf=0,5

RMN ¹H (300 MHz ; CDCl3) : d : 0,87 (t ; *J*=6,5 Hz ; 3H ; C**H₃**-CH₂) ; 1,24 (m ; 28H ; 14C**H₂**) ; 1,44 (s ; 9H ; C(C**H₃**)₃) ; 1,67 (quin ; 2H ; C**H₂**-CH₂-CONH) ; 2,42 (t ; *J*=7,5 Hz ; 2H ; C**H₂**-CONH) ; 3,3 (t ; *J*=5,0 Hz ; 2H ; BocNH-C**H₂**) ; 3,45-3,8 (m ; 362H ; xC**H₂**(PEG), CH₂CONH-C**H₂**)

### Synthèse du composé (C)

Le composé (B) (1,5 g ; 0,29 mmol) a été dissous dans 10 mL de dichlorométhane et 4 mL d'acide trifluoroacétique (TFA). La conversion en composé (C) a été suivie par CCM (révélateur ninhydrine). Après 1 heure sous agitation, le solvant a été évaporé par coévaporation avec du toluène (qui élimine le TFA). Le produit a été séché sous vide pour donner 1,3 g de composé (C) (poudre blanche), soit un rendement de 86,7 %

CCM (CH₂Cl₂/ MeOH 9/1): Rf=0,27

RMN ¹H (300 MHz ; CDCl3) : d : 0,87 (t ; *J*=6,5 Hz ; 3H ; C**H₃**-CH₂) ; 1,24 (m ; 28H ; 14C**H₂**) ; 1,60 (quin ; 2H ; C**H₂**-CH₂-CONH) ; 2,15 (t ; *J*=7,5 Hz ; 2H ; C**H₂**-CONH) ; 3,17 (bt ; 2H ; C**H₂**-NH₃⁺ ) ; 3,4 (m ; 2H ; CH₂CONH-C**H₂**) ; 3,5-3,8 (m ; 360H ; xC**H₂**(PEG)) ; 6,14 (bs ; 1H ; N**H**CO) ; 7,9 (bs ; 2H ; N**H₂**/N**H₃+**)

### Synthèse du tensioactif (LII)

Sous argon, du composé (C) (0,5 g ; 0,1 mmol) et de la DIEA (52 mL ; 0,3 mmol) ont été dissous dans du dichlorométhane (10 mL). Après 5 minutes sous agitation du Succinimidyl 3-(2-pyridyldithio)propionate (SPDP) (93 mg ; 0,3 mmol) ont été ajoutés dans le milieu réactionnel. La disparition de l'amine a été suivie par CCM (CH₂Cl₂/MeOH 9/1). Après 1 heure de réaction, le produit a été précipité à deux reprises dans l'éther pour donner après filtration 400 mg de tensioactif (LII) (poudre jaunâtre) soit un rendement de 76 %

CCM (CH₂Cl₂/ MeOH 9/1): Rf=0,42

RMN 1H (300 MHz ; CDCl3) : d : 0,88 (t ; *J*=6,5 Hz ; 3H ; C**H₃**-CH₂) ; 1,25 (m ; 28H ; 14C**H₂**) ; 1,63 (quin ; 2H ; C**H₂**-CH₂-CONH) ; 2,17 (t ; *J*=7,5 Hz ; 2H ; C**H₂**-CONH) ; 2,62 (t ; *J*=7 Hz ; 2H ; C**H₂-**SS) ; 3,09 (t ; *J*=7 Hz ; 2H ; NHCO-C**H₂**-CH₂-SS) ; 3,42 (m ; 2H ; C**H₂**-NHCO) ; 3,48-3,8 (m ; 360H ; xC**H₂**(PEG) ; C**H₂**-NHCO) ; 6,11 (bt ; 1H ; N**H**) ; 6,79 (bt ; 1H ; N**H**) ; 7,11 (m ; 1H ; C**H**pyr) ; 7,67 (m ; 2H ; 2C**H**pyr) ; 8,49 (m ; 1H ; C**H**pyr)

### ◆ Préparation d'émulsions 2 comprenant un tensioactif de formule (LII)

Des émulsions 2 ont été préparées en suivant les procédures décrites dans WO 2010/018223 avec les compositions indiquées dans le tableau 1, la dissolution complète du Myrj S40 et du tensioactif de formule (LII) ayant nécessité de chauffer la solution à 55°C et en mélangeant puis émulsifiant les phases aqueuse et huileuse par sonication selon les paramètres décrits dans le tableau 2.

**Tableau 1 : compositions des émulsions 2**

| | Fournisseur | Emulsion | Emulsion | Emulsion | Emulsion |
|---|---|---|---|---|---|
| | | 2 (20%) | 2 (15%) | 2 (10%) | 2 (5%) |
| Huile de soja (mg) | CRODA | 68 | | | |
| Suppocire^{®} NB (mg) | Gattefossé | 272 | | | |
| Lécithine (mg) | Lipoïd | 65 | | | |
| Eau PBS 1X (mL) | - | 2,5 | | | |
| Myrj S40 (mg) | CRODA | 276 | 293 | 310 | 328 |
| tensioactif (LII) (mg) | - | 69 | 52 | 35 | 17 |
| m (tensioactif (LII)) / [m (tensioactif (LII) + m (Myrj S40)) * (%) | - | 20 | 15 | 10 | 5 |
| % phase dispersée | | 23 | | | |
| Dil (mg) | | 4 | | | |

| | | | | | |
|---|---|---|---|---|---|
| * Rapport de la masse de tensioactif de formule (LII) sur la masse de l'ensemble (tensioactif de formule (LII) / Myrj S40) (en %). La masse de l'ensemble (tensioactif de formule (LII) / Myrj S40) est de 345 mg pour toutes les émulsions. | | | | | |

**Tableau 2 : Paramètres de sonication utilisés**

| Volume du lot | Sonde (φ) | Puissance Pmax | Temps de sonication | Pulse on/off |
|---|---|---|---|---|
| 3.25 mL | 3 mm | 28% | 5 min | 10s / 30s |

Pour mieux visualiser la stabilité et la formation du matériau formé ultérieurement, les gouttelettes ont été colorées par incorporation d'un fluorophore, le 3,3'-dioctadecylindocarbocyanine (Dil) (4 mg / émulsion)).

### ◆ Préparation d'émulsions 1 comprenant un tensioactif de formule (LI)

Les émulsions 1 comprenant un tensioactif de formule (LI) de formule suivante : 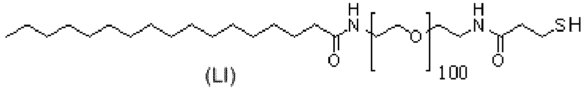 ont été obtenues à partir de l'émulsion 2 préparée ci-dessus.

Le groupement thiol du tensioactif (LI) a été obtenu par déprotection des fonctions -S-S-pyridinyle par un agent réducteur dithiothréitol (DTT).

Par exemple, pour l'émulsion 1(20%) obtenue à partir de l'émulsion 2(20%) susmentionnée, on a prélevé 1,5 mL de l'émulsion 2(20%) à laquelle on a ajouté 23 mg de DTT (soit 20eq molaire / SSpyr). La réaction a été laissée sous agitation (plateau mobile) pendant 2 heures puis

### ◆ Préparation du matériau selon l'invention à partir des émulsions 1 et 2

Les émulsions 1 et 2 préparées ci-dessus ont été purifiées par dialyse contre du PBS 1X (MW Cut off 12000-14000 Da ; 500 mL ; 24 heures).

La taille des gouttelettes des émulsions a été est déterminée par mesure en DLS (Zêta Sizer Nano ZS, Malvern). Les émulsions présentent une distribution en taille similaire avec des tailles de gouttelettes de 65 nm avec un indice de polydispersité de 0,112.

Les émulsions ont éventuellement été diluées par ajout de phase aqueuse, pour obtenir des pourcentages en phases huileuses dispersées de 1, 5, 15 ou 23%.

Pour former le matériau selon l'invention, des émulsions 1 et 2 ayant :
- la même proportion en phase dispersée, et
- des rapports de la masse de tensioactif de formule (LII) sur la masse de l'ensemble (tensioactif de formule (LII) / Myrj S40), et de la masse de tensioactif de formule (LI) sur la masse de l'ensemble (tensioactif de formule (LI) / Myrj S40), identiques,
ont été utilisées.

On a mélangé un volume équivalent d'émulsions 1 et 2, la solution a ensuite été agitée et un matériau homogène s'est formé rapidement après 2 à 4 minutes d'agitation. Une liaison disulfure a été formée entre les tensioactifs de formules (LI) de l'émulsion 1 et (LII) de l'émulsion 2 pour former le tensioactif de formule (I) suivante :

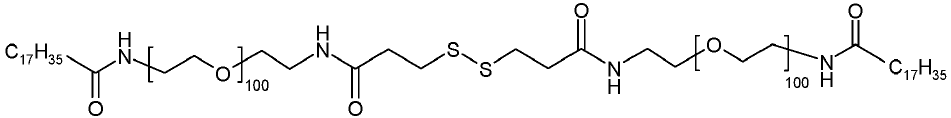

Ce tensioactif de formule (I) (dans lequel G représente -S-S-) assure la liaison covalente entre les gouttelettes du matériau formé.

Plusieurs essais ont été réalisés avec des pourcentages en phases huileuses dispersées différents (1, 5, 15 ou 23%).

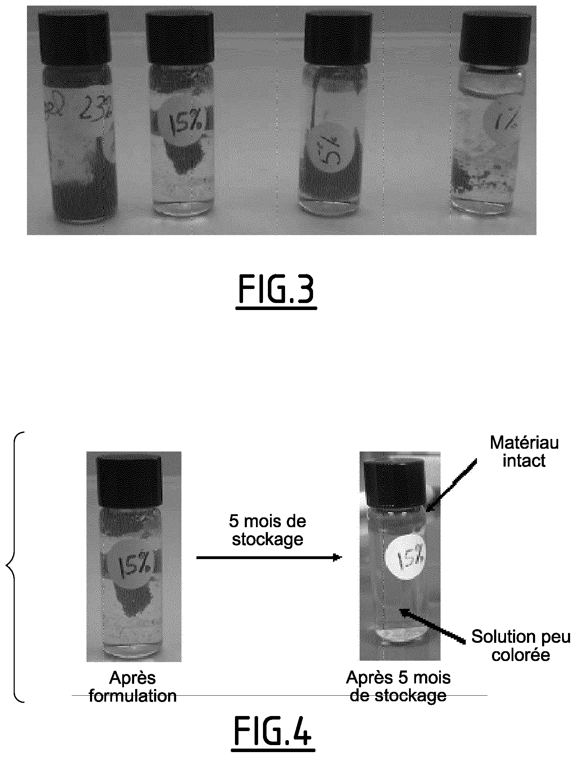

La Figure 3 annexée représente quatre photos de matériaux obtenus avec des émulsions 1 et 2 et comprenant des phases huileuses dispersées de 1, 5, 15 ou 23%. Quelque soit les proportions de phases dispersées des émulsions 1 et 2, le matériau se forme rapidement.

Lorsque la phase dispersée est supérieure à 15%, lors de la mise en contact des 2 émulsions, on a procédé comme suit afin d'obtenir un matériau homogène
- Vortexer
- Chauffer pour refluidifier
- vortexer
- Chauffer pour refluidifier
- Vortexer
- Laisser reposer

### ◆ Influence du rapport m (tensioactif (LII)) / [m (tensioactif (LII) + m (Myrj S40))

Des essais de préparation de matériau selon l'invention ont été réalisés à partir d'émulsions ayant des rapports m (tensioactif (LII)) / [m (tensioactif (LII) + m (Myrj S40)) différents (les rapports de la masse de tensioactif de formule (LII) sur la masse de l'ensemble (tensioactif de formule (LII) / Myrj S40) de l'émulsion 2, et de la masse de tensioactif de formule (LI) sur la masse de l'ensemble (tensioactif de formule (LI) / Myrj S40) de l'émulsion 1 étant toujours identiques) pour obtenir des matériaux ayant un rapport m (tensioactif (I)) / [m (tensioactif (I) + m (Myrj S40)) différents, à savoir 5, 10, 15 et 20% comme mentionné dans le tableau 1. Le matériau se forme plus facilement lorsque ledit rapport est supérieur ou égal à 15%.

### ◆Préparation du matériau selon l'invention à partir d'émulsions 1 et 2 comprenant des gouttelettes de plus grande taille (125 nm)

La préparation d'une émulsion 2 décrite ci-dessus ont été reproduites avec les mêmes composants, mais en faisant varier leurs proportions comme indiqué dans le tableau 3 suivant et avec des paramètres de sonication identiques.

**Tableau 3 : compositions de l'émulsion 2 (20%)bis**

| | Emulsion 2 (20%)bis |
|---|---|
| Huile de soja (mg) | 150 |
| Suppocire^{®} NB (mg) | 450 |
| Lécithine (mg) | 45 |
| Eau PBS 1X (mL) | 2.39 |
| Myrj S40 (mg) | 172 |
| tensioactif (LII) (mg) | 43 |
| m (tensioactif (LII)) / [m (tensioactif (LII) + m (Myrj S40)) * (%) | 20 |
| Dil (mg) | 4 |

La taille des gouttelettes de l'émulsion 2 (20%)bis obtenue a été mesurée par DLS (Zêta Sizer Nano ZS, Malvern). Elles présentent une distribution en taille avec une moyenne de 125 nm et un indice de polydispersité de 0,13

Là encore, l'émulsion 1 (20%)bis comprenant un tensioactif de formule (LI) de formule suivante : 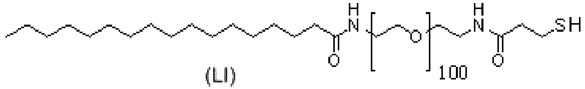 a été obtenue à partir de l'émulsion 2 (20%)bis préparée ci-dessus comme suit :
On a prélevé 1,625 mL d'émulsion 2 (20%)bis auxquels on a ajouté 16 mg de DTT (soit 22eq molaire / SSpyr) pour former l'émulsion 1 (20%)bis comprenant le tensioactif de formule (LI). La réaction a été laissée sous agitation (plateau mobile) pendant 15 heures.

Ensuite, les émulsion 1 (20%)bis et émulsion 2 (20%)bis ont été mises à dialyser contre du PBS 1X à 2 reprises (MW Cut off 12000-14000 Da ; 500 mL ; 24 heures).

Le matériau a alors été préparé à partir de l'émulsion 1 (20%)bis et de l'émulsion 2 (20%)bis en suivant le même protocole que décrit ci-dessus.

Cette expérience montre qu'il est possible de préparer un matériau comprenant des gouttelettes de taille différente.

### Exemple 2 : Test de résistance du matériau selon l'exemple 1 à la dilution

Le matériau de l'exemple 1 préparé à partir d'émulsions 1 et 2 toutes les deux à 23 % de phase dispersée et dans lequel m (composé (I)) / [m (composé (I) + m (Myrj S40)) = 20% a été récupéré avec une spatule, transvasé dans un pilulier puis 1mL d'une solution aqueuse de PBS (1x) a été ajouté.

La résistance à la dilution du matériau est totale, le matériau conservant sa structure même après dilution. La densité du matériau est inférieure à celle de l'eau (le matériau flotte). Si une forte agitation (c'est-à-dire à 3000 tours/ minute sur un appareil à vortexer Vortex Top-Mix 3, Fischer Scientific) est appliquée, le matériau se fragmente en petits morceaux pour ensuite finir par se re-solidariser par processus de crémage au bout d'environ 6 heures.

### Exemple 3 : Tests de stabilité du matériau selon l'exemple 1

Le matériau de l'exemple 1 préparé à partir d'émulsions 1 et 2 toutes les deux à 23 % de phase dispersée et dans lequel m (composé (I)) / [m (composé (I) + m (Myrj S40)) = 20% a été récupéré avec une spatule, transvasé dans un pilulier puis 1mL d'une solution aqueuse de PBS (1x) a été ajouté. Ensuite, le mélange obtenu a été stocké 5 mois à température ambiante (25°C). Les photos de la figure 4 montrent que la coloration de la solution de PBS reste faible même après 5 mois de stockage, ce qui montre que les gouttelettes du matériau restent liées les unes aux autres et que le matériau selon l'invention est stable.

Le test a été reproduit en remplaçant la solution aqueuse de PBS par du sérum physiologique (sérum de veau foetal) (Sigma Aldrich). Le matériau reste intact dans ce milieu pendant 16 heures à 37°C ou 50°C, ce qui montre une stabilité dans des milieux physiologiques.

### Exemple 4 : Clivage du groupe G du tensioactif de formule (I) du matériau de l'exemple 1

Le matériau de l'exemple 1 préparé à partir d'émulsions 1 et 2 toutes les deux à 23 % de phase dispersée et dans lequel m (composé (I)) / [m (composé (I) + m (Myrj S40)) = 20% a été utilisé.

Le groupe disulfure du tensioactif de formule (I) du matériau a été clivé par un agent réducteur de pont disulfure, le dithiothréitol ou réactif de Cleland.

On a ajouté 10 mg de DTT (dithiothréitol ou réactif de Cleland) afin de réduire les ponts disulfures intra-particules sur la totalité du matériau. On a alors observé une dissolution immédiate du matériau, c'est-à-dire une désagrégation des gouttelettes qui se sont réparties dans l'ensemble de la phase aqueuse du milieu. La taille des gouttelettes présentes dans la solution une fois le clivage des ponts disulfures opéré, est identique à celle des gouttelettes des émulsions 1 et 2 (avant création des liaisons covalentes disulfure).

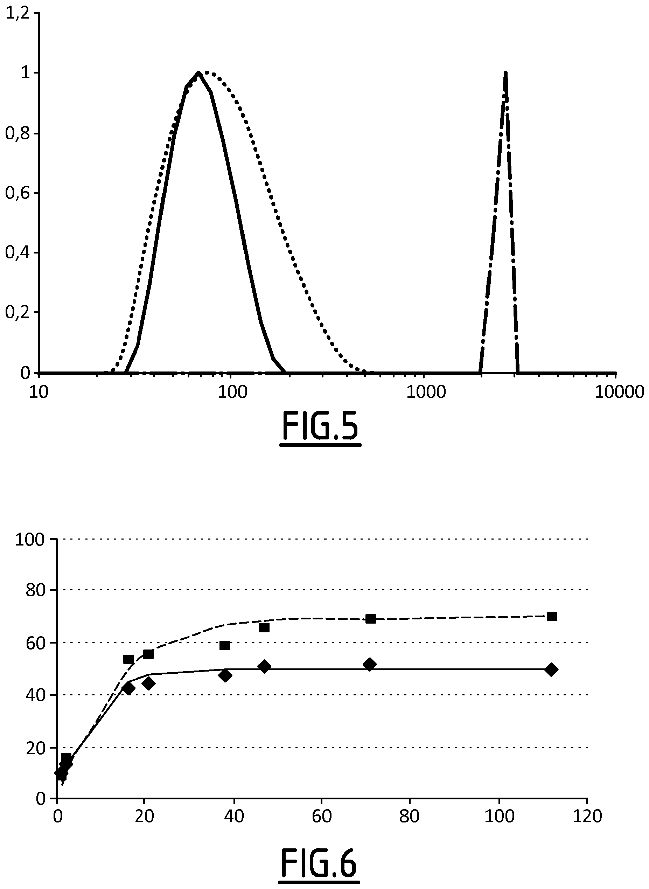

Le caractère colloïdal de l'émulsion ainsi obtenue est vérifié par DLS (diffusion dynamique de la lumière) (Zêta Sizer Nano ZS, Malvern). La taille des gouttelettes en suspension est sensiblement identique pour l'émulsion 1 et pour l'émulsion obtenue après clivage de la fonction disulfure du matériau, alors que la taille des agrégats en suspension (amas de gouttelettes formant le matériau) est bien supérieure (figure 5).

### Exemple 5 : Emulsions comprenant un tensioactif de formule (L) (exemple illustratif ne faisant pas partie de l'invention)

### ◆ Mise en évidence de la possibilité d'introduire plus de tensioactifs de formule (L) dans une émulsion que de tensioactifs de type DSPE-PEG

### • Préparation d'un tensioactif de formule (L) dans laquelle R₂ représente C₁₇H₃₅, A₂ représente O, n représente 100 et Φ représente un groupe succinimidyle

Un tensioactif de formule (L) dans lequel R₂ représente C₁₇H₃₅, A₂ représente O, n représente 100 et Φ représente un groupe succinimidyle 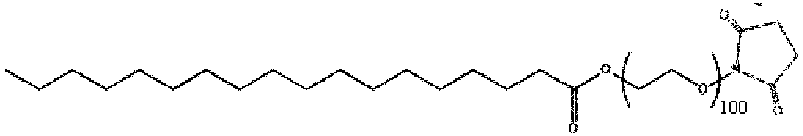 a été prépare en suivant le schéma réactionnel suivant :

Dans un ballon anhydre et sous argon, on a dissout du Myrj S59 (2.345g ; 0,5 mmol) dans du dioxane sec (15 mL) en chauffant pour obtenir une solution limpide. Ensuite, le milieu réactionnel a été ramené à température ambiante (25°C), avant l'ajout du carbonate de disuccinimidyl (0.77g ; 3 mmol) dissous dans l'acétone sec (3 mL). Le 4-diméthylaminopyridine (0.37 g ; 3 mmol), préalablement dissous dans l'acétone sec (3 mL) a alors été ajouté lentement et sous agitation au milieu réactionnel. La réaction a été suivie par CCM (CH₃Cl/MeOH) 5/1. Après 5 heures sous agitation à température ambiante, le produit a été précipité dans le diéthyléther froid (100 mL), et le solide ainsi obtenu a été centrifugé pour être isolé, puis redissous dans l'acétone, précipité de nouveau, et ce à plusieurs reprises. Le tensioactif de formule (L) attendu a été séché sous vide, pour être obtenu sous forme d'une poudre blanche (rendement 72 %).

RMN ¹H (300 MHz ; MeOD) : d : 0,87 (t ; *J*=6,5 Hz ; 3H ; C**H₃**-CH₂) ; 1,25 (m ; 28H ; 14C**H₂**) ; 1,61 (quin ; *J*=7,5 Hz ; 2H ; C**H₂**-CH₂-COO) ; 2,32 (t ; *J*=7,5 Hz ; 2H ; C**H₂**-COO) ; 2.85 (s, 4H, C**H**₂ NHS) ;3,57-3,95 (m ; 362 H ; xC**H₂**(PEG) ; 4.22 (t ; *J*=5 Hz ; 2H ; C**H₂**-OOC-CH₂)

### • Préparation d'une émulsion 3 comprenant le tensioactif de formule (L) et d'une émulsion comprenant un tensioactif dérivé de DSPE-PEG (à titre comparatif)

Des émulsions dans lesquelles les gouttelettes comprennent en surface le groupe fonctionnalisable ester N-hydrosuccinimidyl (NHS) ont été prépérées. Ces émulsions comprennent soit le tensioactif de formule (L) préparé ci-dessus (poids moléculaire d'environ 5000 g mol⁻¹), soit un tensioactif dérivé de DSPE-PEG-NHS (poids moléculaire: 3400 g mol⁻¹) et elles ont été préparées en suivant les procédures décrites dans WO 2010/018223 avec les compositions respectivement indiquées dans les tableaux 4 et 5. 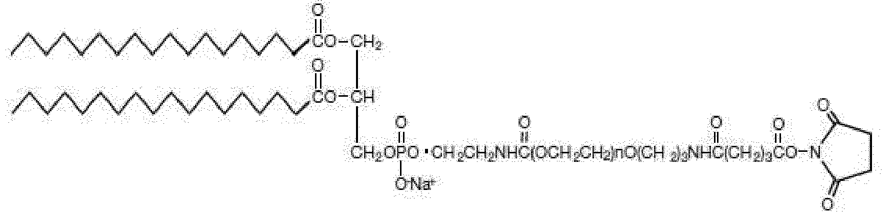 DSPE-PEG-NHS (avec n = 75) de NOF (poids moléculaire : 3400 g mol⁻¹)

**Tableau 4 : Composition de l'émulsion 3 comprenant un tensioactif de formule (L)**

| | % de tensioactif de formule (L) en masse /masse totale de l'émulsion | | | | |
|---|---|---|---|---|---|
| | 4,5% | 8,0% | 12,0% | 15,0% | 30,0% |
| Huile soja purifiée (mg) | 85 | 85 | 85 | 85 | 85 |
| Suppocire NC (mg) | 255 | 255 | 255 | 255 | 255 |
| Lécithine Lipoid s75 (mg) | 65 | 65 | 65 | 65 | 65 |
| Myrj 52 (mg) | 314.8 | 291.4 | 264.5 | 244.4 | 184.1 |
| tensioactif de formule (L) (mg) | 71.3 | 126.8 | 190.1 | 237.7 | 380.3 |
| PBS (µL) | 1208. 9 | 1176.9 | 1140. 3 | 1112. 9 | 1030. 6 |

**Tableau 5 : Composition de l'émulsion comparative comprenant du DSPE-PEG-NHS**

| | % de DSPE-PEG-NHS en masse/ masse totale de l'émulsion | |
|---|---|---|
| | 3.0% | 4.5% |
| Huile soja purifiée (mg) | 85 | 85 |
| Suppocire NC (mg) | 255 | 255 |
| Lécithine Lipoid s75 (mg) | 65 | 65 |
| Myrj 52 (mg) | 322.4 | 314.8 |
| DSPE-PEG-NHS (mg) | 44.1 | 58.8 |
| PBS (µL) | 771.5 | 778.6 |

La préparation d'émulsions comprenant le tensioactif de formule (L) a été possible à tous les pourcentages massiques de tensioactif de formule (L) testés (jusqu'à 30% en masse de surfactant/masse totale testé). Par contre, la préparation d'émulsions comprenant du DSPE-PEG-NHS n'a pas été possible au-delà de 4,5% en masse de DSPE-PEG-NHS car le milieu devenait trop visqueux pour être formulé.

Il est donc possible de préparer une émulsion avec plus de tensioactif de formule (L) que de tensioactif DSPE-PEG-NHS. Ainsi, les gouttelettes d'une émulsion préparée avec le tensioactif de formule (L) ont plus de fonctions NHS en surface.

### • Greffage d'un agent d'intérêt en surface des émulsions préparées ci-dessus

Les gouttelettes des émulsions préparées ci-dessus présentent en surface des groupements NHS fonctionnalisables par des agents d'intérêts comportant un groupement amine (NH₂). Dans l'exemple ci-dessous, on a couplé en surface des gouttelettes un Fluorophore-NH₂ (5-FAM cadavérine).

Pour chacune des émulsions, on a utilisé des volumes d'émulsions tels que le nombre de fonction NHS par litre d'émulsion était de 4 µmol, c'est-à-dire un volume d'émulsion suivant :

**Tableau 6 : volume d'émulsion utilisé pour la fonctionnalisation par la 5-FAM cadavérine**

| % de tensioactif de formule (L) en masse/masse totale de l'émulsion | Volume d'émulsion utilisé |
|---|---|
| | (µL) |
| 4.5 | 864.8 |
| 8 | 488.5 |
| 12 | 324.9 |
| 15 | 260 |

On a mélangé le volume d'émulsion indiqué dans le tableau 6 ci-dessus avec 3,85 mg d'EDC (5 éq., soit 20 µmol) et 10,86 mg de sulfo-NHS (50 µmol) pendant 15 minutes à température ambiante, à l'abri de la lumière (pH=6). 15 µL de 5-FAM cadavérine à 0,40 µM, soit 6 µMoles (1,5 éq à pH=7,4) ont été ajoutés pendant 2h à température ambiante, à l'abri de la lumière (échantillon).

Des témoins ont été préparés en reproduisant ces expériences en mélangeant le volume d'émulsion indiqué dans le tableau 6 ci-dessus avec 3,85 mg d'EDC (5 éq., soit 20 µmol) et 10,86 mg de sulfo-NHS (50 µmol) pendant 15 minutes à température ambiante, à l'abri de la lumière (pH=6). (pas d'ajout ultérieur de 5-FAM cadavérine)

L'expérience a également été reproduite avec l'émulsion préparée à partir du tensioactif DSPE-PEG-NHS.

Les émulsions obtenues ont été dialysées contre PBS 1X stérile (membrane Da=12 400) pendant 16h (Avec changement de l'eau de dialyse 2x).

La quantité de 5-FAM-cadavérine greffée à la surface des gouttelettes a ensuite dosée par spectrophotométrie d'absorbance (Cary 300, Varian) et confirmée par spectrofluorimétrie (LS50B, Perkin-Elmer). Le signal de diffusion éventuel des nanoparticules a été corrigé en soustrayant celui des témoins négatifs incubés dans les mêmes conditions mais sans 5-FAM-cadavérine. Les rendements de greffage (ratio entre nombre de fluorophores greffés à la surface sur nombre de fonctions NHS introduites) sont regroupés dans le tableau 7.

**Tableau 7 : rendements de greffage de la 5-FAM-cadavérine sur des émulsions fonctionnalisées comprenant soit un tensioactif DSPE-PEG-NHS, soit un tensioactif de formule (L)**

| Taille des gouttelettes (nm) | tensioactif | % de tensioactif en masse/ masse totale de l'émulsion | Rendement de greffage (%) |
|---|---|---|---|
| 50 | DSPE-PEG-NHS | 4,5 | 10 |
| 50 | tensioactif de formule (L) | 4,5 | 10 |
| 50 | | 8 | 57 |
| 50 | | 12 | 51 |
| 50 | | 15 | 58 |

En utilisant les émulsions comprenant un tensioactif de formule (L) comprenant plus de fonctions NHS en surface (% de tensioactif en masse/masse totale de l'émulsion plus élevé), on a obtenu un meilleur rendement de greffage. Le nombre de 5-FAM-cadavérine (fluorophore - agent d'intérêt) greffé de façon covalente à la surface des gouttelettes est donc plus important lorsque le tensioactif de formule (L) est utilisé.

### ◆ Comparaison des fuites de tensioactifs de formule (L) avec A₂ représente NH ou O

Des émulsions comprenant des tensioactifs de formule (L) avec A₂ représente NH ou O et greffées par de l'isothiocyanate de fluorescéine (FITC) (agent d'intérêt) ont été préparées.

### • Préparation d'un tensioactif de formule (L) dans laquelle R₂ représente C₁₇H₃₅, A₂ représente O, n représente 100 et Φ représente un groupe amine sur lequel du FITC a été greffé

Le schéma réactionnel suivant a été suivi :

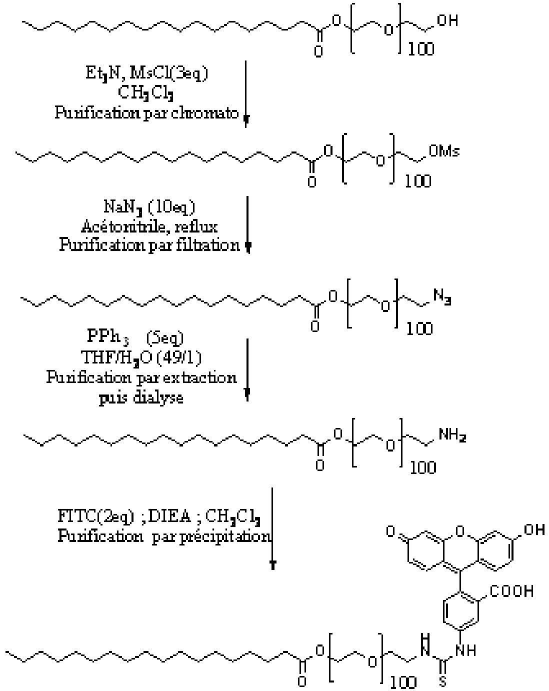

Synthèse de

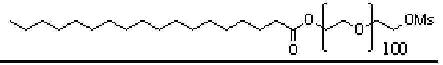

Sous argon du Myrj S100 anhydre (10 g ; 1,98 mmol) et de la triéthylamine (0.8 mL ; 5,94 mmol) ont été dissous dans du dichlorométhane stabilisé sur amylène et anhydre (50 mL). Après 5 minutes sous agitation, la température a été abaissée à 0°C puis du chlorure de mésyle (0,46 mL ; 5,94 mmol) a été ajouté au milieu réactionnel. Après 24 heures à température ambiante, l'excès de chlorure de mésyle a été quenché à froid par de l'éthanol. Après 5 minutes d'agitation, le solvant a été évaporé sous vide. Le produit a ensuite été chromatographié sur colonne de silice avec comme éluant un gradient dichlorométhane / méthanol (9,5/0,5 à 9/1) pour obtenir un solide blanc avec un rendement de 23 %.

RMN 1H (300 MHz ; CDCl3) : d : 0,84 (t ; *J*=6,5 Hz ; 3H ;C**H₃**-CH₂) ; 1,21 (m ; 28 H ; 14C**H₂**) ; 1,57 (quin ; *J*=7,5 Hz ; 2H ; C**H₂**-CH₂-COO) ; 2,28 (t ; *J*=7,5 Hz ; 2H ; C**H₂**-COO) ; 3.04 (s ; 3H ; C**H₃**-S) ; 3,6-3,8 (m ; 360 H ; xC**H₂**(PEG)) ; 4.18 (t ; *J*=5 Hz ; 2H ; C**H₂**-OOC-CH₂) ; 4.33 (m ; 2H ; C**H₂**-OMs)

Synthèse de

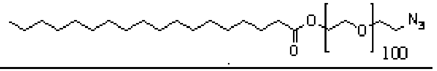

Le composé de l'étape précédente (2,33 g ; 0,45 mmol) et de l'azoture de sodium (0,29 g ; 4,54 mmol) ont été mis en suspension dans de l'acétonitrile (23,3 mL). Le mélange a été porté à 85°C pendant 2 jours. Le solvant a été évaporé, le mélange réactionnel a été redissout dans le dichlorométhane et le NaNs en suspension a été filtré. Le dichlorométhane a été évaporé sous vide pour obtenir le composé attendu sous forme d'une poudre blanche (rendement 93 %).

RMN 1H (300 MHz ; CDCl3) : d : 0,88 (t ; *J*=6,5 Hz ; 3H ; C**H₃**-CH₂) ; 1,25 (m ; 28 H ; 14C**H₂**) ; 1,6 (quin ; *J*=7,5 Hz ; 2H ; C**H₂**-CH₂-COO) ; 2,32 (t ; *J*=7,5 Hz ; 2H ; C**H₂**-COO) ; 3,4 (m ; 2H ; C**H₂**-N₃) ; 3,6-3,8 (m ; 360H ; xC**H₂**(PEG)) ; 4.22 (t ; *J*=5 Hz ; 2H ; C**H₂**-OOC-CH₂)

Synthèse de

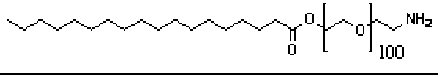

Le composé de l'étape précédente (2,15 g ; 0,42 mmol) et de la triphénylphosphine (560 mg ; 2,12 mmol) ont été dissous dans du THF (20 mL). Après 10 minutes d'agitation, 0,4 mL d'eau ont été ajoutés au milieu réactionnel. Au cours de la réaction, l'apparition de l'amine a été suivie par CCM (CH₂Cl₂ / MeOH 9/1) avec comme révélateur la ninhydrine. Après 2 jours sous agitation, le solvant a été évaporé et le produit a été repris dans l'hexane et extrait avec du méthanol. Les phases méthanols ont été réunies et évaporées à sec. Le produit a été repris dans l'eau, dialysé (pH 6-7) puis lyophilisé pour donner le la composé attendu sous forme de poudre blanche (rendement 90 %).

CCM (CH₂Cl₂/ MeOH 9/1): Rf=0,27

RMN 1H (300 MHz ; CDCl3) : d : 0,88 (t ; *J*=6,5 Hz ; 3H ; C**H₃**-CH₂) ; 1,25 (m ; 28 H ; 14C**H₂**) ; 1,6 (quin ; *J*=7,5 Hz ; 2H ; C**H₂**-CH₂-COO) ; 2,32 (t ; *J*=7,5 Hz ; 2H ; C**H₂**-COO) ; 2.88 (t, *J*=5 Hz ; 2H ; C**H₂**-NH₂) ; 3,53 (t ; *J*=5 Hz; 2H ; OC**H₂**-CH₂-NH₂) ; 3,6-3,8 (m ; 360 H ; xC**H₂**(PEG)) ; 4.22 (t ; *J*=5 Hz ; 2H ; C**H₂**-OOC-CH₂)

Synthèse de

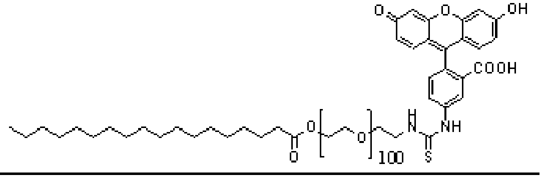

Dans un ballon anhydre et sous argon, on a dissous le composé de l'étape précédente (100 mg ; 0,02 mmol) dans du dichlorométhane (2 mL) et de la DMF (0,1 mL). Après 3 minutes sous agitation, on a ajouté la FITC (isomère 1 à 90 %) (15,4 mg ; 0,04 mmol) et de la DIEA (7 mL ; 0,04 mmol). La réaction a été suivie par CCM (CH₂Cl₂/MeOH/AcOH) 9/1/0,1. Après 10 minutes sous agitation, le solvant a été évaporé sous vide, le produit a été précipité dans l'éther et le solide ainsi obtenu a été lavé par de l'acétate d'éthyle. Le composé attendu a été obtenu sous forme d'une poudre jaune (rendement 76 %).

CCM (CH₂Cl₂/ MeOH 9/1): Rf=0,37

RMN ¹H (300 MHz ; MeOD) : d : 0,91 (t ; *J*=6,5 Hz ; 3H ; C**H₃**-CH₂) ; 1,30 (m ; 28H ; 14C**H₂**) ; 1,62 (quin ; *J*=7,5 Hz ; 2H ; C**H₂**-CH₂-COO) ; 2,35 (t ; *J*=7,5 Hz ; 2H ; C**H₂**-COO) ; 3,55-3,9 (m ; 362 H ; xC**H₂**(PEG) ; C**H₂**-NHFITC) ; 4.22 (t ; *J*=5 Hz ; 2H ; C**H₂**-OOC-CH₂) ; 6,6 (dd ; *J*=2 Hz ; *J*=8,5 Hz ; 2H ; 2CHaro) ; 6,7 (d ; *J*= 2 Hz ; 2H ; 2C**H**aro) ; 6.82 (d ; *J*=8.5 Hz ; 2H ; 2C**H**aro) ; 7,2 (d ; *J*= 8 Hz, 1H ; C**H**aro ) ; 7,86 (d ; *J*= 8 Hz, 1H ; C**H**aro) ; 8,21 (s ; 1H ; C**H**aro)

### • Préparation d'un tensioactif de formule (L) dans laquelle R₂ représente C₁₇H₃₅, A₂ représente NH, n représente 100 et Φ représente un groupe amine sur lequel du FITC a été greffé

La synthèse a été réalisée à partir du composé (C) de l'exemple 1 de formule selon le schéma réactionnel suivant :

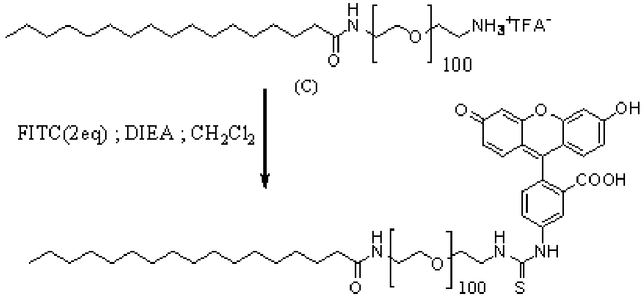

Dans un ballon anhydre et sous argon, on a dissous le composé (C) (250 mg ; 0,05 mmol) dans du dichlorométhane (5 mL) et de la DMF (0,2 mL). Après 3 minutes sous agitation, on a ajouté le FITC (isomère 1 à 90 %) (30 mg ; 0,075 mmol) et de la DIEA (25 mL ; 0,15 mmol). La réaction a été suivie par CCM (CH₂Cl₂/MeOH/AcOH) 9/1/0,1. Après 10 minutes sous agitation, le solvant a été évaporé sous vide, le produit a été précipité dans l'éther et le solide ainsi obtenu a été lavé par de l'acétate d'éthyle. Le solide a été repris dans l'eau à pH 7. Après dialyse (pore 1000 Da), la solution de composé attendu a été lyophilisée et le composé attendu (200 mg) a été obtenu sous forme d'une poudre orange (rendement 76 %).

CCM (CH₂Cl₂/ MeOH 9/1): Rf=0,37

RMN ¹H (300 MHz ; MeOD) : d : 0,91 (t ; *J*=6,5 Hz ; 3H ; CH₃-CH₂) ; 1,31 (m ; 28 H ; 14CH₂) ; 1,62 (quin ; *J*=7,5 Hz ; 2H ; CH₂-CH₂-CONH) ; 2,2 (t ; *J*=7,5 Hz ; 2H ; CH₂-CONH) ; 3,41 (m ; 2H ; CH₂-NHCO) ; 3,55-3.9 (m ; 364 H ; xCH₂(PEG) ; CH₂-NH-FITC) ; 6,56 (m ; J=9 Hz ; J=2,5 Hz ; 4H ; 4CHaro) ; 7,15 (d ; J=9 Hz ; 2H ; 2CHaro) ; 7,2 (d ; J=8.5 Hz ; 1 H ; C**H**aro) ; 7,76 (dd ; *J*=8.5 Hz ; *J*=2,5 Hz ; 1H ; C**H**aro) ; 7,87 (d ; *J*=2,5 Hz ; 1H ; C**H**aro)

### • Préparation d'émulsions 3 comprenant soit le tensioactif de formule (L) dans lequel A₂ représente NH soit le tensioactif de formule (L) dans lequel A₂ représente O

Des émulsions 3 fonctionnalisées par le groupe FITC comprenant les composants du tableau 8 ci-dessous ont été préparées en suivant les procédures décrites dans WO 2010/018223.

**Tableau 8: Composition des émulsions 3 A₂ = NH et 3 A₂ = O**

| | | |
|---|---|---|
| | Emulsion 3 A₂ = NH | Emulsion 3 A₂ = O |
| Huile soja purifiée (mg) | 85 | 85 |
| Suppocire NC (mg) | 255 | 255 |
| Lécithine Lipoid s75 (mg) | 65 | 65 |
| Myrj S40 (mg) | 328 | 328 |
| PBS (µL) | 771.5 | 778.6 |
| C₁₇H₃₅-CO-NH-[(CH₂)₂-O]₁₀₀-(CH₂)₂-NH-FITC (mg) | 17 | - |
| C₁₇H₃₅-CO-O-[(CH₂)₂-O]₁₀₀-(CH₂)₂-NH-FITC(mg) | - | 17 |

Des émulsions comprenant des gouttelettes de 50 nm de diamètre ont été obtenues dans chaque cas.

### • Fuite des tensioactifs hors des gouttelettes des émulsions 3 formées

On a analysé la désorption des tensioactifs lors d'une dialyse de 48 heures. Les tensioactifs ont été quantifiés dans le dialysat par mesure de l'absorbance à 490 nm (les tensioactifs dans le dialysat correspondent à ceux ayant fui hors de gouttelettes). Les conditions de dialyses étaient les suivantes : 400 mL d'émulsion 3 à 20 % (w/w) dans un « Quick dialyser», puis mise en dialyse dans 400 mL de PBS. La dialyse a duré 120 heures. La fuite de tensioactif (C₁₇H₃₅-CO-NH-[(CH₂)₂-O]₁₀₀-(CH₂)₂-NH-FITC ou C₁₇H₃₅-CO-O-[(CH₂)₂-O]₁₀₀-(CH₂)₂-NH-FITC) a été suivie en prélevant régulièrement 300 mL du dialysat. Les résultats sont illustrés sur la figure 6.

Ainsi, après 48 heures de dialyse, on constate que 50% du tensioactif C₁₇H₃₅-CO-NH-[(CH₂)₂-O]₁₀₀-(CH₂)₂-NH-FITC ont fui hors des gouttelettes de l'émulsion 3 A₂ = NH alors que 70% de tensioactif C₁₇H₃₅-CO-O-[(CH₂)₂-O]₁₀₀-(CH₂)₂-NH-FITC ont fui hors des gouttelettes de l'émulsion 3 A₂ = O. Le tensioactif avec A₂ = NH désorbent donc moins des gouttelettes que le tensioactif avec A₂ = O.

### Exemple 6 : Emulsion comprenant un matériau comprenant un tensioactif de formule (I) comprenant un groupe G comprenant une fonction clivable non réversible

Dans cet exemple, on met en oeuvre un composé homo-bifonctionnel pour former le surfactant de formule (I) permettant de générer la liaison covalente entre les gouttelettes.

Les gouttelettes portant en surface une fonction amine primaire réactive sont mises à réagir avec un composé dialdéhyde, le glutaraldéhyde, pour former une base de Schiff (imine), laquelle est ensuite réduite (amination réductrice). L'amine secondaire formée forme alors une liaison covalente non réversible entre les gouttelettes.

Plus spécifiquement, la réaction mise en jeu est du type illustré dans le schéma ci-dessous:

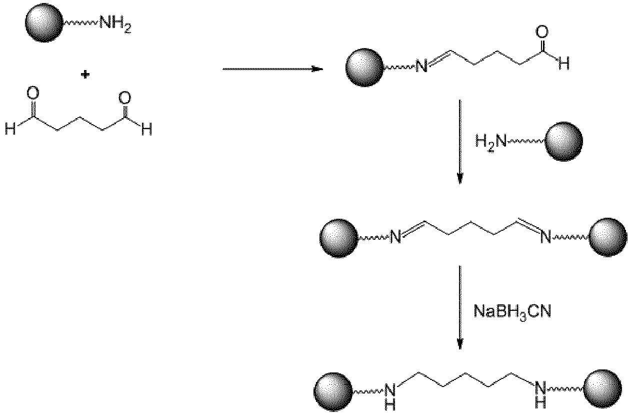

On a préparé une émulsion comportant des gouttelettes portant en surface un groupe amine primaire terminale en suivant le mode opératoire de l'exemple 1, avec les composants pour la phase huileuse et aqueuse tels que détaillés dans le tableau 9 ci-dessous. Après purification, l'émulsion LNP-NH2 obtenue comporte 11 % w/w de phase dispersée.

A 1 mL d'émulsion ainsi obtenue, on a ajouté 0.28 µl de glutaraldéhyde à 50% dans l'eau et on laisse réagir à température ambiante pendant 1h. On a ajouté ensuite à nouveau 1 mL d'émulsion puis on a laissé réagir pendant 12 h avant d'ajouter 282 µl de cyanoborohydrure de sodium en solution à 10 mM (NaCNBH₃).

On a observé dans le mélange réactionnel la formation d'agrégats visibles à l'œil nu. Ces agrégats peuvent être isolés en pipetant du tube délicatement le milieu réactionnel contenant les gouttelettes n'ayant pas réagi. On obtient un gel sur les parois du tube.

Lorsque le gel formé a été remis en contact avec du tampon phosphate (PBS 1X) frais, on n'a observé aucune dissolution due à la dilution, mettant en évidence l'existence d'un gel chimique. Si l'on soumet ce gel à une agitation intense, tel que le vortex, on observe que le gel se désintègre en agrégats fins pour se reformer après 1h30 de repos à la surface de la solution tampon.

**Tableau 9: Composition de l'émulsion LNP-NH2**

| | masse (mg) | | | | | |
|---|---|---|---|---|---|---|
| | Phase lipidique | | | Phase aqueuse | | |
| | Huile soja purifiée | Cire Suppocire NC | Lécithine Lipoid s75 | Myrj^{®} S40 | SA-PEG(100)-NH₂ (composé C de l'exemple 1) | PBS 1X |
| | 68 | 272 | 65 | 312 | 33 | 771.5 |

### Exemple 7 : Formation de gel à partir d'une émulsion LNP-NH₂, LNP-SH et un cross-linker sulfo-SMCC

Dans cet exemple, on met en oeuvre un cross-linker (Succinimidyl 4-(N-maleimidomethyl)cyclohexane-1-carboxylate, sulfo-SMCC) pour générer une liaison covalente entre des gouttelettes portant des groupes -NH₂ (LNP-NH2) en surface et d'autres portant des groupes -SH en surface (LNP-SH) et former un gel.

Les réactions mises en jeu sont illustrées dans le schéma ci-dessous.

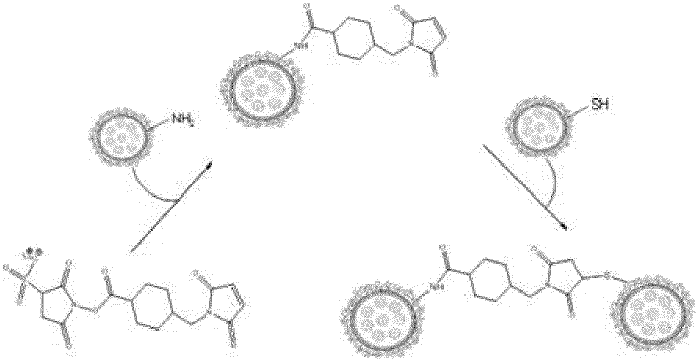

### A. Préparation d'une émulsion LNP-SH

La phase huileuse a été préparée comme dans l'exemple 1, en utilisant les ingrédients et proportions indiquées dans le tableau 10 ci-dessous. Un peu de dichlorométhane a été ajouté pour améliorer la solubilité dans la phase huileuse, il a été évaporé après. Un fluorophore (DiD) a été ajouté au prémix (80 µL, 10 mM dans l'éthanol) pour permettre une meilleure visualisation ultérieure des gouttelettes.

**Tableau 10A: Composition de l'émulsion LNP-SH**

| | masse (mg) | | | | | |
|---|---|---|---|---|---|---|
| | Phase lipidique | | | Phase aqueuse | | |
| | Huile soja purifiée | Cire Suppocire NC | Lécithine Lipoid s75 | Myrj^{®} S40 | SA_{CONH}PEG100-SSpyr | PBS 1X |
| | 68 | 272 | 65 | 276 | 69 | 2500 |

La phase aqueuse a été préparée comme dans l'exemple 1, en utilisant les ingrédients et proportions indiquées dans le tableau 10A ci-dessous. Le surfactant SA_{CONH}PEG100-S-S-Pyr (surfactant LII de l'exemple 1) est ajouté à hauteur de 20 % massique par rapport au total de surfactant LII. Pour la dissolution des surfactants, le mélange a été chauffé à 55°C.

L'émulsion a été préparée en ajoutant la phase aqueuse obtenue dans le flacon contenant la phase huileuse (encore chaude à 45°C), puis en soniquant le mélange à 50°C dans les conditions indiquées dans le tableau 2 ci-dessus, mais avec une puissance Pmax de 30%.

Ensuite, 46 mg de DTT (dithiotréitol soit 20 éq molaire) ont été ajoutés à la suspension de LNP-SS-PYR puis la dispersion de particules a été agité pendant 2 heures sur un plateau mobile afin de réduire les fonctions S-S-PYR sous forme thiol (-SH).

Enfin, les LNP-SH sont dialysées contre du PBS 1X à 2 reprises (MW Cut off 12000-14000 Da ; 500 mL ; 24 heures). Après dialyse, les gouttelettes LNP-SH sont filtrées sur des filtres de 0.2 µm.

### B. Préparation d'une émulsion LNP-NH₂

La phase huileuse a été préparée comme décrit précédemment pour l'émulsion LNP-SH, en utilisant les mêmes ingrédients et proportions.

La phase aqueuse a été préparée comme dans l'exemple 1, en utilisant les ingrédients et proportions indiquées dans le tableau 10B ci-dessous. Le surfactant SA_{CONH}PEG100-NH₃⁺TFA⁻ (surfactant C de l'exemple 1) est ajouté à hauteur de 20 % massique par rapport au total de surfactant C. Pour la dissolution des surfactants, le mélange a été chauffé à 55°C.

**Tableau 10B: Composition de l'émulsion LNP-NH2**

| | masse (mg) | | | | | |
|---|---|---|---|---|---|---|
| | Phase lipidique | | | Phase aqueuse | | |
| | Huile soja purifiée | Cire Suppocire NC | Lécithine Lipoid s75 | Myrj^{®} S40 | SA_{CONH}PEG100-NH3⁺TFA⁻ | PBS 1X |
| | 85 | 272 | 65 | 276 | 69 | 2500 |

L'émulsion a été préparée en ajoutant la phase aqueuse dans le flacon contenant la phase huileuse (encore chaude à 45°C) puis en soniquant le mélange à 50°C dans les conditions indiquées dans l'exemple précédent.

L'émulsion LNP-NH₂ ainsi obtenue a été dialysée contre du PBS 1X à 2 reprises (MW Cut off 12000-14000 Da ; 500 mL ; 24 heures). Après dialyse, les gouttelettes LNP-NH2 sont filtrées sur des filtres de 0.2 µm.

Une solution du Sulfo-SMCC (Succinimidyl 4-(N-maleimidomethyl)cyclohexane-1-carboxylate, Pierce, 0.87 mL, 4.58 mM, 4 µmol) a été ajoutée à la dispersion de LNP-NHₐ⁺TFA⁻ (0.1 mL, 0.4 µmol de fonctions amine). Le mélange a été agité pendant 3 heures sur un plateau mobile. Ensuite, une purification par colonne d'exclusion stérique (Disposable PD-10, GE Healthcare) a été réalisée pour éliminer le Sulfo-SMCC en excès. Après purification par colonne, les fractions contenant les gouttelettes ont été rassemblées.

### C. Préparation d'un gel à partir de LNP-SH et LNP-NH₂

Afin de préparer le gel, l'émulsion LNP-NH₂ obtenue ci-dessus a été ajoutée à l'émulsion LNP-SH (0.1 mL, 0.4 µmol de fonctions thiols). Après 2 heures de réaction sous agitation, on a constaté la formation d'agrégats de gel insolubles dans l'eau.

### Exemple 8 : Formation de gel à partir de LNP-SS-Pyr et cross-linker SH-PEG-SH

On a préparé une émulsion contenant le tensioactif SA_{CONH}PEG₁₀₀-SPDP (surfactant LII de l'exemple 1) de composition indiquée dans le tableau 11 ci-dessous, selon le protocole indiqué dans l'exemple précédent.

L'émulsion obtenue a été purifiée par dialyse (MWCO 12-14 kDa) contre du PBS et diluées de façon à obtenir une fraction massique en gouttelettes de 20%.

**Tableau 11: Composition de l'émulsion LNP-SPDP**

| | masse (mg) | | | | | |
|---|---|---|---|---|---|---|
| | Phase lipidique | | | Phase aqueuse | | |
| | Huile soja purifiée | Cire Suppocire NC | Lécithine Lipoid s75 | Myrj^{®} S40 | SA_{CONH}PEG₁₀₀-SPDP (composé LII de l'exemple 1) | PBS 1X |
| | 150 | 450 | 45 | 172 | 43 | 1140 |

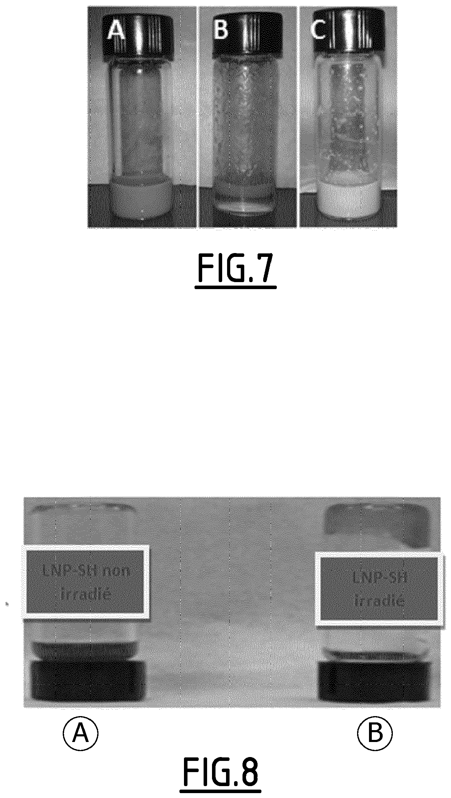

Une quantité de 500 µL de cette émulsion (100 mg de gouttelettes dispersées) ont été mises à réagir avec 0.9 mg de poly(éthylène glycol) dithiol (masse moléculaire moyenne 1000 Da) à température ambiante avec un ratio thiol/SPDP de 1:1 sous agitation modérée. Un gel a commencé à se former quelques minutes après le mélange des réactifs. Le gel formé a été détruit par ajout d'un excès de DTT (dithiothréitol) pour redonner les gouttelettes en dispersion dans le tampon aqueux. Les photographies de la Figure 7 illustrent les changements d'aspect de la formulation LNP-SPDP initiale (Fig. 7A), après ajout de PEG dithio (Fig. 7B) et après ajout de DTT (Fig.7C).

L'étude de la taille de particules dans le gel puis après destruction met en évidence que le processus n'a pas modifié la morphologie des gouttelettes (voir tableau 12 ci-dessous).

**Tableau 12 : propriétés physicochimiques**

| | Diamètre hydrodynamique (nm) | Pdl |
|---|---|---|
| LNP-SPDP (avant gel) | 128 ± 2 | 0.114 ± 0.001 |
| LNP-SH (après gel) | 135 ± 2 | 0.097 ± 0.013 |

Le principe de formation et de la destruction du gel est schématisé dans les schémas A et B ci-dessous.

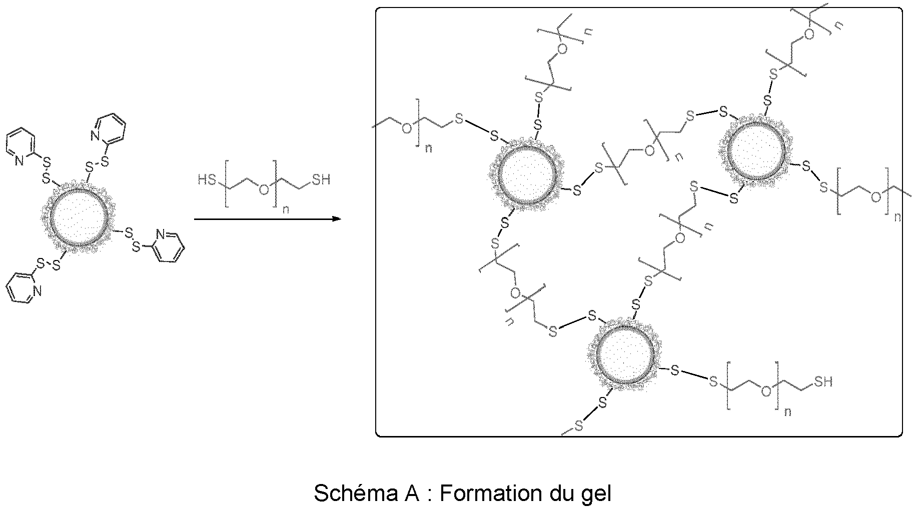

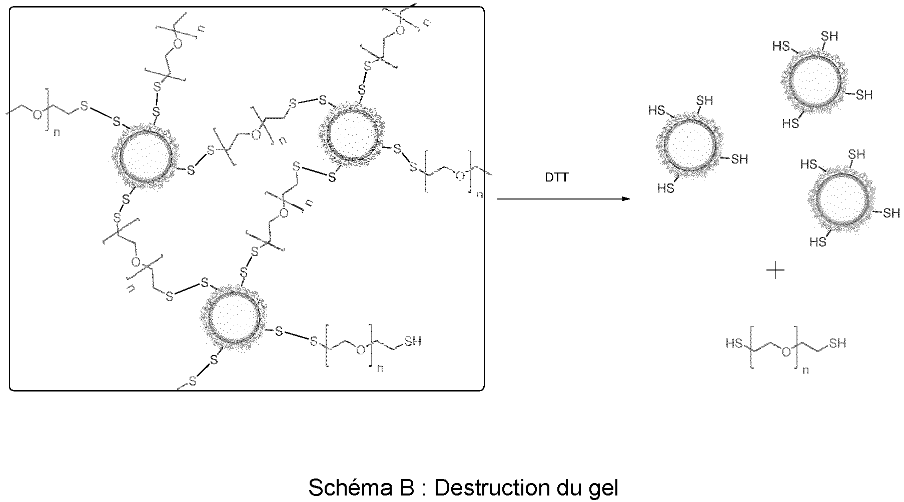

### Exemple 9 : Formation de gels par irradiation

### A. Préparation d'une émulsion de LNP-SH

La phase huileuse a été préparée comme dans l'exemple 1, en utilisant les ingrédients et proportions indiquées dans le tableau 12 ci-dessous. Un peu de dichlorométhane a été ajouté pour améliorer la solubilité dans la phase huileuse, il a été évaporé après. Un fluorophore (DiD) a été ajouté au prémix (80 µL, 10 mM dans l'éthanol) pour permettre une meilleure visualisation ultérieure des particules.

La phase aqueuse a été préparée comme dans l'exemple 1, en utilisant les ingrédients et proportions indiquées dans le tableau 12 ci-dessous. Le surfactant SA_{CONH}PEG100-S-S-Pyr (surfactant LII de l'exemple 1) est ajouté à hauteur de 20 % massique par rapport au total de surfactant LII. Pour la dissolution des surfactants, le mélange a été chauffé à 55°C.

L'émulsion a été préparée en ajoutant la phase aqueuse obtenue dans le flacon contenant la phase huileuse (encore chaude à 45°C), puis en soniquant le mélange à 50°C dans les conditions indiquées dans le tableau 2 ci-dessus, mais avec une puissance Pmax de 30%.

Enfin, les LNP-SH sont dialysées contre du PBS 1X à 2 reprises (MW Cut off 12000-14000 Da ; 500 mL ; 24 heures). Après dialyse, les gouttelettes LNP-SH sont filtrées sur des filtres de 0.2 µm.

**Tableau 13: Composition de l'émulsion LNP-SH**

| | masse (mg) | | | | | |
|---|---|---|---|---|---|---|
| | Phase lipidique | | | Phase aqueuse | | |
| | Huile soja purifiée | Cire Suppocire NC | Lécithine Lipoid s75 | Myrj^{®} S40 | SA_{CONH}PEG₁₀₀-SSPyr (composé LII de l'exemple 1) | PBS 1X |
| | 68 | 272 | 65 | 276 | 69 | 2500 |

### B. Préparation d'un gel sous l'action de la lumière

De l'émulsion de LNP-SH obtenue ci-dessus (pourcentage massique 23 %), on a introduit 1 mL dans un flacon. Ensuite, on a irradié l'échantillon à 365 nm avec une puissance de 15 mW (lampe MA750 OSRAM, HBO).

Après 1h30 d'irradiation, on a constaté la formation d'un gel chimique insoluble dans le tampon PBS 1X (Figure 8).

Le mécanisme de gélification est attribué à l'irradiation des fonctions thiols à la surface des gouttelettes, conduisant à la formation de radicaux -S° à la surface des LNP, lesquels sont susceptibles de conduire à la formation de ponts disulfure liant les gouttelettes entre elles.

Afin de vérifier si cette gélification est due à la présence de fonctions thiols à la surface des gouttelettes lipidiques, on a répété l'exemple mais en remplaçant les gouttelettes LNP-SH par des gouttelettes standards (LNP avec des fonctions terminales - OH et non -SSPyr). Après irradiation pendant 1h30, on n'a constaté ni formation de gel ni changement de viscosité.

Par ailleurs, il a été constaté que le gel est détruit lorsque les ponts disulfures sont réduits. En effet, lorsque l'on a fait réagir les gels LNP-S-S-LNP photo-formés avec le DTT (dithiotréitol, 10 équivalents par rapport au nombre de fonctions SH), on a constaté que le gel est redevenu liquide après deux minutes d'agitation.

### C. Mesures rhéologiques

Afin d'étudier l'évolution de la formation du gel chimique au cours du temps d'irradiation, on a utilisé un rhéomètre oscillant (AR2000 EX, TA Instruments) permettant de mesurer les modules de viscosité et d'élasticité de l'échantillon, à une fréquence d'oscillation de 1 Hz, pendant son irradiation (360 nm, puissance de 60 mW/cm²), en disposant 300 µL d'émulsion LNP-SH sur la surface en Quartz au-dessus de la lampe UV.

Au cours du temps d'irradiation, on a observé une augmentation à la fois du module de viscosité (G ") et du module d'élasticité (G'), indicatif de la gélification. Après 80 minutes d'irradiation, on a constaté que G' est devenu supérieur à G", et delta, défini tel que tangente (delta) = G'/G", est devenu inférieur à 45 °C, ce qui indique le passage d'un liquide viscoélastique à un solide viscoélastique.

La même expérience réalisée avec l'émulsion de gouttelettes LNP portants des fonctions hydroxyles terminales (LNP-myrj s100) permet de vérifier qu'aucune évolution des propriétés rhéologiques n'a lieu au cours de l'irradiation.

### Exemple 10 : Formation de gels à partir de SA-PEG-ONHPOC

### A. Synthèse du surfactant SA_{CONH}PEG100NHCO-ONH-POC

Il s'agit d'un surfactant de type polyéthylène glycol (100 unités) lié à l'une extrémité à une chaîne grasse (C18) via une fonction amide et à l'autre extrémité à une autre fonction amide liée à la fonction oxyamine protégée par un groupement NPPOC (2-(2-nitrophényl)-propyl).

Sa structure (produit 3) et son schéma de synthèse sont détaillés ci-dessous.

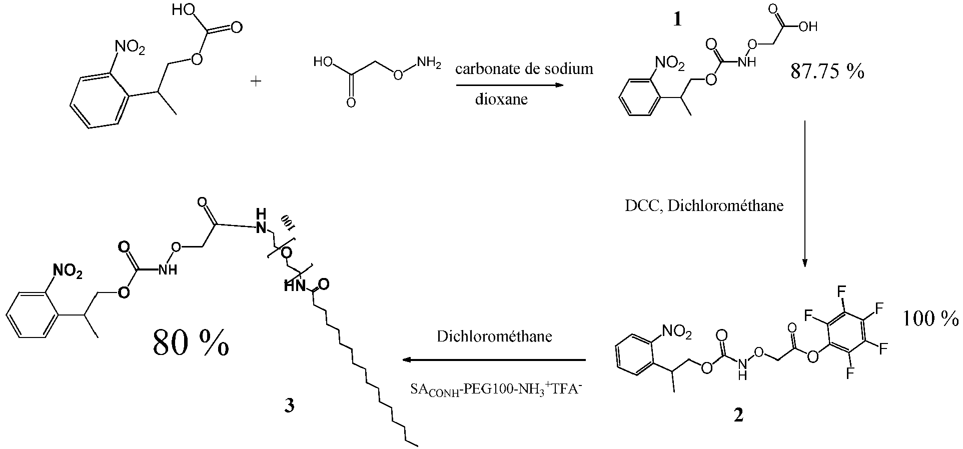

### Synthèse du produit 1

Du chlorydrate de carboxyméthoxylamine (1 g, 4.57 mmol) a été dissout dans une solution aqueuse de carbonate de sodium à 10 % (25 mL). La solution a été refroidie à 0°C et l'on a ajouté, goutte à goutte, une solution de chloroformiate de 2-(2-nitrophényl)-propyl (CI-NPPOC) (2.20 g, 9.1 mmol) dans du dioxane (20 mL). L'agitation a été maintenue pendant 3 heures à température ambiante. Ensuite, on a évaporé à sec le milieu réactionnel. Au résidu obtenu, on a additionné de l'eau (250 mL) et on a lavé la phase aqueuse avec l'éther diéthylique (200 mL). La phase aqueuse a été acidifiée avec une solution aqueuse d'acide chlorhydrique 1N jusqu'à pH 3 et extraite avec du dichlorométhane (3 × 250 mL). Enfin, les phases organiques ont été réunies et séchées sur sulfate de sodium anhydre, et évaporées.

Le produit brut a été purifié par chromatographie sur gel de silice (dichlorométhane puis dichlorométhane/ méthanol 97/3, v/v). Le produit 1 a été obtenu sous forme d'une poudre blanche (1.17 g, 3.9 mmol, 87.75 %).

RMN-¹H (200 MHz, CDCl₃):□δ ppm 1.34 (3 H, d, CH₃); 3.5 (1H, m, CH); 3.76 (2 H, d, CH₂O); 4.37 (s, 2 H, COCH₂O); 7.3-7.6 (4 H_{Ar}, m); 8.8 (1 H, s, COOH).

### Synthèse du produit 2

Le produit 1 (1.17 g, 3.9 mmol) a été dissout dans le dichlorométhane (5 mL) et on l'on a ajouté du pentafluorophénol (906,5 mg, 4.63 mmol), puis goutte à goutte une solution de DCC (877.26 mg, 4.63 mmol) dans le dichlorométhane. Le mélange a été agité pendant 4 heures et ensuite filtré. Après évaporation, le produit 2 a été obtenu sous forme d'huile jaune (1.8 g, 4.63 mmol, 100 %).

RMN-¹H (200 MHz, CDCl₃):□ δ ppm 1.34 (3 H, d, CH₃); 3.5 (1H, m, CH); 3.76 (2 H, d, CH₂O); 4.37 (s, 2 H, COCH₂O); 7.3-7.6 (4 H_{Ar}, m).

### Synthèse du produit 3

Sous argon, du SA_{CONH}PEG100-NH₃⁺TFA⁻ (surfactant C de l'exemple 1, 519.14 mg ; 0,1 mmol) et de la DIEA (25 µL, 0.2 mmol) ont été dissout dans du dichlorométhane (10 mL). Après 5 mn d'agitation, on a ajouté au milieu réactionnel le produit 2 (50.54 mg, 0.13 mmol). Après 2 heures de réaction, le solvant a été évaporé. Le produit brut a été purifié par dialyse dans l'eau distillée (MW Cut off 1000 Da ; 2 L ; 48 heures). Enfin, le produit 3 a été lyophilisé pour conduire à l'obtention d'une poudre blanche (428.53 mg, 0.08 mmol, 80 %).

RMN-¹H (200 MHz, CDCl₃):□ δ ppm 0.88 (3 H, t, CH₃); 1.25 (28 H, m, 14 CH2); 1.34 (3 H, d, CH₃); 1.89 (2 H, m, CH₂-CH₂-CONH); 2.17 (2 H, t, CH2-CONH); 3.34 (2 H, m, CH2-NH₃⁺); 3.4 (1H, m, CH); 3.5-3.70 (400 H, m, CH₂ PEG) ; 3.76 (2 H, d, CH₂O); 4.37 (s, 2 H, COCH₂O); 617 (1 H, s, NH) ; 7.3-7.6 (4 H_{Ar}, m).

### B. Préparation de l'émulsion de LNP- ONH-POC

La phase huileuse a été préparée comme dans l'exemple 1, en utilisant les ingrédients et proportions indiquées dans le tableau 14 ci-dessous. Un peu de dichlorométhane a été ajouté pour améliorer la solubilité dans la phase huileuse, il a été évaporé après. Un fluorophore (DiD) a été ajouté au prémix (80 µL, 10 mM dans l'éthanol) pour permettre une meilleure visualisation ultérieure des particules.

La phase aqueuse a été préparée comme dans l'exemple 1, en utilisant les ingrédients et proportions indiquées dans le tableau 12 ci-dessous. Le surfactant SA_{CONH}PEG100NHCO-ONH-POC est ajouté à hauteur de 20 % massique par rapport au total de surfactant PEGylé. Pour la dissolution des surfactants, le mélange a été chauffé à 55°C.

L'émulsion a été préparée en ajoutant la phase aqueuse obtenue dans le flacon contenant la phase huileuse (encore chaude à 45°C), puis en soniquant le mélange à 50°C dans les conditions indiquées dans le tableau 2 ci-dessus, mais avec une puissance Pmax de 30%.

Enfin, les gouttelettes sont dialysées contre du PBS 1X à 2 reprises (MW Cut off 12000-14000 Da ; 500 mL ; 24 heures). Après dialyse, les gouttelettes sont filtrées sur des filtres de 0.2 µm.

**Tableau 14: Composition de l'émulsion à surfactant à groupement NPPOC**

| | masse (mg) | | | | | |
|---|---|---|---|---|---|---|
| | Phase lipidique | | | Phase aqueuse | | |
| | Huile soja purifiée | Cire Suppocire NC | Lécithine Lipoid s75 | Myrj^{®} S40 | SA_{CONH}PEG₁₀₀-NCOONH-POC | PBS 1X |
| | 68 | 272 | 65 | 275 | 70 | 2500 |

### C. Caractérisation

La taille et la charge des LNP- ONHPOC ont été mesurées par DLS (Diffusion quasi-élastique de la lumière) par un Nanosizer (Zetasizer Malvern).

**Tableau 15 : Caractérisation des gouttelettes**

| | Diamètre moyen | Indice de polydispersité | Potentiel Zêta (mv) dans l'eau | Potentiel Zêta (mv) dans PBS 0.1X |
|---|---|---|---|---|
| 3.25 mL | 54 | 0.119 | -3.51 | -3.3 |

### D. Mise en évidence des fonctions ONH-POC à la surface des LNP

Afin de mettre évidence la présence des fonctions oxyamines protégées à la surface des LNP, un échantillon (0.6 mL, 3.96 mM, 2.38 µmol de fonctions -ONH-POC) a été introduit dans un flacon puis on y a ajouté quelques gouttes de NaOH pour rendre le milieu plus basique (pH 9), ce qui favorise la déprotection des oxyamines sous irradiation.

La taille et le potentiel zêta des particules ont été mesurés après irradiation pour voir l'effet du pH et de la lumière sur les particules. Les résultats sont consignés dans le tableau 16 ci-dessous.

On constate que la photo-déprotection des fonctions -ONHPOC a été réalisée sans modifier significativement la taille et la charge de surface des nanoparticules.

**Tableau 16 : taille et potentiel zêta après déprotection des fonctions oxyamines**

| | Diamètre moy (nm) | Indice de polydispersité | Potentiel Zêta (mv) |
|---|---|---|---|
| SACONH-PEG100-ONHPPOC pH 7,4 | 54 | 0,119 | -3,3 |
| SACONH-PEG100-ONHPPOC H 9 ; hv 10 mn | 55 | 0,161 | -1,9 |

Comme les fonctions oxyamines (O-NH₂) réagissent très vite avec les fonctions aldéhydes, la formation des fonctions -ONH₂ a été mise en évidence par réaction avec la fluorescéine-aldéhyde de formule ci-dessous (6.6 mM, 1.08 mL, 7.13 µmol).

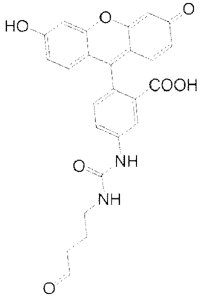

Les deux réactifs ont été mis en présence pendant 2 h sous agitation sur un plateau mobile à l'abri de la lumière. Ensuite, une purification par colonne d'exclusion stérique (Sephadex G-25 Medium, GE Healthcare) a été réalisée pour se débarrasser de toutes les molécules fluorescéines qui n'ont pas réagi avec les LNP- ONH₂.

Les fractions d'élution recueillies ont été analysées par spectrofluorimétrie sur un lecteur de plaques Tecan (Tecan Infinité M1000). Les analyses ont été réalisées dans deux plages de longueurs d'onde : 1) avec une excitation à 490 nm et le recueil de l'émission à 520 nm (signaux dus à la fluorescéine du réactif CHO-fluorescéine) ; 2) avec une excitation à 640 nm et le recueil de l'émission à 690 nm (signaux dus au DiD inclus dans le coeur des particules).

Le protocole a été répété avec une émulsion de LNP-Myrj S100 ne portant pas de fonctions ONH-POC à leur surface. Par ailleurs, on a fait réagir des LNP-ONHPOC n'ayant pas été irradiées et donc pas déprotégées, avec la fluorescéine-aldéhyde dans les même proportions que précédemment. Enfin, le protocole a été répété après avoir inhibé les fonctions oxyamines (-ONH₂) déprotégées avec de l'acétone avant de les mettre en contact avec la fluorescéine-aldéhyde.

On constate à partir des profils d'élution obtenus avec les deux types de gouttelettes que les fonctions -ONH-POC présentes à la surface des gouttelettes, irradiées et donc déprotégées pour aboutir aux fonctions -O-NH₂, ont pu réagir avec la fonction aldéhyde du fluorophore fluorescéine aldéhyde (co-élution de la fluorescéine-greffée à la surface des nanoparticules et du DiD inclus dans leur coeur). En revanche, aucune réactivité vis-à-vis de la fluorescéine-aldéhyde a été mise en évidence pour les contrôles négatifs.

### E. Gélification des LNP-ONHPOC après photo-déprotection et ajout de glutaraldéhyde

Une quantité de 0.6 mL de formulation de LNP-ONHPOC (3.66 mM) obtenue ci-dessus a été déprotégée en présence de NaOH (pH 9) par irradiation pendant 10 minutes à 365 nm (15 mW, lampe MA750 OSRAM, HBO).

A cette solution ont été ajoutés 2 µL de solution de glutaraldéhyde à 50 % dans l'eau (soit 22 µmol et 10 équivalents de fonctions aldéhyde par rapport aux fonctions - ONHPOC déprotégées en -ONH₂). On a constaté un changment de coloration - de bleu à rouge - et la formation d'un gel.

Le mécanisme de la gélification supposé est illustré dans le schéma ci-dessous.

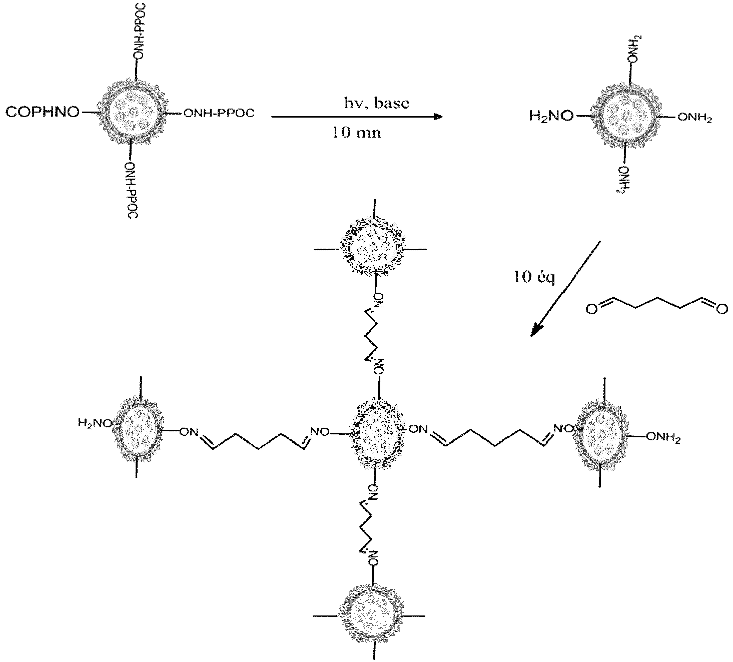

Le changement de couleur constaté lors de l'étape d'irradiation (déprotection des fonctions oxyamines) est attribué à une dégradation du fluorophore DiD encapsulé dans le coeur des nanoparticules lors de l'irradiation en milieu basique.

Le gel reste stable même après plusieurs heures après ajout de tampon PBS 1X, ce qui met en évidence l'obtention d'un gel chimique.

### Exemple 11 : Formation de gels avec un tensioactif à fonction maléimide

### A. Synthèse de SA-PEG-maléimide

Il s'agit du surfactant PEG à 100 unités d'éthylènes glycol, dont une extrémité est liée à l'acide stéarique (C18) par une liaison amide et l'autre extrémité à la fonction maléimide par une deuxième liaison amide.

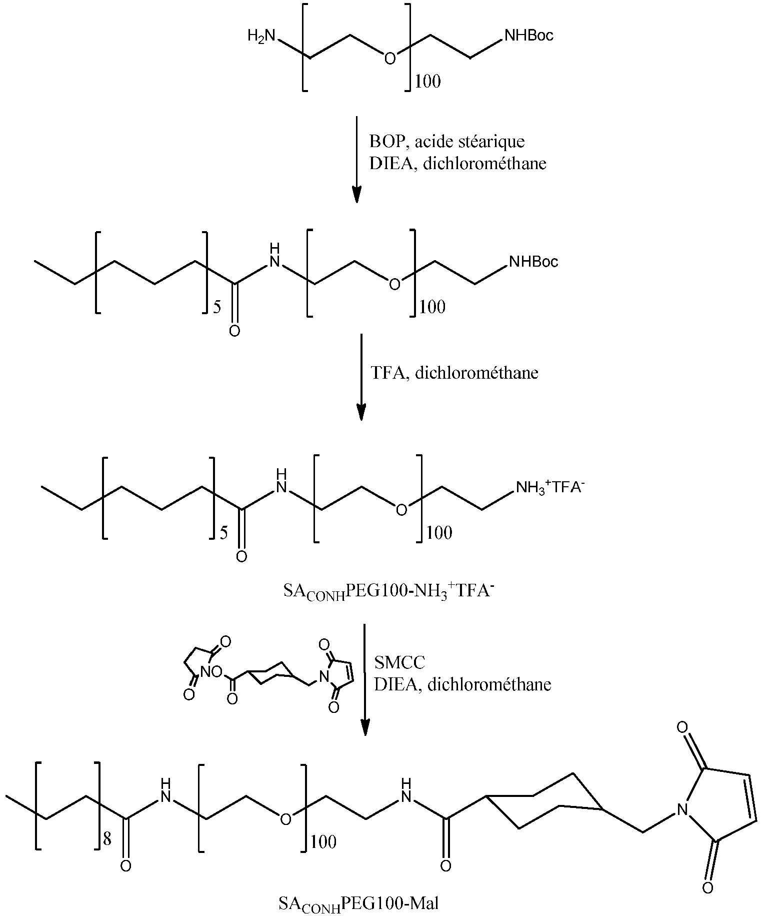

La synthèse de ce composé est similaire à celle du SA_{CONH}PEG100-SPDP (ou SSPyr, surfactant LII de l'exemple 1). Comme illustré dans le schéma ci-dessus, l'intermédiaire commun SA_{CONH}PEG100-NH₃⁺TFA⁻ (surfactant C de l'exemple 1), lequel est obtenu en deux étapes (couplage du PEG-NHBoc-NH₂ avec l'acide stéarique puis déprotection du groupement Boc), une substitution nucléophile de ce dernier sur le réactif SMCC (« maléimide-cyclohexane--NHS ») conduit au produit désiré SA_{CONH}PEG100-Mal.

### Synthèse du produit SA_{CONH}PEG100-Mal

Sous argon, du SA_{CONH}PEG100-NH₃⁺TFA⁻ (PM : 5191,44 ; 0,1 g ; 0,02 mmol) et de la DIEA (PM : 129,25 ; 5 µL ; 2 éq. ; 0,04 mmol) sont dissous dans du dichlorométhane (2 mL). Après 5 minutes sous agitation du SMCC (PM: 334,32 ; 20 mg ; 0,06 mmol ; 3 éq) est ajouté dans le milieu réactionnel. La disparition de l'amine est suivie par CCM (CH₂Cl₂ / MeOH 9/1). Après 1 heure de réaction, et évaporation du solvant, le produit est précipité à deux reprises dans l'éther pour donner après filtration mg de SA_{CONH}PEG100-Mal (poudre blanche).

CCM (CH₂Cl₂/ MeOH 9/1): Rf = 0,25

RMN ¹H (300 MHz ; CDCl₃) : d : 0,88 (t ; C**H**₃-CH₂) ; 1,25 (m ; C**H**₂ stéarate) ; 1,50 (m; C**H**₂ du cyclohexane); 1,60 (m ; C**H**₂-CH₂-CONH) ; 1,9 (m ; cyclo-C**H**-CH₂-) ; 2,20 (t ; C**H**₂-CONH) ; 3,42 (m ; C**H**₂-NHCO) ; 3,45 (m ; C**H**₂-Mal) ; 3,48-3,8 (m ; xC**H**₂(PEG) ; C**H**₂-NHCO) ; 6,11 (bt ; N**H**) ; 6,70 (s ; **H**C=C**H** du maléimide)

### B. Préparation d'une émulsion de gouttelettes de 50 nm (LNP-maléimide 50)

Une émulsion de gouttelettes contenant le tensioactif SA_{CONH}PEG₁₀₀-maleimide sont préparées avec la composition indiquée dans le tableau 17 ci-dessous, comme décrit dans l'exemple 7 précédent pour préparation de la phase huileuse, de la phase aqueuse, mélange des 2 phases puis sonication. Les particules sont purifiées par dialyse (MWCO 12-14 kDa) contre du PBS et diluées de façon à obtenir une fraction massique de 20%.

**Tableau 17: composition des LNP-maleimide 50**

| | masse (mg) | | | | | |
|---|---|---|---|---|---|---|
| | Phase lipidique | | | Phase aqueuse | | |
| F 50 | Huile soja purifiée | Cire Suppocire NC | Lécithine Lipoid s75 | Myrj^{®} S40 | SA_{CONH}PEG₁₀₀-maleimide | PBS 1X |
| | 68 | 272 | 65 | 276 | 69 | 2500 |

### C. Préparation d'une émulsion de gouttelettes de 120 nm (LNP-maléimide 120)

Une émulsion de gouttelettes contenant le tensioactif SA_{CONH}PEG₁₀₀-maleimide sont préparées avec la composition indiquée dans le tableau 18 ci-dessous, comme décrit dans l'exemple 7 précédent pour préparation de la phase huileuse, de la phase aqueuse, mélange des 2 phases puis sonication. Les gouttelettes sont purifiées par dialyse (MWCO 12-14 kDa) contre du PBS et diluées de façon à obtenir une fraction massique de 20%.

**Tableau 18: composition des LNP-maleimide F120**

| | masse (mg) | | | | | |
|---|---|---|---|---|---|---|
| | Phase lipidique | | | Phase aqueuse | | |
| F 120 | Huile soja purifiée | Cire Suppocire NC | Lécithine Lipoid s75 | Myrj^{®} S40 | SA_{CONH}PEG₁₀₀-maleimide | PBS 1X |
| | 450 | 150 | 45 | 172 | 43 | 1140 |

### D. Formation de gel à partir de l'émulsion LNP-mal et le cross-linker SH-PEG-SH

Une quantité de 100 mg de l'émulsion LNP-mal préparée (500 µL de suspension de gouttelettes de 120 nm ou 50 nm de diamètre) sont mises à réagir avec 0.9 mg de poly(éthylène glycol) dithiol (masse moléculaire moyenne en masse 1000 Da) à température ambiante avec un ratio thiol/maléimide de 1:1. Le mélange est mis sous agitation modérée.

Un gel commence à se former quelques minutes après le mélange des réactifs.

### Exemple 12 : Formation de gels photo-dissociables

### A. Synthèse du surfactant SA_{CONH}PEG100NH-ONB-Maléimide

Il s'agit d'un surfactant de type polyéthylène glycol (100 unités) dont une extrémité est liée à une chaîne grasse (C18) via une fonction amide et de l'autre possède une autre fonction amide liée à un groupe maléimide via un groupe photoclivable de type orthonitrobenzyle (ONB). Sa structure et son schéma de synthèse sont détaillés ci-dessous.

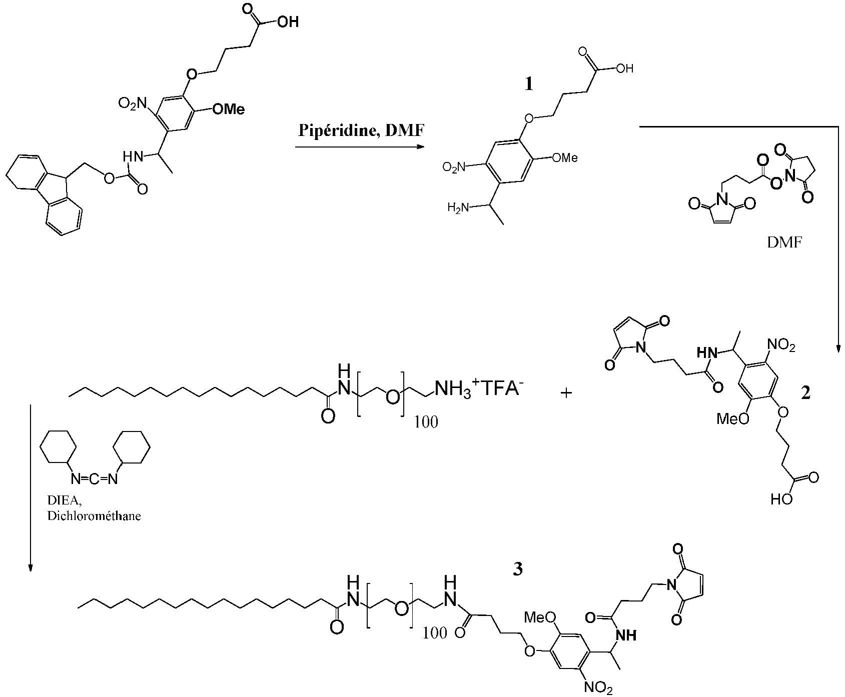

### Synthèse du produit 1

Le Fmoc-Photo-Linker (950 mg, 1.82 mmol, fournisseur IRIS BIOTECH GMBH) a été mis à réagir avec la pipéridine (237.26 µl, 2.36 mmol) dans 12 mL de DMF. On a maintenu l'agitation pendant 3 heures sous argon. Le milieu réactionnel a été évaporé à sec. La réaction a été suivie par CCM en utilisant l'éluant dichlorométhane/méthanol (9/1). Le produit 1 a été obtenu sous forme d'une huile jaune. Le produit brut a été engagé tel quel dans l'étape suivante.

### Synthèse du produit 2

Le produit 1 a été dissout dans le diméthylformamide (15 mL) auquel a été ajouté l'ester 4-maléimidobutyric acid N-hydroxysuccinimide (665.63 mg, 2.366 mmol). Le mélange a été agité pendant 3 heures sous argon et la réaction a été suivie par CCM : éluant dichlorométhane/méthanol (9/1). Après l'évaporation du DMF, le produit brut a été purifié par chromatographie sur gel de silice (éluant : acétate d'éthyle puis dichlorométhane/méthanol 9/1). Le produit 2 a été obtenu sous forme d'une huile jaune (571.42 mg, 1.22 mmol, 67% rendement des 2 étapes).

**RMN-¹H (200 MHz, CDCl₃)** : ppm 1.51 (3H, d, CH₃); 1.89 (2H, m, N-CH₂- **CH₂**-CH₂) ; 2.05-2.21 (4H, m, NH-CO-**CH₂** et O-CH₂-**CH₂**); 2.51 (2H, t, COOH-**CH₂**); 3.52 (2H, t, O-**CH₂**) ; 3.92 (3H, s, O-**CH3**) ; 4.11 (2H, t, N-**CH₂**) ; 5.52 (1H, m, **CH-**CH₃) ; 6.69 (2H, s, 2CH maléimide) ; 7-7.55 (2H_{Ar}, 2s); 8.02 (1H, N**H**).

SM (ESI mode positif) : M_{calc} = 463.44 (C₂₁H₂₅N₃O₉) ; m/z = 486.2 [M+Na]⁺

### Synthèse du produit 3

Sous Argon, du SA_{CONH}PEG100-NHa⁺TFA⁻ (surfactant C de l'exemple 1, 165 mg, 0.033 mmol) et de la DIEA (17.3 µL, 0.1 mmol) ont été dissout dans du dichlorméthane (5 mL). Après 5 minutes d'agitation, le produit 2 (47.62 mg, 0.1 mmol) et du DCC (20 mg, 0.1 mmol) ont été ajoutés au milieu réactionnel. Après 3 heures de réaction, le mélange réactionnel a été précipité dans l'éther et purifié par chromatographie sur gel de silice. Le produit 3 a été obtenu sous forme de poudre jaune.

**RMN-¹H (200 MHz, CDCl₃)** : d ppm 0.88 (3 H, t, CH₃); 1.07 (2 H, m, C**H₂**-CH₂-CONH); 1.11 (2 H, t, C**H2**-CONH); 1.19 (28 H, m, 14 CH2); 1.63 (2H, m, N-CH₂- **CH₂**-CH₂) ; 1.86 (3H, d, CH₃); 2.1-2.2 (6H, m, NH-CO-**CH₂**, O-CH₂-**CH₂** et COOH-**CH₂**); 3.46 (2H, t, O-**CH₂**) ; 3.5-3.70 (400 H, m, CH₂ PEG) ; 3.96 (3H, s, O-**CH3**) ; 4.11 (2H, t, N-**CH₂**) ; 5.14 (1H, m, **CH**-CH₃) ; 5.65 (2H, s, 2CH maléimide) ; 6.97-7.53 (2H_{Ar}, 2s); 8.32 (1H, NH).

### B. Préparation d'une émulsion de gouttelettes de 50 nm (LNP PNB maléimide 50)

Une émulsion contenant le tensioactif SA_{CONH}PEG₁₀₀-ONB-maleimide est préparée avec la composition indiquée dans le tableau 19 ci-dessous, comme décrit dans l'exemple 7 précédent pour préparation de la phase huileuse, de la phase aqueuse, mélange des 2 phases puis sonication. Les particules sont purifiées par dialyse (MWCO 12-14 kDa) contre du PBS et diluées de façon à obtenir une fraction massique de 20%.

**Tableau 19: composition des LNP-ONB-maleimide 50**

| | masse (mg) | | | | | |
|---|---|---|---|---|---|---|
| | Phase lipidique | | | Phase aqueuse | | |
| F 50 | Huile soja purifiée | Cire Suppocire NC | Lécithine Lipoid s75 | Myrj^{®} S40 | SA_{CONH}PEG₁₀₀-ONB-maleimide | PBS 1X |
| | 68 | 272 | 65 | 276 | 69 | 2500 |

### C. Préparation d'une émulsion de gouttelettes de 120 nm (LNP PNB maléimide 120)

Une émulsion contenant le tensioactif SA_{CONH}PEG₁₀₀-maleimide est préparée avec la composition indiquée dans le tableau 20 ci-dessous, comme décrit dans l'exemple 7 précédent pour préparation de la phase huileuse, de la phase aqueuse, mélange des 2 phases puis sonication. Les particules sont purifiées par dialyse (MWCO 12-14 kDa) contre du PBS et diluées de façon à obtenir une fraction massique de 20%.

**Tableau 20: composition des LNP-ONB-maleimide F120**

| | masse (mg) | | | | | |
|---|---|---|---|---|---|---|
| | Phase lipidique | | | Phase aqueuse | | |
| F 120 | Huile soja purifiée | Cire Suppocire NC | Lécithine Lipoid s75 | Myrj^{®} S40 | SA_{CONH}PEG₁₀₀-ONB-maleimide | PBS 1X |
| | 450 | 150 | 45 | 172 | 43 | 1140 |

### D. Formation de gel à partir de l'émulsion LNP-ONB-mal et le cross-linker SH-PEG-SH

Une quantité de 100 mg de l'émulsion préparée (500 µL de suspension, LNP formulation 120 nm ou 50 nm) sont mises à réagir avec 0.9 mg de poly(éthylène glycol) dithiol (masse moléculaire moyenne en masse 1000 Da) à température ambiante avec un ratio thiol/maléimide de 1:1. Le mélange est mis sous agitation modérée.

Un gel commence à se former quelques minutes après le mélange des réactifs. Ce gel se forme grâce à la formation entre particules de ponts SA-PEG₅₀₀₀-ONB-mal-S-PEG₁₀₀₀-S-mal-ONB-PEG₅₀₀₀-SA.

### E. Photo-destruction du gel formé à partir de LNP-ONB-mal et cross-linker SH-PEG-SH

Le gel chimique formé précédemment, dont la cohésion est assurée par des liaisons entre particules de type SA-PEG₅₀₀₀-ONB-mal-S-PEG₁₀₀₀-S-mal-ONB-PEG₅₀₀₀-SA est photoclivable par irradiation à 365 nm en raison de la présence du groupe ONB.

## Revendications

1. Procédé de préparation d'un matériau comprenant une phase aqueuse continue et une phase dispersée sous forme de gouttelettes comprenant :
- un phospholipide,
- un lipide solubilisant comprenant au moins un glycéride d'acides gras,
- un co-tensioactif comprenant une chaîne composée de motifs d'oxyde d'éthylène ou d'oxyde d'éthylène et d'oxyde de propylène,
- une huile, et
- un tensioactif de formule (I) suivante :
(L₁-X₁-H₁-Y₁)ᵥ-G-(Y₂-H₂-X₂-L₂)_{w} (I),
dans laquelle :
- les radicaux L₁-X₁-H₁- et L₂-X₂-H₂- consistent indépendamment en un groupe de formule suivante : 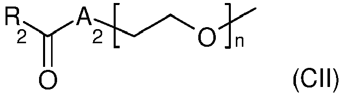 dans laquelle :
- R₂ représente une chaîne hydrocarbonée linéaire comprenant de 11 à 23 atomes de carbone,
- A₂ représente O ou NH,
- n représente un nombre entier de 3 à 500,
- Y₁ et Y₂ sont indépendamment choisis parmi :
- une liaison simple,
- un groupe Z choisi parmi -O-, -NH-, -O(OC)-, -(CO)O-, -(CO)NH-, -NH(CO)-, - O-(CO)-O-, -NH-(CO)-O-, -O-(CO)-NH- et -NH-(CO)-NH,
- un groupe Alk étant un alkylène comprenant de 1 à 6 atomes de carbone, et
- un groupe Z-Alk, Alk-Z, Alk-Z-Alk ou Z-Alk-Z où Alk et Z sont tels que définis ci-dessus et où les deux groupes Z du groupe Z-Alk-Z sont identiques ou différents,
- G représente un groupe de liaison et comprend au moins un groupe G' ayant l'une des formules suivantes : 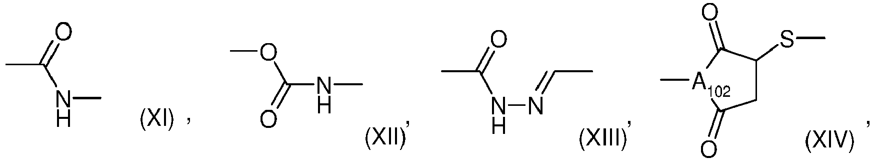 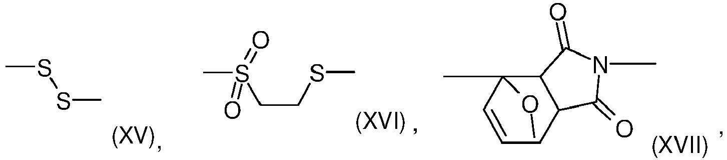 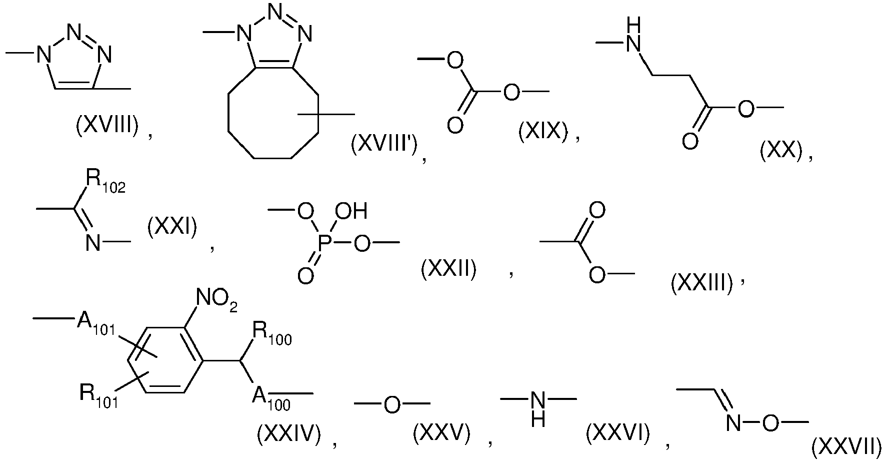 où A₁₀₂ représente CH ou N, R₁₀₂ représente H ou une chaîne hydrocarbonée linéaire comprenant de 1 à 6 atomes de carbone, A₁₀₁ représente -O-, -NH-(CO)- ou -O-(CO)-, R₁₀₀ représente H ou un méthyle, A₁₀₀ représente -O- ou -NH- et R₁₀₁ représente H, Me ou -OMe,
v et w représentent 1, ledit procédé comprenant la mise en contact :
- d'une émulsion 1 comprenant une phase aqueuse continue et une phase dispersée sous forme de gouttelettes comprenant un phospholipide et un tensioactif de formule (LI) suivante :
L₁-X₁-H₁-Y₁-G₁ (LI),
- avec une émulsion 2 comprenant une phase aqueuse continue et une phase dispersée sous forme de gouttelettes comprenant un phospholipide et un tensioactif de formule (LII) suivante :
G₂-Y₂-H₂₋X₂-L₂ (LII)
où G₁ et G₂ sont des groupes susceptibles de réagir pour former le groupe G, dans des conditions permettant la réaction des tensioactifs de formules (LI) et (LII) pour former le tensioactif de formule (I), ce par quoi des liaisons covalentes entre les gouttelettes de la phase dispersée se forment.

2. Procédé de préparation selon la revendication 1, dans lequel la réaction de G₁ et G₂ pour former le groupe G est réalisée par irradiation du mélange formé par l'émulsion 1 et l'émulsion 2 au moyen d'un rayonnement lumineux.

3. Procédé de préparation selon la revendication 1 ou 2, dans lequel la phase aqueuse de l'émulsion 1 et/ou de l'émulsion 2 présente un pH compris entre 5,5 et 8,5, de préférence entre 6 et 7,5.

4. Procédé de préparation selon l'une quelconque des revendications 1 à 3, dans lequel le groupe G comprend un seul groupe G' et les groupes G₁ et G₂ sont des groupes susceptibles de réagir l'un avec l'autre pour former le groupe G.

5. Procédé de préparation selon l'une quelconque des revendications 1 à 3, dans lequel le groupe G comprend plusieurs groupes G', le procédé comprenant la mise en contact des émulsions 1 et 2 avec un composé susceptible de réagir avec les tensioactifs de formules (LI) et (LII) pour former le groupe G.

6. Matériau susceptible d'être obtenu par le procédé selon l'une des revendications 1 à 5, comprenant une phase aqueuse continue et une phase dispersée sous forme de gouttelettes liées entre-elles de façon covalente par le tensioactif de formule (I).

7. Matériau selon la revendication 6, dans lequel le tensioactif de formule (I) a la formule (I') suivante : 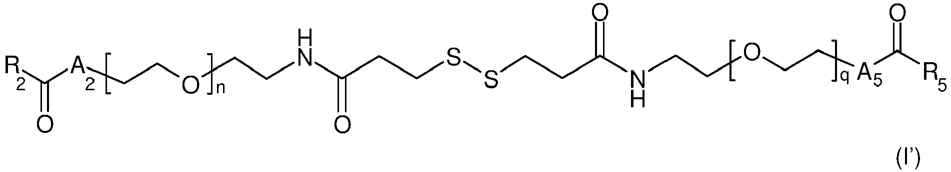 dans laquelle :
- R₂ et R₅ représentent indépendamment une chaîne hydrocarbonée linéaire comprenant de 11 à 23 atomes de carbone,
- A₂ et A₅ représentent O ou NH, et
- n et q représentent indépendamment des nombres entiers de 3 à 500.

8. Matériau selon l'une quelconque des revendications 6 à 7, dans lequel, dans la formule (I), le groupe G comprend une fonction clivable.

9. Matériau selon la revendication 6, dans lequel le rapport de la masse de tensioactif de formule (I) sur la masse de l'ensemble (tensioactif de formule (I) / co-tensioactif) est supérieur ou égal à 15%.

10. Matériau selon l'une quelconque des revendications 6 à 9, dans lequel le potentiel zêta des gouttelettes en phase aqueuse est compris entre -20 mV et + 20 mV.

11. Matériau selon l'une quelconque des revendications 6 à 10, comprenant un agent d'intérêt.

12. Matériau selon la revendication 11, où l'agent d'intérêt est un agent thérapeutique.

## Patentansprüche

1. Herstellungsverfahren eines Materials, umfassend eine kontinuierliche wässrige Phase und eine dispergierte Phase in Form von Tröpfchen, umfassend:
- ein Phospholipid,
- ein solubilisierendes Lipid, umfassend mindestens ein Glycerid von Fettsäuren,
- ein Co-Tensid, umfassend eine Kette aus Ethylenoxid-Einheiten, oder
- von Ethylenoxid und Propylenoxid,
- ein Öl, und
- ein Tensid folgender Formel (I):
(L₁-X₁-H₁- Y₁)ᵥ-G-(Y₂-H₂-X₂-L₂)_{w} (I),
wobei:
- die Radikale L₁-X₁-H₁- und L₂-X₂-H₂- unabhängig aus einer Gruppe folgender Formel bestehen: 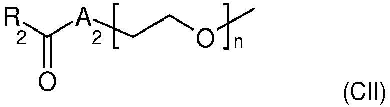 wobei:
- R₂ eine lineare Kohlenwasserstoffkette darstellt, umfassend 11 bis 23 Kohlenstoffatome,
- A₂ O oder NH darstellt,
- n eine ganze Zahl von 3 bis 500 darstellt,
- Y₁ und Y₂ unabhängig ausgewählt sind aus:
- einer Einfachbindung,
- einer Z-Gruppe, ausgewählt aus -O-, -NH-, -O(OC)-, -(CO)O-, -(CO)NH-, - NH(CO)-, - O-(CO)-O-, -NH-(CO)-O-, -O-(CO)-NH- und -NH-(CO)-NH,
- wobei eine Alk-Gruppe ein Alkylen ist, umfassend 1 bis 6 Kohlenstoffatome, und eine Z-Alk-, Alk-Z-, Alk-Z-Alk- oder Z-Alk-Z-Gruppe, wobei Alk und Z wie oben definiert sind und wobei die zwei Z-Gruppen der Z-Alk-Z-Gruppe gleich oder verschieden sind,
- G eine Verbindungsgruppe darstellt und mindestens eine G'-Gruppe umfasst, die eine der folgenden Formeln aufweist: 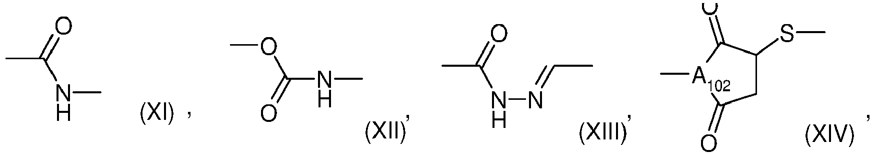 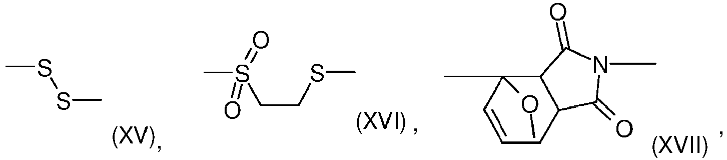 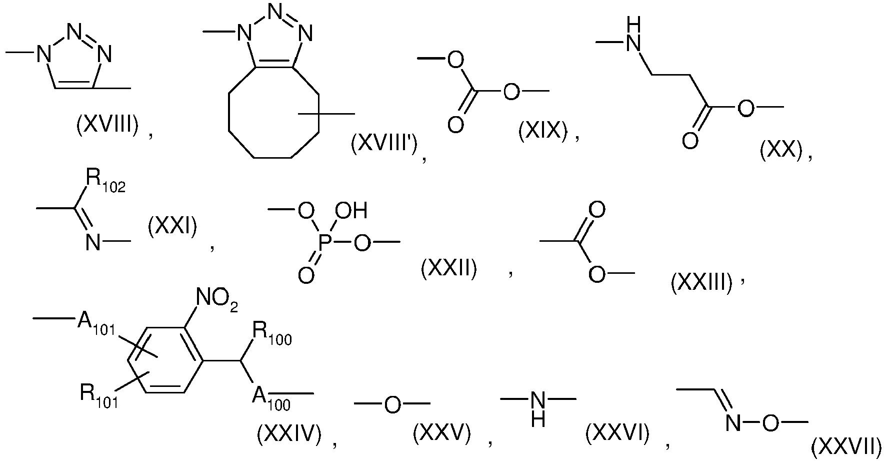 wobei A₁₀₂ CH oder N darstellt, R₁₀₂ H oder eine lineare Kohlenwasserstoffkette umfassend 1 bis 6 Kohlenstoffatome darstellt, A₁₀₁ -O-, -NH-(CO)- oder -O-(CO)-darstellt, R₁₀₀ H oder Methyl darstellt, A₁₀₀ -O- oder -NH- darstellt und R₁₀₁ H, Me oder - OMe darstellt,
v und w 1 darstellen, das Verfahren umfassend das Inkontaktbringen:
- einer Emulsion 1, umfassend eine kontinuierliche wässrige Phase und eine dispergierte Phase in Form von Tröpfchen, umfassend ein Phospholipid und ein oberflächenaktives Mittel folgender Formel (LI):
L₁-X₁-H₁-Y₁-G₁ (LI),
- mit einer Emulsion 2, umfassend eine kontinuierliche wässrige Phase und eine dispergierte Phase in Form von Tröpfchen, umfassend ein Phospholipid und ein oberflächenaktives Mittel folgender Formel (LII):
G₂-Y₂-H₂.X₂-L₂ (LII)
wobei G₁ und G₂ Gruppen sind, die reagieren können, um die Gruppe G zu bilden, unter Bedingungen, die die Reaktion der Tenside der Formeln (LI) und (LII) zum Bilden des Tensids von Formel (I) ermöglichen, wodurch kovalente Bindungen zwischen den Tröpfchen der dispersen Phase gebildet werden.

2. Herstellungsverfahren nach Anspruch 1, wobei die Reaktion von G₁ und G₂ zum Bilden der Gruppe G durch Bestrahlung des aus der Emulsion 1 und der Emulsion 2 gebildeten Gemischs mittels Lichtstrahlung erfolgt.

3. Herstellungsverfahren nach Anspruch 1 oder 2, wobei die wässrige Phase der Emulsion 1 und/oder der Emulsion 2 einen pH zwischen 5,5 und 8,5, vorzugsweise zwischen 6 und 7,5, aufweist.

4. Herstellungsverfahren nach einem der Ansprüche 1 bis 3, wobei die Gruppe G eine einzelne Gruppe G' umfasst und die Gruppen G₁ und G₂ Gruppen sind, die miteinander reagieren können, um die Gruppe G zu bilden.

5. Herstellungsverfahren nach einem der Ansprüche 1 bis 3, wobei die Gruppe G mehrere Gruppen G' umfasst, wobei das Verfahren das Inkontaktbringen der Emulsionen 1 und 2 mit einer Verbindung umfasst, die mit den Tensiden der Formeln (LI) und (LII) reagieren kann, um die Gruppe G zu bilden.

6. Material, das durch das Verfahren nach einem der Ansprüche 1 bis 5 erlangt werden kann, umfassend eine kontinuierliche wässrige Phase und eine dispergierte Phase in Form von Tröpfchen, die durch das Tensid von Formel (I) kovalent miteinander verbunden sind.

7. Material nach Anspruch 6, wobei das Tensid von Formel (I) die folgende Formel (I') aufweist: 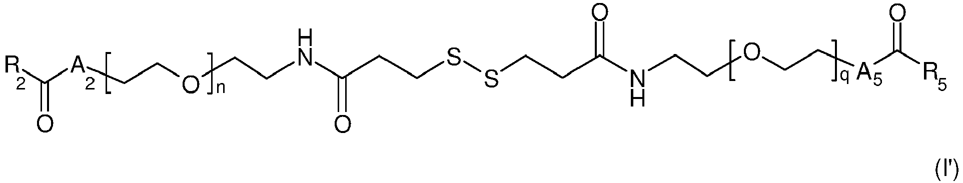 wobei:
- R₂ und R₅ unabhängig eine lineare Kohlenwasserstoffkette darstellen, umfassend 11 bis 23 Kohlenstoffatome,
- A₂ und A₅ O oder NH darstellen, und
- n und q unabhängig ganze Zahlen von 3 bis 500 darstellen.

8. Material nach einem der Ansprüche 6 bis 7, wobei in Formel (I) die Gruppe G eine spaltbare Funktion umfasst.

9. Material nach Anspruch 6, wobei das Verhältnis der Tensidmasse von Formel (I) zu der Masse der Gesamtheit (Tensid von Formel (I) / Co-Tensid) größer als oder gleich wie 15 % ist.

10. Material nach einem der Ansprüche 6 bis 9, wobei das Zetapotenzial der Tröpfchen in der Wasserphase zwischen -20 mV und +20 mV liegt.

11. Material nach einem der Ansprüche 6 bis 10, umfassend ein Mittel von Interesse.

12. Material nach Anspruch 11, wobei das Mittel von Interesse ein therapeutisches Mittel ist.

## Claims

1. A method for preparing a material comprising of a continuous aqueous phase and a dispersed phase dispersed in the form of droplets containing:
- a phospholipid,
- a solubilising lipid including at least one glyceride of fatty acids,
- a co-surfactant containing a chain composed of units of ethylene oxide or of ethylene oxide and of propylene oxide,
- an oil, and
- a surfactant having the following formula (I):
(L₁-X₁-H₁-Y₁)ᵥ-G-(Y₂-H₂-X₂-L₂)_{w} (I),
wherein:
the radicals L₁-X₁-H₁- and L₂-X₂-H₂- independently consist of one of the groups having the following formulas: 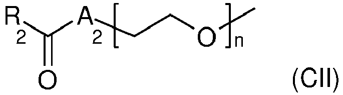 in which:
- R₂ represents a linear hydrocarbon chain containing from 11 to 23 carbon atoms,
- A₂ represents O or NH,
- n represents an integer from 3 to 500,
- Y₁ and Y₂ are independently selected from among:
- a single bond,
- a Z group selected from among -O-, -NH-, -O(OC)-, -(CO)O-, -(CO)NH-, - NH(CO)-,
-O-(CO)-O-, -NH-(CO)-O-, -O-(CO)-NH- et -NH-(CO)-NH,
- a Alk group being an alkylene containing from 1 to 6 carbon atoms, and
- a Z-Alk, Alk-Z, Alk-Z-Alk or Z-Alk-Z group, where Alk and Z are as defined here above and where the two Z groups of the Z-Alk-Z group are identical or different,
- G represents a linking group and includes at least one G' group having one of the following formulas: 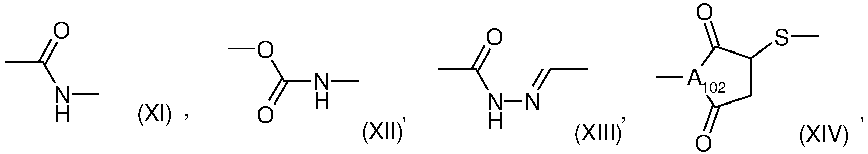 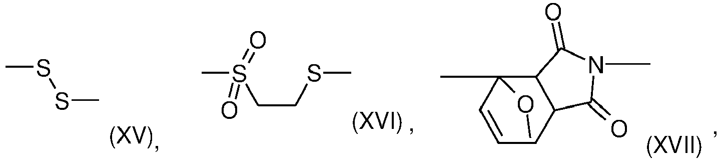 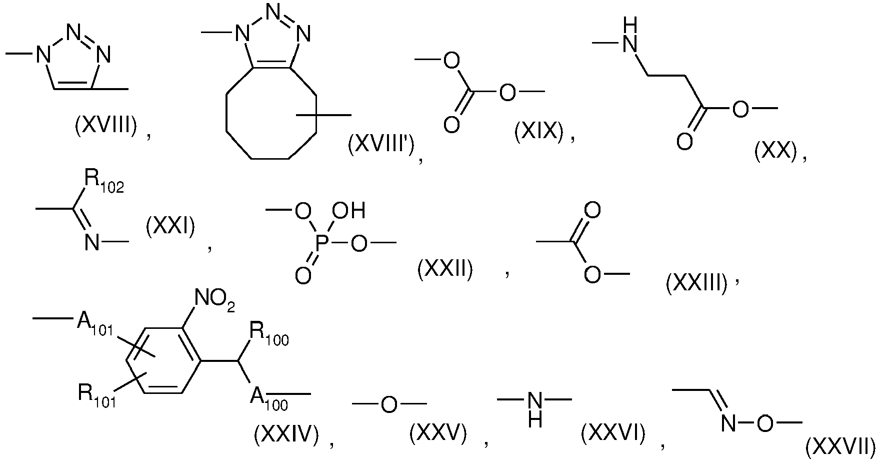 where A₁₀₂ represents CH or N, R₁₀₂ represents H or a linear hydrocarbon chain containing from 1 to 6 carbon atoms, A₁₀₁ represents -O-, -NH-(CO)- ou -O-(CO)-, R₁₀₀ represents H or a methyl, A₁₀₀ represents -O- or -NH- and R₁₀₁ represents H, Me or -OMe, and
- v and w are 1,
the said method comprising bringing into contact:
- an emulsion 1 comprising a continuous aqueous phase and a dispersed phase, dispersed in the form of droplets containing an phospholipid and a surfactant having the following formula (LI):
L₁-X₁-H₁-Y₁-G₁ (LI),
- with an emulsion 2 comprising a continuous aqueous phase and a dispersed phase, dispersed in the form of droplets containing an phospholipid and a surfactant having the following formula (LII):
G₂-Y₂-H₂₋X₂-L₂ (LII)
where G₁ and G₂ are groups that are capable of reacting in order to form the G group, under conditions that allow for the reaction of the surfactants having the formulas (LI) and (LII) in order to form the surfactant having the formula (I), whereby covalent bonds between the droplets of the dispersed phase are formed.

2. Method of preparation according to claim 1, wherein the reaction of G₁ and G₂ in order to form the G group is carried out by irradiation of the mixture formed by the emulsion 1 and the emulsion 2 by means of a light radiation.

3. Method of preparation according to claim 1 or 2, wherein the aqueous phase of the emulsion 1 and / or of the emulsion 2 has a pH comprised between 5.5 and 8.5, and preferably between 6 and 7.5

4. Method of preparation according to any one of claims 1 to 3, wherein the G group comprises a single G' group and the G₁ and G₂ groups are able to react with each other in order to form the G group.

5. Method of preparation according to any one of claims 1 to 3, wherein the G group comprises several G' groups, the method comprising bringing into contact the emulsions 1 and 2 with a compound able to react with the surfactants having the formulas (LI) and (LII) in order to form the G group.

6. A material obtainable by the method according to one of claims 1 to 5, comprising of a continuous aqueous phase and a dispersed phase, dispersed in the form of droplets bonded to each other in a covalent manner by the surfactant having the formula (I).

7. Material according to claim 6, wherein the surfactant having the formula (I) has the following formula (I'): 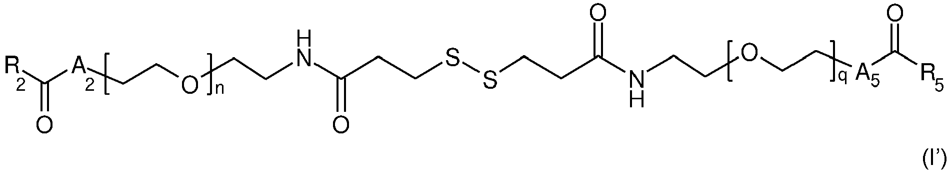 in which:
- R₂ and R₅ independently represent a linear hydrocarbon chain containing from 11 to 23 carbon atoms,
- A₂ and A₅ represent O or NH, and
- n and q independently represent integers from 3 to 500.

8. Material according to any one of claims 6 to 7, wherein, in the formula (I), the G group includes a cleavable function.

9. Material according to claim 6, wherein the ratio of the mass of surfactant having the formula (I) over the mass of the ensemble (surfactant having the formula (I) / co-surfactant) is greater than or equal to 15%.

10. Material according to any one of claims 6 to 9, wherein the zeta potential of the droplets in the aqueous phase is comprised between -20 mV and +20 mV.

11. Material according to any one of claims 6 to 10, comprising an agent of interest.

12. Material according to claim 11, wherein the agent of interest is a therapeutic agent.
